# EUROPEAN PATENT APPLICATION

(11) **EP 2 853 897 A1**
(43) Date of publication of application: **01.04.2015**
(21) Application number: 14198032.6
(22) Date of filing: 07.05.2009
(51) Int. Cl.: G01N 33/68, A61K 31/472

(54) **Sensory receptors for chronic fatigue and pain and uses thereof**

(30) Priority: 08.05.2008 US 51530 P
(62) Divisional of application: 09743672.9
(71) Applicant: University Of Utah Research Foundation, Salt Lake City, UT 84108 (US)
(72) Inventor: Light, Alan R., Park City, UT 84089 (US); Light, Kathleen C., Park City, UT 84089 (US); Hugen, Ronald W., Salt Lake City, UT 84116 (US); White, Andrea T., Salt Lake City, UT 84108 (US)
(74) Representative: Michalski Hüttermann & Partner Patentanwälte mbB

(57) **Abstract**

The present invention relates generally to the fields of pathological muscle pain/fatigue conditions. More specifically, the invention concerns a method for diagnosing pathological muscle pain/fatigue conditions. The invention also concerns a method for of determining responsiveness of a pathological muscle/fatigue condition to treatment with a pharmaceutical agent, as well as a method for screening the efficacy of a pharmaceutical agent for the ability to treat pathological muscle/fatigue conditions.

## Description

### BACKGROUND

In muscle, nociceptive afferent neurons are mostly Group III and IV that correspond in conduction velocity and size to cutaneous Aδ and C afferent respectively (Graven-Nielsen and Mense, 2001; Kaufman et al., 2002)]. Most previous examinations of response properties of afferent neurons innervating skeletal muscle have used mechanical stimuli to evoke responses (Adreani et al., 1997; Bove and Light, 1995; Diehl et al., 1993; Hayes et al., 2006; Hoheisel et al., 2004; Hoheisel et al., 2005; Kaufman et al., 2002; Mense and Meyer, 1985). Equally important are chemical stimuli, since these are critical for understanding fatigue and pain states induced by various levels of exercise in working muscles. Chemical mediators liberated by muscle contraction include lactic acid, ATP, and increasing protons. Previous investigations tested these on muscle afferents along with non-physiological chemicals such as hypertonic saline, acid saline, and capsaicin. However, in order to activate significant numbers of muscle-innervating primary afferent neurons, the concentrations of these chemicals were often at levels that are rarely present in skeletal muscle. Nevertheless, these investigations suggested key roles for the molecular receptor types ASIC (Acid Sensing Ion Channels), P2X (Purinergic type 2X) and TRPV (Transient Receptor Potential of the Vanilloid type) in mediating muscle pain induced by metabolites (Adreani and Kaufman, 1998; Gao et al., 2007; Hoheisel et al., 2004; Kaufman and Rybicki, 1987; Leffler et al., 2006; Reinohl et al., 2003; Rotto and Kaufman, 1988).

Acid Sensing Ion Channels, also referred to as ASIC channels and/or amiloride sensitive ion channels, are membrane protein complexes formed by three subunits (See Gouaux et al., Nature 449, 316-322 (2007)). To date, there have been six isoforms isolated coded by four different genes. Two isoforms have been spliced into two variants, namely ASIC1a and ASIC1b and ASIC2a and ASIC2b. Other isoforms include ASIC3 (also known as DRASIC) and ASIC4. Most muscle C-afferent nerve fibers express large amounts of ASIC3, while some also express TRPV 1. It has been shown that ASIC3 does not give sustained response until the pH levels reach levels lower than physiological levels. This has led to another controversy. Part of the issue surrounding ASIC3 revolved around lactic acid. It is known that lactic acid does not cause muscle pain, however ASIC3 is more sensitive to lactic acid than to pH. This is thought to be due to a Ca⁺⁺ chelating mechanism. It has been determined that ASIC3 becomes activated at closer to physiologic ranges of lactic acid (pH of 7-7.3).

Another issue surrounding ASIC3 revolves around ATP. ATP is released during normal muscle contractions. If ATP is added to a sample, ASIC3 receptors become much more sensitive. It has also been determined that ATP signals via the P2X5 receptor can enhance sensitivity of ASIC3 (See Molliver et al., Mol. Pain., Nov 23;1:35 (2005). Molliver et al. showed 1) ASIC3 is expressed in a restricted population of nociceptors and probably in some non-nociceptors; 2) co-expression of ASIC3 and CGRP, and the absence of P2X3, are distinguishing properties of a class of sensory neurons, some of which innervate blood vessels. As described below, ASIC3 combined with P2X5 has appropriate sensitivity to detect normal resting metabolite levels and the mechanism does nnot require ion flux through P2X5 for modulation of ASIC3.

The mismatch between previous data and the poor activation of muscle sensory neurons by physiological or pathophysiological concentrations of known agonists for these receptors points to a need for better understanding of the factors involved. Molecular receptors found on cardiac and skeletal muscle afferent neurons do not respond to a single metabolite in the physiological range, but rather to a combination of two or more factors produced by muscle contraction (Naves and McCleskey, 2005; Spelta et al., 2004). It might also be possible that at least two different molecular receptors cooperate to enhance the sensitivity of detection for protons, and that these two receptors co-localize on cardiac and skeletal muscle afferent endings. The co-operation between these molecular receptors apparently allows the sensory nerve endings to precisely detect and quantify the amounts of muscle metabolites produced by contraction (Connor et al., 2005; Immke and McCleskey, 2001; Immke and McCleskey, 2003; Molliver et al., 2005; Sutherland et al., 2001; Yagi et al., 2006).

In addition to muscle pain, muscle Group III and IV primary afferent neurons are very important as the afferent arm of sympathetic reflexes evoked by muscle contraction (Kaufman and Hayes, 2002). Primary afferent neurons detecting metabolites produced by contracting muscle were shown to evoke sympathetic reflexes more than 70 years ago (Alam and Smirk, 1937). In these initial observations, it was shown that afferent receptors within the muscle might be responsible for detecting these metabolites. Later experiments confirmed that small-diameter primary afferent fibers carried the message to the central nervous system causing these reflexes (Coote et al., 1971; McCloskey and Mitchell, 1972). In addition, early experiments indicated that these reflexes were accompanied by the sensation of "tiredness or pressure" from the fatiguing muscle when lower levels of metabolites were present, and sensations of muscle pain were reported only when higher levels of metabolites accumulated (Alam and Smirk, 1937). It is therefore possible that primary afferent endings in muscle could detect and code muscle work using both mechanical and metabolic information. These "work receptors" were later defined as "ergoreceptors" -sensory endings encoding muscle work, a subset of which could be "metaboreceptors"-sensory endings that encode muscle metabolites levels produced by muscle work (Kniffki et al., 1981).

Despite the early belief that muscle afferents specific for detecting non-nociceptive metabolic information might exist, ergoreceptors and metaboreceptors have only rarely been recorded (Adreani and Kaufman, 1998; Kaufman et al., 1983; Kaufman et al., 1984; Kniffki et al., 1978; Mense and Stahnke, 1983; Rotto and Kaufman, 1988; Thimm and Baum, 1987). Whether specific populations of muscle sensory afferents for nociception vs innocuous metaboreception exist is under investigation (Kniffki et al., 1978; Kniffki et al., 1981; Mense, 1996; Mense and Stahnke, 1983).

As such, there remains a need for better ways to detect and diagnose fatigue and pain, including a need for specific biomarkers of the disease. An additional need exists for improved methods and compositions for the treatment of fatigue and pain. The compositions and methods disclosed herein will provide a means to address such issues.

All references cited herein, including patent applications and publications, are incorporated by reference in their entirety.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate several embodiments of the invention and together with the description, serve to explain the principles of the invention. These are non-limiting examples.
Figure 1 shows the effects of antagonists to ASICs (A-317567 - filled triangles) and TRPV1 (JYL-1433 and LJO-328 - open circles and squares respectively) at indicated doses on responses to increased metabolites as presented in previous graphs. Controls (filled diamonds) received only the metabolite increases indicated on the X axis. For the antagonist applications, each well averaged 108 + 5 neurons imaged for calcium increases. Statistical analyses used wells as the sample size.
Figure 2 shows the effects of the P2X antagonists TNP-ATP and PPADS at various doses on responses of DRG neurons to increases in metabolites. Note that very low concentrations of TNP-ATP (10 nM) facilitated responses (inset graph) while concentrations of TNP-ATP that antagonize P2X5 (1µM) reduced low metabolite responses, and concentrations that block all P2X receptors including P2X4 (in mouse) (200 µM) blocked nearly all responses to metabolites.
Figure 3 shows a percent increase in mRNA levels of various receptors eight days after carageenan injection into the gastrocnemius muscle on one side (causes inflammation) into control (normal) mice or ASIC3 knock-out (KO) mice.
Figure 4 shows percent change from baseline of Alpha-2, Beta-1 and Beta-2 mRNA for adrenergic receptors in unfit CFS/FMS patients vs. control patients after various periods post-exercise.
Figure 5 shows a proposed model of ASIC2, P2X5, TRPV1, and Adβ2 effects on autonomic reflexes, the central nervous system (fatigue and pain), the central immune system, as well as the peripheral immune system.
Figure 6 shows a graph of the fold increase of mRNA expression of metabolite detection genes after up to 48 hours exercise.
Figure 7 shows a graph of the fold increase of mRNA expression of adrenergic function genes after up to 48 hours exercise.
Figure 8 shows a graph of the fold increase of mRNA expression of cytokine and cytokine receptor genes after up to 48 hours exercise.
Figure 9 shows graphs of scores for physical fatigue, mental fatigue, and pain by group. Left panels are baseline scores and right panels represent changes from baseline to 30 min and 8, 24, and 48 hrs post-exercise.
Figure 10 show graphs of white cell counts (top panel) and red cell counts (bottom panel) for CFS patients and Controls at baseline and at 0.5, 8, 24, and 48 hours following exercise.
Figure 11 shows a graph of IL-10 levels at baseline and at 0.5, 8, 24, and 48 hours post-exercise by group.
Figure 12 shows graphs of changes from baseline to 8 hrs post-exercise by group for IL-10 and IL-13 (top panels), IL-6 and IL-8 (middle panels) and IL-1β and IL-12 (bottom panels).
Figure 13 shows graphs of group changes following exercise for IL-13 (top panel), CD40L (middle panel), and TNFα (bottom panel). Contrasts indicated significant across group decreases in IL-13 at 24 and 48 hrs post-exercise. For the high SF group, a within group increase in IL-13 was noted at 8 hrs post-exercise. Across group decreases in CD40L were observed at 0.5 and 24 hrs post-exercise.
Figure 14 shows the relationship between increases in IL-6 from baseline to 8 hrs post-exercise (top panel) and increases from baseline to 8 hrs post-exercise in CD40L (bottom panel) as they relate to increases in physical fatigue from baseline to 48 hrs post-exercise in CFS patients.

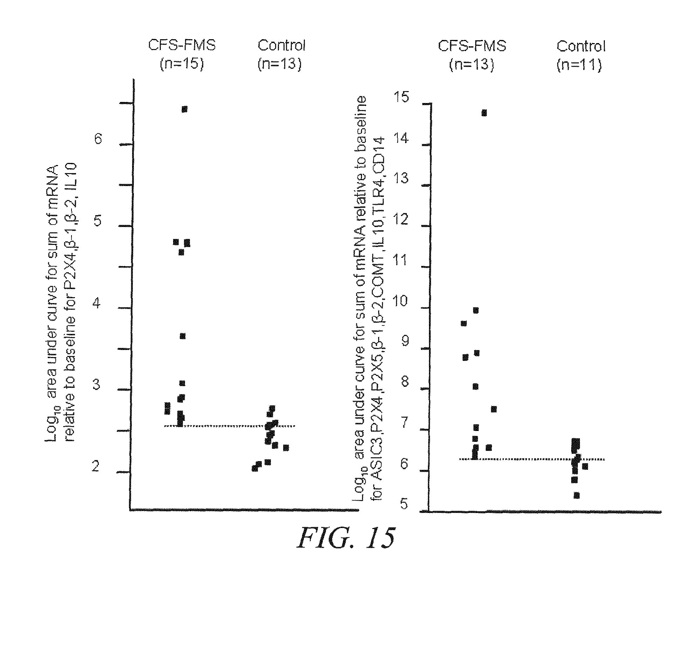

Figure 15 shows point plots of AUC for sum of 4 genes (left) and 9 genes (right). Dotted lines indicate the lowest values for CFS-FMS patients.

### DETAILED DESCRIPTION OF THE INVENTION

The present invention comprises methods and compositions comprising a combination of molecular receptors, including but not limited to ASIC, P2X5 and/or P2X4, and TRPV1 that can be used to detect metabolites by muscle-innervating sensory neurons, and theraptutic agents that are effective therein. The present invention comprises methods and compositions to detect chronic fatigue, fibromyalgia, chronic widespread pain, cancer-related fatigue, and other pathological muscle/fatigue conditions. Disclosed herein are methods of using ASIC, P2X5 and/or P2X4, and TRPV1 receptors as biomarkers for syndrome remission of chronic fatigue, fibromyalgia, chronic widespread pain, cancer-related fatigue, and other pathological muscle/fatigue conditions as well as for the use of treatments of the same.

All patents, patent applications and publications cited herein, whether supra or infra, are hereby incorporated by reference in their entireties into this application in order to more fully describe the state of the art as known to those skilled therein as of the date of the invention described and claimed herein.

It is to be understood that this invention is not limited to specific synthetic methods, or to specific recombinant biotechnology methods unless otherwise specified, or to particular reagents unless otherwise specified, to specific pharmaceutical carriers, or to particular pharmaceutical formulations or administration regimens, as such may, of course, vary. It is also to be understood that the terminology used herein is for the purpose of describing particular embodiments only and is not intended to be limiting.

### Definitions and Nomenclature

The terminology used herein is for the purpose of describing particular embodiments only and is not intended to be limiting.

As used in the specification and the appended claims, the singular forms "a," "an" and "the" can include plural referents unless the context clearly dictates otherwise. Thus, for example, reference to "a compound" includes mixtures of compounds, reference to "a pharmaceutical carrier" includes mixtures of two or more such carriers, and the like.

Ranges may be expressed herein as from "about" one particular value, and/or to "about" another particular value. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

The word "or" as used herein means any one member of a particular list and also includes any combination of members of that list.

By "sample" is meant an animal; a tissue or organ from an animal; a cell (either within a subject, taken directly from a subject, or a cell maintained in culture or from a cultured cell line); a cell lysate (or lysate fraction) or cell extract; or a solution containing one or more molecules derived from a cell or cellular material (e.g. a polypeptide or nucleic acid), which is assayed as described herein. A sample may also be any body fluid or excretion (for example, but not limited to, blood, urine, stool, saliva, tears, bile) that contains cells or cell components.

By "modulate" is meant to alter, by increasing or decreasing.

By "normal subject" is meant an individual who does not have pathological muscle/fatigue conditions.

By "pathological muscle/fatigue condition(s)" is meant to mean a condition that is associated with symptoms, including but not limited to weakness, aches, soreness and pains in muscles and/or connective tissues and/or joints, sleep disorders, excessive sleep, disrupted or unrefreshing sleep, general malaise, fever, sore throat, tender lymph nodes, impaired memory and/or mental fog, insomnia, depression, fatigue or tiredness not relieved by rest, post-exertional malaise, depression, anxiety, muscle tightness in the morning, muscle stiffness or heaviness, generalized soreness or aches, headaches, facial pain, cognitive impairment (memory lapses, slowing of thought processes or loss of concentration), gastrointestinal complaints (visceral pain, digestive system disorders, flatulency), frequent urination, diarrhea, constipation and dysmenorrheal. Pathological muscle/fatigue conditions include, but are not limited to chronic fatigue syndrome, fibromyalgia, chronic widespread pain, cancer-related fatigue, multiple sclerosis, myalgic encephalomyelitis, myeloencephalitis, Gulf War syndrome, post-infectious fatigue, post-viral fatigue, post vaccination fatigue, post-trauma fatigue, immune dysfunction syndrome, chronic stress-related fatigue, systemic lupus erythematosus, osteoarthritis, rheumatoid arthritis, postural orthostatic tachycardia syndrome, irritable bowel syndrome, multiple chemical sensitivity, repeptitive stress injury and athletic overtraining syndrome.

By "chronic fatigue syndrome" (also referred to as "CFS") is meant a debilitating disorder characterized by profound tiredness or fatigue not relieved by rest. Patients with CFS may become exhausted with only light physical exertion, and must often function at a level of activity substantially lower than their capacity before the onset of illness. In addition to the key defining characteristic of fatigue, CFS patients generally report various nonspecific symptoms, including weakness, slowness of both mental and physical functioning, muscle aches and pains, excessive sleep, post-exertional malaise or worsening of symptoms lasting more than 24 hours, fever, change in type or severity of headaches, sore throat, tender lymph nodes, impaired memory and/or mental concentration, insomnia or unrefreshing sleep, and depression or anxiety. Patients with CFS also suffer from disordered sleep, localized tenderness, and complaints of diffuse pain and fatigue.

By "fibromyalgia" is meant a syndrome characterized by chronic and intense generalized pain, or widespread chronic pain over multiple areas of the body; the pain is not limited to muscle tissue and may also be experienced in the joints, connective tissues, gastrointestinal tract and skin. In fibromyalgia, such generalized chronic pain is often accompanied by symptoms including fatigue, malaise, depression, anxiety, muscle tightness in the morning, muscle stiffness and sleep disorders. Other accompanying symptoms also include headaches, facial pain, cognitive impairment (memory lapses, loss of concentration), gastrointestinal complaints (visceral pain, digestive system disorders, flatulency), frequent urination, diarrhea, constipation and dysmenorrheal.

The term "metabolite detecting gene" refers to a gene capable of encoding a metabolite function peptide. Metabolite function peptides are peptides capable of detecting metabolites including, but not limited to, metabolites produced by muscle contraction. For example, a metabolite detecting gene can refer to a gene capable of encoding a metabolite function peptide capable of detecting any one of the following either alone, or by combining with other gene products that detect one or more of the following: protons (acid), ATP, ADP, or Adenosine, lactate, citrate, pyruvate, heat (above normal body temperature), di-protonated phosphate, ammonia, prostaglandins, carbon dioxide, and potassium.

The term "adrenergic function gene" refers to a gene capable of encoding an adrenergic function peptide. For example, an adrenergic function gene can refer to a gene capable of encoding an adrenergic function receptor. An adrenergic receptor is a class of G protein-coupled receptors that are targets of catecholamine. Adrenergic receptors can specifically bind and are activated by their endogenous ligands, the catecholamines adrenaline (epinephrine) and noradrenaline (norepinephrine).

The term "immune function gene" refers to a gene capable of encoding a peptide related to immune function. For example, an immune function gene can be capable of encoding a cytokine or receptor.

The amino acid abbreviations used herein are conventional one letter codes for the amino acids and are expressed as follows: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid.

"Peptide" as used herein refers to any peptide, oligopeptide, polypeptide, gene product, expression product, or protein. For example, a peptide can be a receptor. A polypeptide is comprised of consecutive amino acids. The term "polypeptide" encompasses naturally occurring or synthetic molecules.

In addition, as used herein, the term "peptide" or "polypeptide" refers to amino acids joined to each other by peptide bonds or modified peptide bonds, e.g., peptide isosteres, etc. and may contain modified amino acids other than the 20 gene-encoded amino acids. The polypeptides can be modified by either natural processes, such as post-translational processing, or by chemical modification techniques which are well known in the art. Modifications can occur anywhere in the polypeptide, including the peptide backbone, the amino acid side-chains and the amino or carboxyl termini. The same type of modification can be present in the same or varying degrees at several sites in a given polypeptide. Also, a given polypeptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleotide or nucleotide derivative, covalent attachment of a lipid or lipid derivative, covalent attachment of a phosphytidylinositol, disulfide bond formation, demethylation, formation of cysteine or pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, and transfer-RNA mediated addition of amino acids to protein such as arginylation. (See Proteins - Structure and Molecular Properties 2nd Ed., T.E. Creighton, W.H. Freeman and Company, New York (1993); Posttranslational Covalent Modification of Proteins, B.C. Johnson, Ed., Academic Press, New York, pp. 1-12 (1983)).

As used herein, the term "amino acid sequence" refers to a list of abbreviations, letters, characters or words representing amino acid residues.

The phrase "nucleic acid" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acids of the invention can also include nucleotide analogs (e.g., BrdU), and non-phosphodiester internucleoside linkages (e.g., peptide nucleic acid (PNA) or thiodiester linkages). In particular, nucleic acids can include, without limitation, DNA, RNA, cDNA, gDNA, ssDNA, dsDNA or any combination thereof

By "increased susceptibility to develop a pathological muscle/fatigue condition" is meant a subject who has a greater than normal chance of developing a pathological muscle/fatigue condition, compared to the general population. Such subjects could include, for example, subjects whose metabolite detecting gene expression levels or metabolite detecting receptor levels in a subject are higher than levels from a normal subject as detected after exercise.

By an "effective amount" of a compound as provided herein is meant a sufficient amount of the compound to provide the desired effect. The exact amount required will vary from subject to subject, depending on the species, age, and general condition of the subject, the severity of disease (or underlying genetic defect) that is being treated, the particular compound used, its mode of administration, and the like. Thus, it is not possible to specify an exact "effective amount." However, an appropriate "effective amount" may be determined by one of ordinary skill in the art using only routine experimentation.

By "knockout mutation" is meant an alteration in the nucleic acid sequence that reduces the biological activity of the polypeptide normally encoded therefrom by at least 80% relative to the unmutated gene. The mutation may, without limitation, be an insertion, deletion, frameshift, or missense mutation. A "knockout animal," for example, a knockout mouse, is an animal containing a knockout mutation. The knockout animal may be heterozygous or homozygous for the knockout mutation. Such knockout animals are generated by techniques that are well known in the art.

By "treat" is meant to administer a compound or molecule to a subject, such as a human or other mammal (for example, an animal model), that has a pathological muscle/fatigue condition or an increased susceptibility for developing pathological muscle/fatigue condition, in order to prevent or delay a worsening of the effects of the disease or condition, or to partially or fully reverse the effects of the disease. To "treat" can also refer to non-pharmacological methods of preventing or delaying a worsening of the effects of the disease or condition, or to partially or fully reversing the effects of the disease. For example, "treat" is meant to mean a course of action to prevent or delay a worsening of the effects of the disease or condition, or to partially or fully reverse the effects of the disease other than by administering a compound. For example, "treat" or "treatment" can include, but is not limited to, changes in diet, nutritional supplements, cognitive behavioral therapy, support groups, desensitization therapy, guided imagery, counseling, physical therapy, occupational therapy, chiropractic therapy, massage therapy, electroconvulsive therapy (ECT), surgery, anesthesia, psychotherapy, acupuncture, hypnosis, graded exercise routines, aerobic training, strength training,Yoga, Tai Chi, breath training, relaxation training, meditation, etc.

By "prevent" is meant to minimize the chance that a subject who has a susceptibility for developing a pathological muscle/fatigue condition will develop a pathological muscle/fatigue condition or one or more symptoms associated with a pathological muscle/fatigue condition.

By "specifically binds" is meant that an antibody recognizes and physically interacts with its cognate antigen (for example, a ASIC, P2X5, P2X4, and TRPV1 receptor) and does not significantly recognize and interact with other antigens; such an antibody may be a polyclonal antibody or a monoclonal antibody, which are generated by techniques that are well known in the art.

By "probe," "primer," or oligonucleotide is meant a single-stranded DNA or RNA molecule of defined sequence that can base-pair to a second DNA or RNA molecule that contains a complementary sequence (the "target"). The stability of the resulting hybrid depends upon the extent of the base-pairing that occurs. The extent of base-pairing is affected by parameters such as the degree of complementarity between the probe and target molecules and the degree of stringency of the hybridization conditions. The degree of hybridization stringency is affected by parameters such as temperature, salt concentration, and the concentration of organic molecules such as formamide, and is determined by methods known to one skilled in the art. Probes or primers specific for c-Met nucleic acids (for example, genes and/or mRNAs) have at least 80%-90% sequence complementarity, preferably at least 91%-95% sequence complementarity, more preferably at least 96%-99% sequence complementarity, and most preferably 100% sequence complementarity to the region of the c-Met nucleic acid to which they hybridize. Probes, primers, and oligonucleotides may be detectably-labeled, either radioactively, or non-radioactively, by methods well-known to those skilled in the art. Probes, primers, and oligonucleotides are used for methods involving nucleic acid hybridization, such as: nucleic acid sequencing, reverse transcription and/or nucleic acid amplification by the polymerase chain reaction, single stranded conformational polymorphism (SSCP) analysis, restriction fragment polymorphism (RFLP) analysis, Southern hybridization, Northern hybridization, in situ hybridization, electrophoretic mobility shift assay (EMSA).

By "specifically hybridizes" is meant that a probe, primer, or oligonucleotide recognizes and physically interacts (that is, base-pairs) with a substantially complementary nucleic acid (for example, a c-met nucleic acid) under high stringency conditions, and does not substantially base pair with other nucleic acids.

By "high stringency conditions" is meant conditions that allow hybridization comparable with that resulting from the use of a DNA probe of at least 40 nucleotides in length, in a buffer containing 0.5 M NaHPO₄ pH 7.2, 7% SDS, 1 mM EDTA, and 1% BSA (Fraction V), at a temperature of 65°C, or a buffer containing 48% formamide, 4.8X SSC, 0.2 M Tris-Cl, pH 7.6, 1X Denhardt's solution, 10% dextran sulfate, and 0.1% SDS, at a temperature of 42°C. Other conditions for high stringency hybridization, such as for PCR, Northern, Southern, or in situ hybridization, DNA sequencing, etc., are well-known by those skilled in the art of molecular biology. (See, for example, F. Ausubel et al., Current Protocols in Molecular Biology, John Wiley & Sons, New York, NY, 1998).

### COMPOSITIONS

The present invention comprises methods and compositions for effecting molecular receptors that work alone or in combination to detect metabolites produced by muscle contraction and muscle injury. These receptors include, but are not limited to, an ASIC receptor (including, but not limited to all isoforms, including ASIC1a, ASIC1b, ASIC2a, ASIC2b, ASIC3, and ASIC4) a P2X receptor (including, but not limited to P2X1, P2X2, P2X3, P2X4, P2X5, P2X6, and P2X7, for example P2X5 for moderate metabolite levels and P2X4 for high levels of metabolites) and a TRPV receptors (including, but not limited to TRPV1, TRPV2, TRPV3, TRPV4, TRPV5, and TRPV6). For example, increased levels of ASIC, P2X, and/or TRPV receptors can be indicative of a pathological muscle/fatigue condition. In addition, evaluating the levels or concentrations of other molecular receptors including addition metabolite detecting, adrenergic function or immune function genes or peptides can also be used in the methods described herein.

The present invention relates, in part to genes and peptides that can be used as biomarkers for pathological muscle/fatigue conditions. For example, one or more metabolite detecting, adrenergic function or immune function genes or peptides can be used as biomarkers for pathological muscle/fatigue conditions.

A metabolite detecting peptide can be a metabolite detecting receptor. Metabolite detecting receptors can be gated ion channels. For example, metabolite detecting receptors can be gated ion channels that respond to metabolite increases in skeletal muscle. Metabolite detecting receptors include members of the gated ion channel family that can be comprised of at least one subunit selected from the group consisting of, but not limited to, a member of the DEG/ENaC, P2X, and TRP gene superfamilies.

Metabolite detecting receptors can comprise at least one subunit selected from the group consisting of, but not limited to, αENaC, βENaC, γENaC, δENaC, ASIC1a, ASIC1b, ASIC2a, ASIC2b, ASIC3, ASIC4, BLINaC, hINaC, P2X₁, P2X₂, P2X₃, P2X₄, P2X₅, P2X₆, P2X₇, TRPV1, TRPV2, TRPV3, TRPV4, TRPV5, TRPV6, TRPA1 and TRPM8.

Furthermore, metabolite detecting receptors can be homomultimeric or heteromultimeric. The heteromultimeric metabolite detecting receptors can include but are not limited to the following combinations: αENaC, βENaC and γENaC; αENaC, βENaC and δENaC; ASIC1a and ASIC3; ASIC1b and ASIC3; ASIC2a and ASIC3; ASIC2b and ASIC3; ASIC1a, ASIC2a and ASIC3; P2X₁ and P2X₂; P2X₁ and P2X₅; P2X₂ and P2X₃; P2X₂ and P2X₆; P2X₄ and P2X₆; P2X₄ and P2X₁, P2X₄ and P2X₂, TRPV1 and TRPV2; TRPV5 and TRPV6; and TRPV1 and TRPV4, as well as ASIC1a and ASIC2a; ASIC2a and ASIC2b; ASIC1b and ASIC3; and ASIC3 and ASIC2b.

The physiological and pathophysiological function of ASICs or their subunit composition in different cells and tissues are poorly understood, however, their location (mostly neuronal) and gating properties make them an attractive candidate to serve as an acid sensor for pH nociception to convey pain sensation during conditions such as inflammation, ischemia, hematoma, infection and other conditions known to produce tissue acidosis.

Knowledge of the tissue, cellular, and subcellular distributions of these channels can provide important clues as to their function. In the peripheral nervous system (PNS) under physiological conditions, ASICs subunits are found in primary sensory neurons that innervate the skin (Price M P, et al. (2000) Nature 407:1007-1011; Price M P, et al. (2001) Neuron 32:1071-1083), heart (Benson C J, et al. (1999) Circ Res 84:921-928), gastrointestinal tracks (Page A J, et al. (2005b) Gut 54:1408-1415; Page A J, et al. (2004) Gastroenterology 127:1739-1747; Page A J, et al. (2005a) Gut 54:1408-1415), and muscles (Molliver D C, et al. (2005) Mol Pain 1:35). ASICs subunits are also found in the eye (Ettaiche M, et al. J Neurosci 24:1005-1012), ear (Hildebrand M S, et al. (2004) Hear Res 190:149-160), tongue (Ugawa S (2003) Anat Sci Int 78:205-210; Ugawa S, et al. J Neurosci 23:3616-3622), lungs (Gu Q et al. (2006) Am J Physiol Lung Cell Mol Physiol 291:L58-L65), and bones (Jahr H, et al. (2005) Biochem Biophys Res Commun 337:349-354).

Research has shown that ASICs play a role in pain and inflammation. For example, it has been shown that ASICs play a role in pain sensation (Price, M. P. et al., Neuron. 2001; 32(6): 1071-83; Chen, C. C. et al., Neurobiology 2002; 99(13) 8992-8997), including visceral and somatic pain (Aziz, Q., Eur. J. Gastroenterol. Hepatol. 2001; 13(8):891-6); chest pain that accompanies cardiac ischemia (Sutherland, S. P. et al. (2001) Proc Natl Acad Sci USA 98:711-716), and chronic hyperalgesia (Sluka, K. A. et al., Pain. 2003; 106(3):229-39). Recent studies done in humans indicate that ASICs are the primary sensors of acid-induced pain (Ugawa et al., J. Clin. Invest. 2002; 110: 1185-90; Jones et al., J. Neurosci. 2004; 24: 10974-9).

There are seven presently known members of the P2X gene superfamily; P2X₁ (also known as P2RX₁), P2X₂ (also known as P2RX₂), P2X₃ (also known as P2RX₃), P2X₄ (also known as P2RX₄), P2X₅ (also known as P2RX₅), P2X₆ (also known as P2RX₆), and P2X₇ (also known as P2RX₇). P2X protein structure is similar to ASIC protein structure in that they contain two transmembrane spanning domains, intracellular N- and C-termini and a cysteine-rich extracellular loop. In addition, functional P2X receptors, like ASIC receptors can be formed from trimers of individual proteins (Carnally et al., 2008; Jasti et al., 2007; Young et al., 2008). All P2X receptors open in response to the release of extracellular ATP and are permeable to small ions and some have significant calcium permeability. P2X receptors are abundantly distributed on neurons, glia, epithelial, endothelial, bone, muscle and hematopoietic tissues. For a recent review on this gene superfamily, see North, R. A. (2002) Physiol. Rev. 82:1013, incorporated herein by reference.

The P2X receptors can be found in the central and peripheral nervous systems, and studies on the localization of P2X receptors have found P2RX1-P2RX6 in nervous structures involved in nociceptive transmission (reviewed in Chizh and Liles, 2001, Pharmacol Rev. 53:553-568). In contrast to the distribution of the rat P2RX3 transcript, which was detected exclusively in the sensory neurons of trigeminal, dorsal root and nodose ganglia (Chen et al., 1995, Nature 377:428-431), the human P2RX3 transcript was detected in human spinal cord and heart. Expression analysis in both rat and human tissues has determined wide tissue distribution of P2RX4 transcripts in addition to the brain (Soto et al., 1996, Proc. Natl. Acad. Sci. USA 93:3684-3688; Garcia-Guzman et al., 1997, Mol. Pharmacol. 51:109-118).

To date, the transient receptor potential (TRP) superfamily consists of 28 non-selective cation channels subdivided into six main subfamilies: the TRPC (canonical), TRPV (vanilloid), TRPM (melastatin), TRPP (polycystin), TRPML (mucolipin), and the TRPA (ankyrin) groups. The great majority of functionally characterized TRP channels are permeable to Ca²+. The TRP channels are widely distributed and participate in various cellular functions. Although our understanding of the physiological and pathophysiological involvement of many of these channels is limited, evidence exists (e.g., changes in expression levels) that link some of these channels to several diseases. TRP channels play also a role in some systemic reactions and diseases provoked by specific irritants, inflammation mediators, and foreign toxins.

Adrenergic receptors are integral membrane proteins which have been classified into two broad classes, the alpha and the beta adrenergic receptors. The cloning, sequencing and expression of alpha receptor subtypes from animal tissues has led to the subclassification of the α₁.adrenoreceptors into α_{1A}, α_{1B}, and α_{1D}. Similarly, the α₂ adrenoreceptors have also been classified α_{2A}, α_{2B}, and α_{2C} receptors. Each α₂ receptor subtype appears to exhibit its own pharmacological and tissue specificities.

Both the α and β receptor classes of adrenergic receptors mediate the action of the peripheral sympathetic nervous system upon binding of catecholamines, norepinephrine and epinephrine. Norepinephrine is produced by adrenergic nerve endings, while epinephrine is produced by the adrenal medulla. The binding affinity of adrenergic receptors for these compounds forms one basis of the classification: α receptors tend to bind norepinephrine more strongly than epinephrine and much more strongly than the synthetic compound isoproterenol. The preferred binding affinity of these hormones is reversed for the β receptors. In many tissues, the functional responses, such as smooth muscle contraction, induced by alpha receptor activation are opposed to responses induced by β receptor binding.

Among other indications, such as the treatment of glaucoma, hypertension, sexual dysfunction, and depression, adrenergic receptors appear to play an important role in the modulation of pain. There is now abundant evidence that activation of adrenergic receptors can, in certain circumstances, generate impressive analgesic effects. However, under other circumstances, adrenergic receptors can also contribute to chronic neuropathic pain and hyperalgesia. There is also evidence that adrenergic receptors are involved in fatigue as well as pain modulation.

As described herein the term "immune function gene" refers to a gene capable of encoding a peptide related to immune function. For example, an immune function gene can be capable of encoding an immune cytokine or receptor. Immune function genes include, but are not limited to members of the Toll-like receptors (TLRs) family, which include, but are not limited to TLR1, TLR2, TLR3, TLR4, TLR5, TLR6, TLR7, TLR8, TLR9, TLR10, TLR11, TLR12 and TLR13. Immune function genes also include, but are not limited to Interleukins (IL), a group of cytokines that were first seen to be expressed by white blood cells as a means of communication. Members of the interleukin family include, but are not limited to, IL1, IL2, IL3, IL4, IL5, IL6, IL7, IL8, IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL34 and IL35. Immune function genes also include members of the tumor necrosis factor (TNF) family, a family of cytokines that can cause cell death. TNF family members include, but are not limited to, TNFα and TNFβ. Immune function genes also include the Cluster of differentiation 14 also known as CD 14 that is a human gene. The protein encoded by this gene is a component of the innate immune system. CD 14 exists in two forms. It is either anchored into the membrane by a glycosylphosphatidylinositol tail (mCD14) or it appears in a soluble form (sCD14). Soluble CD14 appears either after shedding of mCD14 (48 KDa) or is directly secreted from intracellular vesicles (56 KDa). CD14 takes its name from its inclusion in the cluster of differentiation group of cell surface marker proteins. CD14 acts as a co-receptor (along with the Toll-like receptor TLR 4 and MD-2) for the detection of bacterial lipopolysaccharide (LPS). CD14 can only bind LPS in the presence of lipopolysaccharide-binding protein (LBP). Although LPS is considered its main ligand CD14 also recognizes other pathogen associated molecular patterns.

Local immune system activation begins when damaged cells secrete signals that recruit immune system cells to the area. One type of recruited immune system cell is the macrophage. Macrophages release interleukin-1 beta ("IL-β") and tumor necrosis factor alpha ("TNFα"), pro-inflammatory cytokines heavily involved in orchestrating the immediate and local physiological effects of injury or infection. For instance, once released, pro-inflammatory cytokines promote inflammation (swelling and redness caused by increased blood flow to the area which delivers recruited immune system cells more quickly) and also increase sensitivity to pain (by increasing the excitability and transmission of sensory nerves carrying pain information to the brain). Thus, pro-inflammatory cytokines are involved in the beneficial physiological and recuperative effects of acute pain.

Disclosed herein are solid supports comprising one or more primers, probes, polypeptides, or antibodies capable of hybridizing or binding to one or more of the metabolite detecting, adrenergic function, or immune function genes or peptides described herein attached to the solid support. For example, an aspect of the present invention comprises solid supports comprising one or more primers, probes, polypeptides, or antibodies capable of hybridizing or binding to one or more metabolite detecting, adrenergic function, or immune function genes described herein attached to the solid support.

Solid supports are solid-state substrates or supports with which molecules, such as analytes and analyte binding molecules, can be associated. Analytes, such as calcifying nano-particles and proteins, can be associated with solid supports directly or indirectly. For example, analytes can be directly immobilized on solid supports. Analyte capture agents, such a capture compounds, can also be immobilized on solid supports. For example, disclosed herein are antigen binding agents capable of specifically binding to ASIC, P2X and TRPV1 receptors.

A form of solid support is an array. Another form of solid support is an array detector. An array detector is a solid support to which multiple different capture compounds or detection compounds have been coupled in an array, grid, or other organized pattern.

Solid-state substrates for use in solid supports can include any solid material to which molecules can be coupled. This includes materials such as acrylamide, agarose, cellulose, nitrocellulose, glass, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, polylactic acid, polyorthoesters, polypropylfumerate, collagen, glycosaminoglycans, and polyamino acids. Solid-state substrates can have any useful form including thin film, membrane, bottles, dishes, fibers, woven fibers, shaped polymers, particles, beads, microparticles, or a combination. Solid-state substrates and solid supports can be porous or non-porous. A form for a solid-state substrate is a microtiter dish, such as a standard 96-well type. In preferred embodiments, a multiwell glass slide can be employed that normally contain one array per well. This feature allows for greater control of assay reproducibility, increased throughput and sample handling, and ease of automation.

Different compounds can be used together as a set. The set can be used as a mixture of all or subsets of the compounds used separately in separate reactions, or immobilized in an array. Compounds used separately or as mixtures can be physically separable through, for example, association with or immobilization on a solid support. An array can include a plurality of compounds immobilized at identified or predefined locations on the array. Each predefined location on the array generally can have one type of component (that is, all the components at that location are the same). Each location will have multiple copies of the component. The spatial separation of different components in the array allows separate detection and identification of the polynucleotides or polypeptides disclosed herein.

It is not required that a given array be a single unit or structure. The set of compounds may be distributed over any number of solid supports. For example, at one extreme, each compound may be immobilized in a separate reaction tube or container, or on separate beads or microparticles. Different modes of the disclosed method can be performed with different components (for example, different compounds specific for different proteins) immobilized on a solid support.

Some solid supports can have capture compounds, such as antibodies, attached to a solid-state substrate. Such capture compounds can be specific for calcifying nano-particles or a protein on calcifying nano-particles. Captured calcifying nano-particles or proteins can then be detected by binding of a second, detection compound, such as an antibody. The detection compound can be specific for the same or a different protein on the calcifying nano-particle.

Methods for immobilizing antibodies (and other proteins) to solid-state substrates are well established. Immobilization can be accomplished by attachment, for example, to aminated surfaces, carboxylated surfaces or hydroxylated surfaces using standard immobilization chemistries. Examples of attachment agents are cyanogen bromide, succinimide, aldehydes, tosyl chloride, avidin-biotin, photocrosslinkable agents, epoxides and maleimides. A preferred attachment agent is the heterobifunctional cross-linker N-[γ-Maleimidobutyryloxy] succinimide ester (GMBS). These and other attachment agents, as well as methods for their use in attachment, are described in Protein immobilization: fundamentals and applications, Richard F. Taylor, ed. (M. Dekker, New York, 1991); Johnstone and Thorpe, Immunochemistry In Practice (Blackwell Scientific Publications, Oxford, England, 1987) pages 209-216 and 241-242, and Immobilized Affinity Ligands; Craig T. Hermanson et al., eds. (Academic Press, New York, 1992) which are incorporated by reference in their entirety for methods of attaching antibodies to a solid-state substrate. Antibodies can be attached to a substrate by chemically cross-linking a free amino group on the antibody to reactive side groups present within the solid-state substrate. For example, antibodies may be chemically cross-linked to a substrate that contains free amino, carboxyl, or sulfur groups using glutaraldehyde, carbodiimides, or GMBS, respectively, as cross-linker agents. In this method, aqueous solutions containing free antibodies are incubated with the solid-state substrate in the presence of glutaraldehyde or carbodiimide.

A method for attaching antibodies or other proteins to a solid-state substrate is to functionalize the substrate with an amino- or thiol-silane, and then to activate the functionalized substrate with a homobifunctional cross-linker agent such as (Bis-sulfo-succinimidyl suberate (BS³) or a heterobifunctional cross-linker agent such as GMBS. For cross-linking with GMBS, glass substrates are chemically functionalized by immersing in a solution of mercaptopropyltrimethoxysilane (1% vol/vol in 95% ethanol pH 5.5) for 1 hour, rinsing in 95% ethanol and heating at 120 °C for 4 hrs. Thiol-derivatized slides are activated by immersing in a 0.5 mg/ml solution of GMBS in 1% dimethylformamide, 99% ethanol for 1 hour at room temperature. Antibodies or proteins are added directly to the activated substrate, which are then blocked with solutions containing agents such as 2% bovine serum albumin, and air-dried. Other standard immobilization chemistries are known by those of skill in the art.

Each of the components (compounds, for example) immobilized on the solid support preferably is located in a different predefined region of the solid support. Each of the different predefined regions can be physically separated from each other of the different regions. The distance between the different predefined regions of the solid support can be either fixed or variable. For example, in an array, each of the components can be arranged at fixed distances from each other, while components associated with beads will not be in a fixed spatial relationship. In particular, the use of multiple solid support units (for example, multiple beads) will result in variable distances.

Components can be associated or immobilized on a solid support at any density. Components preferably are immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

Optionally, at least one address on the solid support can be a sequence that encodes one or more of the metabolite detecting, adrenergic function or immune function peptides disclosed herein. Disclosed are solid supports where at least one address is the sequences or portion of sequences set forth in any of the peptide sequences disclosed herein. Solid supports can also contain at least one address is a variant of the sequences or part of the sequences set forth in any of the nucleic acid sequences disclosed herein. Solid supports can also contain at least one address is a variant of the sequences or portion of sequences set forth in any of the peptide sequences disclosed herein.

Disclosed are antigen microarrays for multiplex characterization of antibody responses. For example, disclosed are antigen arrays and miniaturized antigen arrays to perform large-scale multiplex characterization of antibody responses directed against the polypeptides, polynucleotides and antibodies described herein, using submicroliter quantities of biological samples as described in Robinson et al., Autoantigen microarrays for multiplex characterization of autoantibody responses, Nat Med., 8(3):295-301 (2002), which in herein incorporated by reference in its entirety for its teaching of contructing and using antigen arrays to perform large-scale multiplex characterization of antibody responses directed against structurally diverse antigens, using submicroliter quantities of biological samples.

In addition, the receptors described herein may be used as markers for presence or progression of pathological muscle/fatigue conditions. The methods and assays described elsewhere herein may be performed over time, and the change in the level of reactive polypeptide(s) or polynucleotide(s) evaluated. For example, the assays may be performed every 24-72 hours for a period of 6 months to 1 year, and thereafter performed as needed. Assays can be performed prior to, during, or after exercise. For example, immunoreactivity to a given polypeptide in individuals with or without a pathological muscle/fatigue condition can correlate with or predict the development of complications such as fatigue or pain, or the more severe activity of disease

As noted herein, to improve sensitivity, multiple mutations may be assayed within a given sample. Binding agents specific for different proteins, antibodies, nucleic acids thereto provided herein may be combined within a single assay. Further, multiple primers or probes may be used concurrently. The selection of receptors may be based on routine experiments to determine combinations that results in optimal sensitivity. To assist with such assays, specific biomarkers can assist in the specificity of such tests. As such, disclosed herein is a biomarker, wherein the biomarker is capable of binding to or hybridizing with a metabolite detecting, adrenergic function or immune function gene or peptide.

The biomarkers described herein can be in any form that provides information regarding presence or absence of a pathological muscle/fatigue condition or of fatigue or pain. For example, the disclosed biomarkers can be, but is not limited to a nucleic acid molecule, a polypeptide, or an antibody.

### Methods

The disclosed gene and peptides, including metabolite detecting, adrenergic function and immune function genes and peptides disclosed herein can be used in a variety of different methods, for example in prognostic, predictive, diagnostic, and therapeutic methods and as a variety of different compositions.

Also, disclosed herein are sensitive methods for detection and diagnosis of pathological muscle/fatigue condition by assessing metabolite detecting, adrenergic function or immune function gene expression in a subject. An increase in metabolite detecting gene expression in a subject that is significantly above the metabolite detecting gene expression from a normal subject indicates a strong likelihood of pathological muscle/fatigue condition.

Also, disclosed herein are sensitive methods for detection and diagnosis of pathological muscle/fatigue condition by assessing metabolite detecting, adrenergic function or immune function peptide concentration in a subject. An increase in metabolite detecting peptide concentration in a subject that is significantly above the metabolite detecting gene expression from a normal subject indicates a strong likelihood of pathological muscle/fatigue condition.

"Concentration" or "levels" of metabolite detecting, adrenergic function or immune function peptides refers to either the molecular or physiological amounts of the receptors. For example, the concentration or level of ASIC, P2X5, P2X4, and/or TRPV1 receptors can refer to mRNA, DNA, cDNA, or protein concentrations or levels. The concentration or level of metabolite detecting, adrenergic function or immune function peptides can also refer to the number of polypeptides, proteins, or receptors present at a given time.

Disclosed herein are methods of detecting a pathological muscle/fatigue condition in a sample, comprising determining the expression level of one or more metabolite detecting genes in a sample and comparing those expression levels to the expression levels of a normal sample. Disclosed are methods of detecting a pathological muscle/fatigue condition in a sample comprising determining the expression level of one or more metabolite detecting genes in a sample and comparing those expression levels to the expression levels of a normal sample, wherein an increase in the expression level of the metabolite detecting genes above the expression levels in the normal sample indicates the presence of a pathological muscle/fatigue condition.

Disclosed are methods of detecting a pathological muscle/fatigue condition in a sample, comprising determining the expression level of one or more metabolite detecting genes in a sample and comparing those expression levels to the expression levels of a normal sample, further comprising determining the expression level of one or more adrenergic genes in a sample and comparing those levels to a normal sample. Disclosed are methods of detecting a pathological muscle/fatigue condition in a sample comprising determining the expression level of one or more metabolite detecting genes in a sample and comparing those expression levels to the expression levels of a normal sample, further comprising determining the expression level of one or more adrenergic genes in a sample and comparing those levels to a normal sample, wherein an increase in the expression level of one or more adrenergic genes above the levels in the normal sample indicates the presence of a pathological muscle/fatigue condition.

Disclosed herein are methods of detecting a pathological muscle/fatigue condition in a sample, comprising determining the expression level of one or more metabolite detecting genes in a sample and comparing those expression levels to the expression levels of a normal sample, further comprising determining the expression level of one or more immune mediated genes in a sample and comparing those levels to a normal sample.

Disclosed herein are methods of detecting a pathological muscle/fatigue condition in a sample, comprising determining the expression level of one or more metabolite detecting genes in a sample and comparing those expression levels to the expression levels of a normal sample, further comprising determining the expression level of one or more immune mediated genes in a sample and comparing those levels to a normal sample, wherein an increase in the expression level of one or more immune mediated genes above the levels in the normal sample indicates the presence of a pathological muscle/fatigue condition.

For example, disclosed herein are methods of detecting a pathological muscle/fatigue condition, pain, or fatigue in a sample comprising determining the expression level of one or more metabolite detecting genes in a sample and comparing those expression levels to the expression levels of a normal sample, wherein the expression level of one or more metabolite detecting genes or peptides is increased by 10%, 15%, 20%, 25%, 30%, 35%, 40%, 45%, 50%, 55%, 60%, 65%, 70%, 75%, 80%, 85%, 90%, 95%, or 100% when compared to the expression level of a "normal" subject is indicative of a pathological muscle/fatigue condition. In addition, the expression level of one or more adrenergic function genes or peptides can be increased by 10%, 15%, 20%, 25%, 30%, 35%, 40%, 45%, 50%, 55%, 60%, 65%, 70%, 75%, 80%, 85%, 90%, 95%, or 100% when compared to the expression level of a "normal" subject is indicative of a pathological muscle/fatigue condition. In combination with the metabolite detecting or in combination with the metabolite detecting and adrenergic gene or peptide expression, the expression level of one or more immune function genes or peptides can be increased by 10%, 15%, 20%, 25%, 30%, 35%, 40%, 45%, 50%, 55%, 60%, 65%, 70%, 75%, 80%, 85%, 90%, 95%, or 100% when compared to the expression level of a "normal" subject is indicative of a pathological muscle/fatigue condition.

An increase in the expression level of all metabolite detecting, adrenergic function or immune function genes or peptides is not always required to indicate a pathological muscle/fatigue condition. There can be signature patterns of increased or decreased expression levels of one or more metabolite detecting, adrenergic function or immune function genes or peptides.

For example, an increase in the expression level of ASIC3, P2X4 and P2X5 and a decrease in TRPV1 can be indicative of fatigue in a subject with a pathological muscle/fatigue condition. In addition to such an expression pattern, there can also be an increase of α2a, β-1, β-2, and COMT expression as well as an increase in TLR4, CD14 and IL-10 accompanied by a decrease in IL-6 and TNF-αAUC expression.

Other expression results can be correlated to other symptoms of pathological muscle/fatigue conditions. For example, P2X4, P2X5 and β-1 have been found to not be associated with the symptom of pain in a subject with a pathological muscle/fatigue condition whereas increases in the expression of ASIC3, TRPV1, α2a, β-2, and IL-10 are associated with the symptom of pain.

An aspect of the invention is the assessment of the risk of developing a pathological muscle/fatigue condition by comparing the metabolite detecting, adrenergic function or immune function gene or peptide levels in a subject with the levels of a normal subject. When the metabolite detecting gene or peptide levels in a subject are higher than levels from a normal subject, the subject is considered at high-risk for the development of a pathological muscle/ fatigue condition and should seek further diagnosis for pathological muscle/fatigue condition.

Disclosed herein, are methods of detecting pathological muscle/fatigue condition in a sample comprising determining whether the sample comprises an increase in the metabolite detecting, adrenergic function or immune function gene or peptide when compared to levels in a normal sample.

Disclosed herein are methods of determining responsiveness of a pathological muscle/ fatigue condition in a subject to treatment with a pharmaceutical agent, said method comprising, administering the pharmaceutical agent to the subject, determining the expression level of adrenergic and immune function genes in a sample and comparing those expression levels to the expression levels of the subject prior to administering the pharmaceutical agent, wherein a decrease in the expression level of one or more adrenergic and immune function genes in the subject after administering the metabolite detecting receptor inhibitor to the subject indicates responsiveness to the pharmaceutical agent. A pharmaceutical agent can be an agent or compound used to treat, modulate or ameliorate one or more of the symptoms associated with a pathological fatigue condition. A pharmaceutical agent can also be an agent or compound used to treat, modulate or ameliorate fatigue or pain in a subject without a pathological fatigue condition

For example, a pharmaceutical agent can be any of the compounds disclosed in U.S. Patent Application No. 2009/0023773.

Disclosed herein are methods of determining responsiveness of a pathological muscle/ fatigue condition in a subject to treatment with a non-pharmacological treatment such as cognitive behavioral therapy, said method comprising, conducting or exposing the subject to a non-pharmacological treatment, determining the expression level of adrenergic and immune function genes in a sample and comparing those expression levels to the expression levels of the subject prior to conducting or exposing the subject to a non-pharmacological treatment, wherein a decrease in the expression level of one or more adrenergic and immune function genes in the subject after administering the metabolite detecting receptor inhibitor to the subject indicates responsiveness to the non-pharmacological treatment. A non-pharmaceutical treatment can be behavior-based interventions or non-pharmacological medical interventions. Behavior-based and non-pharmacological medical interventions include, but are not limited to, changes in diet, nutritional supplements, cognitive behavioral therapy, support groups, desensitization therapy, guided imagery, counseling, physical therapy, occupational therapy, chiropractic therapy, massage therapy, electroconvulsive therapy (ECT), surgery, anesthesia, psychotherapy, acupuncture, hypnosis, graded exercise routines, aerobic training, strength training,Yoga, Tai Chi, breath training, relaxation training, meditation, etc.

The method described herein can also be used outside a pathological muscle/fatigue condition. The receptors, including ASIC, P2X, and TRPV receptors disclosed herein can be used in a variety of different methods, for example in prognostic, predictive, diagnostic, and therapeutic methods and as a variety of different compositions in otherwise normal or healthy individuals. For example, disclosed herein, are methods of detecting muscle fatigue or a muscle pain condition in a subject or in a sample comprising determining whether the subject or sample comprises an increase in metabolite detecting, adrenergic function or immune function gene or peptide when compared to levels in a normal sample.

The methods described herein can also be used to determine fatigue in normal subjects. For example, the methods described herein can be used to determine fatigue in subjects including healthy individuals, non-professional athletes, competitive sports athletes, Olympic athletes, and professional athletes in training by determining the levels of metabolite detecting, immune function or adrenergic function gene expression before and after a training session. If the athletes continue heavy training without rest, fatigue builds up and may result in injury when a level of the fatigue exceeds their fatigue limit. Athletes are more likely to develop a physical disorder sometimes referred to as an overtraining syndrome under such conditions. The amount of training, the amount of rest, and the performance of athletes during training are usually used as indexes for determination of overtraining. However, it is difficult to detect overtraining based on these indexes because an ability to cope with fatigue, a fatigue limit, or a level of recovery differs from athlete to athlete and the overtraining determination greatly depends on experiences of trainers. Therefore, the disclosed methods would be useful in preventing overtraining by determining the athlete's fatigue limit and thereby preventing serious injury.

Also disclosed herein are methods that can be used to evaluate the efficacy of various clinical interventions. Measurements of the levels of metabolite detecting, immune function or adrenergic function gene expression before and after clinical treatment may be an objective measurement of the efficacy of clinical interventions. In medical fields such as physical therapy or occupational therapy, mechanical devices are used to evaluate and/or increase the strength or dexterity of the subject. The methods disclosed herein can be used independently or in conjunction with any mechanical system in order to determine the fatigue limit of the subject.

The methods disclosed herein also provide for quantification of fatigue in order to prevent repetitive stress injury (RSI). RSI is a major problem facing clinicians and society. Carpal Tunnel Syndrome (CTS), which is one form of RSI, is a significant health problem in the workplace today. The U.S. Department of Labor has concluded that CTS is the "chief occupational hazard disabling workers in epidemic proportions." As society gets older, the incidence of RSI will increase. In addition, as we urge all children to become computer literate, RSI will become a major problem with the youth. The disclosed methods can quantify the degree of impairment of subjects suffering from CTS would be useful in the medical field. The most practical non-evasive method is to evaluate the fatigability of the subject while conducting a standard repetitive task. Fatigability can be evaluated by determining the levels of metabolite by detecting, immune function and adrenergic function, gene expression before and after a task (e.g. exercise). Such a method would be useful to physicians, and physical and occupational therapists to evaluate patients suffering from RSI before and after clinical interventions. Presently, surgical intervention is used to minimize CTS. However, if the patient begins to repeat the same behavior that produced the problem, then the person may develop CTS again.

Disclosed herein are methods of treating a pathological muscle/fatigue condition comprising administering an effective amount of one or more ASIC, P2X, and/or TRPV receptor antagonists. Disclosed are methods of treating a pathological muscle/fatigue condition comprising administering an effective amount of one or more of one or more ASIC, P2X, and/or TRPV receptor antagonists in conjunction with other fatigue therapies. Disclosed herein are methods of treating a pathological muscle/fatigue condition comprising administering an effective amount of one or more ASIC, P2X, and/or TRPV receptor inhibitors. Disclosed are methods of treating a pathological muscle/fatigue condition comprising administering an effective amount of one or more of one or more ASIC, P2X, and/or TRPV receptor inhibitors in conjunction with other fatigue therapies.

Disclosed herein are methods of treating or preventing muscle pain and/or fatigue comprising administering an effective amount of one or more ASIC, P2X, and/or TRPV receptor antagonists. Disclosed are methods of treating or preventing muscle pain and/or fatigue comprising administering an effective amount of one or more of one or more ASIC, P2X, and/or TRPV receptor antagonists in conjunction with other fatigue and/or pain therapies. Disclosed herein are methods of treating or preventing muscle pain and/or fatigue comprising administering an effective amount of one or more ASIC, ASIC, P2X, and/or TRPV receptor inhibitors. Disclosed are methods of treating or preventing muscle pain and/or fatigue comprising administering an effective amount of one or more of one or more ASIC, P2X, and/or TRPV receptor inhibitors in conjunction with other fatigue and/or therapies.

Disclosed herein are methods for treating a subject with a pathological muscle/fatigue condition, the method comprising administering to the subject in need thereof an effective amount of one or more metabolite detecting peptide antagonists as described herein in an amount sufficient to ameliorate or modultate one or more symptoms of said condition.

Disclosed herein are methods of ameliorating or modulating one or more symptoms associated with a pathological muscle/fatigue condition comprising administering to a subject in need thereof an effective amount of one or more metabolite detecting peptide antagonists as described herein.

Disclosed herein are methods of treating pain comprising administering to a subject in need thereof an effective amount of one or more metabolite detecting peptide antagonists as described herein in an amount sufficient to ameliorate or modulate pain.

Disclosed herein are methods of treating fatigue comprising administering to a subject in need thereof an effective amount of one or more metabolite detecting peptide antagonists as described herein in an amount sufficient to ameliorate or modulate fatigue.

The terms "antagonist" or "inhibitor", as used herein, refer to a molecule or a combination of molecules, which modulates or blocks directly or indirectly a biological activity of a gene or peptide. Antagonists and inhibitors may include proteins, nucleic acids, aptamers, carbohydrates, or any other molecules which display the properties described herein above.

The term "agonist", as used herein, refers to a molecule or a combination of molecules, which modulates or induces directly or indirectly a biological activity of an endogenous gene or peptide. Agonists may include proteins, nucleic acids, aptamers, carbohydrates, or any other molecules, which display the properties described herein above.

The term "modulate", as used herein, refers to a change or an alteration in the biological activity of a gene or peptide. Modulation may be an increase or a decrease in peptide activity, a change in binding characteristics, or any other change in the biological, functional or immunological properties of the peptide.

ASIC receptor antagonists include, but are not limited to, amiloride, A-317567, PPC-5650, *Psalmopoeus* venom. P2X5 and P2X4 receptor antagonists include, but are not limited to, TNP-ATP and PPADS. TRPV1 receptor antagonists include, but are not limited to LJO-328.

Additional modulators of metabolite detecting receptors are disclosed and described in U.S. Patent Application No. 2009/0023773, which is hereby incorporated by reference in its entirety for its teaching of the same. For example, the gated ion channel modulators disclosed and described in US Patent Application No. 2009/0023773 can be used to treat/ameliorate diseases and disorders related to pain, inflammation, the neurological system, the gastrointestinal system and genitourinary system. Gated ion channel modulators include, but are not limited to, endogenous extracellular ligands such as anandamide, ATP, glutamate, cysteine, glycine, gamma-aminobutyric acid (GABA), histamine, adenosine, serotonin (5HT), acetylcholine, epinephrine, norepinephrine, protons, ions, e.g., Na⁺, Ca⁺+, K⁺, Cl.sup.-, H⁺, Zn⁺, and/or peptides, e.g., Met-enkephaline, Leu-enkephaline, dynorphin, neurotrophins, and/or the RFamide related peptides, e.g., FMRFamide and/or FLRFamide; to endogenous intracellular ligands such as cyclic nucleotides (e.g. cyclicAMP, cyclicGMP), Ca⁺+ and/or G-proteins; to exogenous extracellular ligands or modulators such as α-amino-3-hydroxy-5-methyl-4-isolaxone propionate (AMPA), amiloride, capsaicin, capsazepine, epibatidine, cadmium, barium, gadolinium, guanidium, kainate, N-methyl-D-aspartate (NMDA). Gated ion channels also include complexes responsive to toxins, examples of which include, but are not limited to, Agatoxin (e.g. α-agatoxin IVA, IVB, co-agatoxin IVA, TK), Agitoxins (Agitoxin 2), Apamin, Argiotoxins, Batrachotoxins, Brevetoxins (e.g. Brevetoxin PbTx-2, PbTx-3, PbTx-9), Charybdotoxins, Chlorotoxins, Ciguatoxins, Conotoxins (e.g. α-conotoxin GI, GIA, GII, IMI, MI, MII, SI, SIA, SII, and/or Et; δ-conotoxins, µ-conotoxin GIIIA, GIIIB, GIIIC and/or GS, ω-conotoxin GVIA, MVIIA MVIIC, MVIID, SVIA and/or SVIB), Dendrotoxins, Grammotoxins (GsMTx-4, ω-grammotoxin SIA), Grayanotoxins, Hanatoxins, Iberiotoxins, imperatoxins, Jorotoxins, Kaliotoxins, Kurtoxins, Leiurotoxin 1, Pricotoxins, Psalmotoxins, (e.g., Psalmotoxin 1 (PcTx1)), Margatoxins, Noxiustoxins, Phrixotoxins, PLTX II, Saxitoxins, Stichodactyla Toxins, sea anemone toxins (e.g. APETx2 from Anthopleura elegantissima), Tetrodotoxins, Tityus toxin K-α, Scyllatoxins and/or tubocurarine.

Other therapies used to treat a pathological muscle/fatigue condition or symptom thereof include, but are not limited to, exercise, tricyclic antidepressants (amitriptyline (Limbitrol, a multi-ingredient drug that contains amitriptyline), desipramine (Norpramin) and nortriptyline (Aventyl, Pamelor) and selective serotonin reuptake inhibitors (SSRIs; including, but not limited to fluoxetine (Prozac, Sarafem), paroxetine (Paxil), sertraline (Zoloft) and bupropion (Wellbutrin). Other fatigue therapies include, but are not limited to, Acetaminophen (Tylenol, others) and nonsteroidal anti-inflammatory drugs (NSAIDs), such as aspirin and ibuprofen (Advil, Motrin, others), Antihistamines, such as fexofenadine (Allegra) and cetirizine (Zyrtec), and decongestants that contain pseudoephedrine (Sudafed), fludrocortisone (Florinef), atenolol (Tenormin) and midodrine (ProAmatine, Orvaten), clonazepam (Klonopin), lorazepam (Ativan) and alprazolam (Xanax), Methylphenidate (Ritalin, Concerta), D-ribose (also called ribose) Acupuncture, Corticosteroids, Immune globulins, interferons, Antiviral drugs, and Cholinesterase inhibitors.

Disclosed herein are functional nucleic acids that can interact with the disclosed receptor nucleic acids. Functional nucleic acids are nucleic acid molecules that have a specific function, such as binding a target molecule or catalyzing a specific reaction. Functional nucleic acid molecules can be divided into the following categories, which are not meant to be limiting. For example, functional nucleic acids include antisense molecules, aptamers, ribozymes, triplex forming molecules, and external guide sequences. The functional nucleic acid molecules can act as affectors, inhibitors, modulators, and stimulators of a specific activity possessed by a target molecule, or the functional nucleic acid molecules can possess a de novo activity independent of any other molecules.

Functional nucleic acid molecules can interact with any macromolecule, such as DNA, RNA, polypeptides, or carbohydrate chains. Thus, functional nucleic acids can interact with the mRNA of polynucleotide sequences disclosed herein or the genomic DNA of the polynucleotide sequences disclosed herein or they can interact with the polypeptide encoded by the polynucleotide sequences disclosed herein. Often functional nucleic acids are designed to interact with other nucleic acids based on sequence homology between the target molecule (for example the receptor nucleic acids) and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place.

For example, disclosed herein are antisense molecules that interact with the disclosed receptor nucleic acids and could thus inhibit the expression of the receptors. Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be in vitro selection experiments and DNA modification studies using DMS and DEPC. It is preferred that antisense molecules bind the target molecule with a dissociation constant (k_{d}) less than or equal to 10⁻⁶, 10⁻⁸, 10⁻¹⁰, or 10⁻¹². A representative sample of methods and techniques which aid in the design and use of antisense molecules can be found in the following non-limiting list of United States patents: 5,135,917, 5,294,533, 5,627,158, 5,641,754, 5,691,317, 5,780,607, 5,786,138, 5,849,903, 5,856,103, 5,919,772, 5,955,590, 5,990,088, 5,994,320, 5,998,602, 6,005,095, 6,007,995, 6,013,522, 6,017,898, 6,018,042, 6,025,198, 6,033,910, 6,040,296, 6,046,004, 6,046,319, and 6,057,437 each of which is herein incorporated by reference in its entirety for their teaching of modifications and methods related to the same.

Disclosed are aptamers that interact with the disclosed receptor nucleic acids and could thus inhibit the expression of the receptors. Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP (United States patent 5,631,146) and theophylline (United States patent 5,580,737), as well as large molecules, such as reverse transcriptase (United States patent 5,786,462) and thrombin (United States patent 5,543,293). Aptamers can bind very tightly with k_{d}s from the target molecule of less than 10⁻¹² M. It is preferred that the aptamers bind the target molecule with a k_{d} less than 10⁻⁶, 10⁻⁸, 10⁻¹⁰, or 10⁻¹². Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a k_{d} with the target molecule at least 10, 100, 1000, 10,000, or 100,000 fold lower than the k_{d} with a background binding molecule. It is preferred when doing the comparison for a polypeptide for example, that the background molecule be a different polypeptide. For example, when determining the specificity of aptamers, the background protein could be ef-1α. Representative examples of how to make and use aptamers to bind a variety of different target molecules can be found in the following non-limiting list of United States patents: 5,476,766, 5,503,978, 5,631,146, 5,731,424, 5,780,228, 5,792,613, 5,795,721, 5,846,713, 5,858,660, 5,861,254, 5,864,026, 5,869,641, 5,958,691, 6,001,988, 6,011,020, 6,013,443, 6,020,130, 6,028,186, 6,030,776, and 6,051,698.

Disclosed are ribozymes that interact with the disclosed receptor nucleic acids and could thus inhibit the expression of the receptors. Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, (for example, but not limited to the following United States patents: 5,334,711, 5,436,330, 5,616,466, 5,633,133, 5,646,020, 5,652,094, 5,712,384, 5,770,715, 5,856,463, 5,861,288, 5,891,683, 5,891,684, 5,985,621, 5,989,908, 5,998,193, 5,998,203, WO 9858058 by Ludwig and Sproat, WO 9858057 by Ludwig and Sproat, and WO 9718312 by Ludwig and Sproat) hairpin ribozymes (for example, but not limited to the following United States patents: 5,631,115, 5,646,031, 5,683,902, 5,712,384, 5,856,188, 5,866,701, 5,869,339, and 6,022,962), and tetrahymena ribozymes (for example, but not limited to the following United States patents: 5,595,873 and 5,652,107). There are also a number of ribozymes that are not found in natural systems, but which have been engineered to catalyze specific reactions de novo (for example, but not limited to the following United States patents: 5,580,967, 5,688,670, 5,807,718, and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrates sequence. Representative examples of how to make and use ribozymes to catalyze a variety of different reactions can be found in the following non-limiting list of United States patents: 5,646,042, 5,693,535, 5,731,295, 5,811,300, 5,837,855, 5,869,253, 5,877,021, 5,877,022, 5,972,699, 5,972,704, 5,989,906, and 6,017,756.

Disclosed are triplex forming functional nucleic acid molecules that interact with the disclosed receptor nucleic acids and could thus inhibit the expression of the receptors. Triplex forming functional nucleic acid molecules are molecules that can interact with either double-stranded or single-stranded nucleic acid. When triplex molecules interact with a target region, a structure called a triplex is formed, in which there are three strands of DNA forming a complex dependent on both Watson-Crick and Hoogsteen base-pairing. Triplex molecules are preferred because they can bind target regions with high affinity and specificity. It is preferred that the triplex forming molecules bind the target molecule with a k_{d} less than 10⁻⁶, 10⁻⁸, 10⁻¹⁰, or 10⁻¹². Representative examples of how to make and use triplex forming molecules to bind a variety of different target molecules can be found in the following non-limiting list of United States patents: 5,176,996, 5,645,985, 5,650,316, 5,683,874, 5,693,773, 5,834,185, 5,869,246, 5,874,566, and 5,962,426.

Disclosed are external guide sequences that form a complex with the disclosed receptor nucleic acids and could thus inhibit the expression of the receptors. External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. (WO 92/03566 by Yale, and Forster and Altman, Science 238:407-409 (1990)).

Similarly, eukaryotic EGS/RNAse P-directed cleavage of RNA can be utilized to cleave desired targets within eukaryotic cells. (Yuan et al., Proc. Natl. Acad. Sci. USA 89:8006-8010 (1992); WO 93/22434 by Yale; WO 95/24489 by Yale; Yuan and Altman, EMBO J 14:159-168 (1995), and Carrara et al., Proc. Natl. Acad. Sci. (USA) 92:2627-2631 (1995)). Representative examples of how to make and use EGS molecules to facilitate cleavage of a variety of different target molecules can be found in the following non-limiting list of United States patents: 5,168,053, 5,624,824, 5,683,873, 5,728,521, 5,869,248, and 5,877,162.

Disclosed are polynucleotides that contain peptide nucleic acids (PNAs) compositions that interact with the disclosed receptor nucleic acids and could thus inhibit the expression of the receptors. PNA is a DNA mimic in which the nucleobases are attached to a pseudopeptide backbone (Good and Nielsen, Antisense Nucleic Acid Drug Dev. 1997; 7(4) 431-37). PNA is able to be utilized in a number of methods that traditionally have used RNA or DNA. Often PNA sequences perform better in techniques than the corresponding RNA or DNA sequences and have utilities that are not inherent to RNA or DNA. A review of PNA including methods of making, characteristics of, and methods of using, is provided by Corey (Trends Biotechnol 1997 June; 15(6):224-9). As such, in certain embodiments, one may prepare PNA sequences that are complementary to one or more portions of an mRNA sequence based on the disclosed polynucleotides, and such PNA compositions may be used to regulate, alter, decrease, or reduce the translation of the disclosed polynucleotides transcribed mRNA, and thereby alter the level of the disclosed polynucleotide's activity in a host cell to which such PNA compositions have been administered.

PNAs have 2-aminoethyl-glycine linkages replacing the normal phosphodiester backbone of DNA (Nielsen et al., Science Dec. 6, 1991; 254(5037):1497-500; Hanvey et al., Science. Nov. 27, 1992; 258(5087):1481-5; Hyrup and Nielsen, Bioorg Med Chem. 1996 January; 4(1):5-23). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc or Fmoc protocols for solid-phase peptide synthesis, although other methods, including a modified Merrifield method, have been used.

PNA monomers or ready-made oligomers are commercially available from PerSeptive Biosystems (Framingham, Mass.). PNA syntheses by either Boc or Fmoc protocols are straightforward using manual or automated protocols (Norton et al., Bioorg Med Chem. 1995 April; 3(4):437-45). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reverse-phase highpressure liquid chromatography, providing yields and purity of product similar to those observed during the synthesis of peptides.

Modifications of PNAs for a given application may be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine. The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (for example, Norton et al., Bioorg Med Chem. 1995 April; 3(4):437-45; Petersen et al., J Pept Sci. 1995 May-June; 1(3):175-83; Orum et al., Biotechniques. 1995 September; 19(3):472-80; Footer et al., Biochemistry. Aug. 20, 1996; 35(33): 10673-9; Griffith et al., Nucleic Acids Res. Aug. 11, 1995; 23(15):3003-8; Pardridge et al., Proc Natl Acad Sci USA. Jun. 6, 1995; 92(12):5592-6; Boffa et al., Proc Natl Acad Sci USA. Mar. 14, 1995; 92(6):1901-5; Gambacorti-Passerini et al., Blood. Aug. 15, 1996; 88(4):1411-7; Armitage et al, Proc Natl Acad Sci USA. Nov. 11, 1997; 94(23):12320-5; Seeger et al., Biotechniques. 1997 September; 23(3):512-7). U.S. Pat. No. 5,700,922 discusses PNA-DNA-PNA chimeric molecules and their uses in diagnostics, modulating protein in organisms, and treatment of conditions susceptible to therapeutics.

Methods of characterizing the antisense binding properties of PNAs are discussed in Rose (Anal Chem. Dec. 15, 1993; 65(24):3545-9) and Jensen et al. (Biochemistry. Apr. 22, 1997; 36(16):5072-7). Rose uses capillary gel electrophoresis to determine binding of PNAs to their complementary oligonucleotide, measuring the relative binding kinetics and stoichiometry. Similar types of measurements were made by Jensen *et al.* using BIAcore™ technology.

Other applications of PNAs that have been described and will be apparent to the skilled artisan include use in DNA strand invasion, antisense inhibition, mutational analysis, enhancers of transcription, nucleic acid purification, isolation of transcriptionally active genes, blocking of transcription factor binding, genome cleavage, biosensors, in situ hybridization, and the like.

As used herein, "peptidomimetic" means a mimetic of a peptide which includes some alteration of the normal peptide chemistry. Peptidomimetics typically enhance some property of the original peptide, such as increase stability, increased efficacy, enhanced delivery, increased half life, etc. Methods of making peptidomimetics based upon a known polypeptide sequence are described, for example, in U.S. Patent Nos. 5,631,280; 5,612,895; and 5,579,250. Use of peptidomimetics can involve the incorporation of a non-amino acid residue with non-amide linkages at a given position. One embodiment of the present invention is a peptidomimetic wherein the compound has a bond, a peptide backbone or an amino acid component replaced with a suitable mimic. Some non-limiting examples of unnatural amino acids which may be suitable amino acid mimics include β-alanine, L-α-amino butyric acid, L-γ-amino butyric acid, L-α-amino isobutyric acid, L-ε-amino caproic acid, 7-amino heptanoic acid, L-aspartic acid, L-glutamic acid, N-ε-Boc-N-α-CBZ-L-lysine, N-ε-Boc-N-α-Fmoc-L-lysine, L-methionine sulfone, L-norleucine, L-norvaline, N-α-Boc-N-δCBZ-L-ornithine, N-δ-Boc-N-a-CBZ-L-ornithine, Boc-p-nitro-L-phenylalanine, Boc-hydroxyproline, and Boc-L-thioproline.

In addition, antibodies to the receptors can be used to inhibit the function of the receptors. For example, isolated antibodies, antibody fragments and antigen-binding fragments thereof, that specifically bind to ASIC, P2X, and/or TRPV receptors can be used in the methods described herein. Optionally, the isolated antibodies, antibody fragments, or antigen-binding fragment thereof can be neutralizing antibodies. The antibodies, antibody fragments and antigen-binding fragments thereof disclosed herein can be identified using the methods disclosed herein.

As used herein, the term "antibody" or "antibodies" can also refer to a human antibody or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods serves to lessen the chance that an antibody administered to a human will evoke an undesirable immune response.

The term "antibodies" is used herein in a broad sense and includes both polyclonal and monoclonal antibodies. In addition to intact immunoglobulin molecules, disclosed are antibody fragments or polymers of those immunoglobulin molecules, and human or humanized versions of immunoglobulin molecules or fragments thereof, as long as they are chosen for their ability to interact with the polypeptides disclosed herein.

"Antibody fragments" are portions of a complete antibody. A complete antibody refers to an antibody having two complete light chains and two complete heavy chains. An antibody fragment lacks all or a portion of one or more of the chains. Examples of antibody fragments include, but are not limited to, half antibodies and fragments of half antibodies. A half antibody is composed of a single light chain and a single heavy chain. Half antibodies and half antibody fragments can be produced by reducing an antibody or antibody fragment having two light chains and two heavy chains. Such antibody fragments are referred to as reduced antibodies. Reduced antibodies have exposed and reactive sulfhydryl groups. These sulfhydryl groups can be used as reactive chemical groups or coupling of biomolecules to the antibody fragment. A preferred half antibody fragment is a F(ab). The hinge region of an antibody or antibody fragment is the region where the light chain ends and the heavy chain goes on.

Disclosed herein are kits that can be used in practicing the methods disclosed herein. The kits can include any reagent or combination of reagent discussed herein or that would be understood to be required or beneficial in the practice of the disclosed methods. For example, the kits could include primers to perform the amplification reactions discussed in certain embodiments of the methods, as well as the buffers and enzymes required to use the primers as intended.

The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules.

The antibodies disclosed herein can also be administered to a subject. Nucleic acid approaches for antibody delivery also exist. The broadly neutralizing antibodies to the polypeptides disclosed herein and antibody fragments can also be administered to subjects or subjects as a nucleic acid preparation (e.g., DNA or RNA) that encodes the antibody or antibody fragment, such that the subject's own cells take up the nucleic acid and produce and secrete the encoded antibody or antibody fragment.

The compositions disclosed herein can be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the inflammatory disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated herein by reference in its entirety for its teaching of an approach for parenteral administration.

The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al, Br. J. Cancer, 58:700-703, (1988); Senter, et al., Bioconjugate Chem., 4:3-9, (1993); Battelli, et al, Cancer Immunol. Immunother., 35:421-425, (1992); Pietersz and McKenzie, Immunolog. Reviews, 129:57-80, (1992); and Roffler, et al, Biochem. Pharmacol, 42:2062-2065, (1991)), all of which are herein incorporated by reference in their entirety for their teaching of the same. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells in vivo. In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration.

Nucleic acids that are delivered to cells which are to be integrated into the host cell genome typically contain integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can become integrated into the host genome.

Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

As described herein, the compositions disclosed herein can be administered in a pharmaceutically acceptable carrier and can be delivered to the subject's cells in vivo or ex vivo by a variety of mechanisms well known in the art (e.g., uptake of naked DNA, liposome fusion, intramuscular injection of DNA via a gene gun, endocytosis and the like).

If ex vivo methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

The nucleic acids, such as, the polynucleotides described herein, can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 3rd Edition (Cold Spring Harbor Laboratory Press, Cold Spring Harbor, N.Y., 2001) Chapters 5, 6) to purely synthetic methods, for example, by the cyanoethyl phosphoramidite method using a Milligen or Beckman System 1Plus DNA synthesizer. Synthetic methods useful for making oligonucleotides are also described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

Effective dosages and schedules for administering the compositions may be determined empirically, and making such determinations is within the skill in the art. The dosage ranges for the administration of the compositions are those large enough to produce the desired effect in which the symptoms of the disorder are affected. The dosage should not be so large as to cause adverse side effects, such as unwanted cross-reactions, anaphylactic reactions, and the like. Generally, the dosage will vary with the age, condition, sex and extent of the disease in the subject, route of administration, or whether other drugs are included in the regimen, and can be determined by one of skill in the art. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

Disclosed herein are methods of preventing a pathological muscle/fatigue condition comprising administering an effective amount of one or more ASIC, P2X, and/or TRPV receptor antagonists to a subject in need of such treatment. Disclosed are methods of preventing a pathological muscle/fatigue condition comprising administering an effective amount of one or more of one or more ASIC, P2X, and/or TRPV receptor antagonists in conjunction with other fatigue therapies to a subject in need of such treatment.

Disclosed herein are methods of reducing one or more symptoms associated with a pathological muscle/fatigue condition comprising administering an effective amount of one or more ASIC, P2X, and/or TRPV receptor antagonists to a subject in need of such treatment. Disclosed are methods of reducing one or more symptoms associated with a pathological muscle/fatigue condition comprising administering an effective amount of one or more of one or more ASIC, P2X, and/or TRPV receptor antagonists in conjunction with other fatigue therapies to a subject in need of such treatment.

Disclosed are methods for treating or preventing a pathological muscle/fatigue condition. Methods comprise inhibiting the increased expression levels of the receptors. Since the sequences of ASIC, P2X, and/or TRPV receptors are known, molecular or gene therapies can be employed as well as peptidomimetics that target these receptors and thus inhibit or decrease their expression. Such treatments can be referred to as the use of inhibitors of ASIC, P2X, and/or TRPV. Non-limiting examples of ASIC, P2X, and/or TRPV inhibitors are herein.

The methods disclosed herein can be performed or carried out on individuals previously diagnosed with one or more of the conditions described herein, individuals not previously diagnosed with one or more of the conditions described herein, individuals at risk for one or more of the conditions described herein, or individuals not at risk for one or more of the conditions described herein. The disclosed methods herein can further comprise a step of determining any of the above conditions or populations described herein. For example, the disclosed methods herein can further comprise a step of determining whether the subject or patient is in need of one or more of the treatments, diagnoses, or preventative/reductive methods described herein.

Research has shown that metabolite detecting receptors play a role in pain and fatigue indicating that compounds that modulate these gated ion channel receptors would be useful in the treatment of diseases and disorders related to muscle pain and fatigue. For example, U.S. Patent Application No. 2009/0023773 discloses compounds that modulate metabolite detecting receptors in order to treat diseases and disorders related to pain, inflammation, the neurological system, the gastrointestinal system and genitourinary system. Therefore, methods disclosed herein for detecting the modulation of metabolite detecting receptors would be useful in the treatment of diseases and disorders related to muscle pain and fatigue.

Disclosed herein are methods of screening the efficacy of a pharmaceutical agent for the ability to treat a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent.

Disclosed herein are methods of screening the efficacy of a pharmaceutical agent for the ability to treat a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent, further comprising determining the expression level of one or more adrenergic genes wherein a decrease in the expression level of the one or more adrenergic genes in the subject after treatment indicates efficacy of the pharmaceutical agent.

Disclosed herein are methods of screening the efficacy of a pharmaceutical agent for the ability to treat a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent, wherein the method further comprises determining the expression level of one or more immune mediated genes wherein a decrease in the expression level of the one or more immune mediated genes in the subject after treatment indicates efficacy of the pharmaceutical agent.

Disclosed are methods of screening the efficacy of a pharmaceutical agent for the ability to ameliorate one or more symptoms associated with a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent.

Disclosed are methods of screening the efficacy of a pharmaceutical agent for the ability to ameliorate one or more symptoms associated with a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent, further comprising determining the expression level of one or more adrenergic genes wherein a decrease in the expression level of the one or more adrenergic genes in the subject after treatment indicates efficacy of the pharmaceutical agent.

Disclosed are methods of screening the efficacy of a pharmaceutical agent for the ability to ameliorate one or more symptoms associated with a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent, further comprising determining the expression level of one or more immune mediated genes wherein a decrease in the expression level of the one or more immune mediated genes in the subject after treatment indicates efficacy of the pharmaceutical agent.

Disclosed herein are methods creating a model for pain and/or fatigue by treating an animal with an agonist or antagonist of metabolite detecting peptides. Such models can be used to determine if treatment with agonists of the metabolite detection genes induces muscle pain and fatigue. Treatment with an agonist of the metabolite detecting genes can induce a pathological muscle pain and/or fatigue condition in a subject that can be detected by determining the expression levels of the metabolite detecting genes, adrenergic function genes and immune function genes. Such models can also be used to determine whether treatment with an antagonist of the metabolite detection genes prevents externally induced muscle pain and/or fatigue in the subject. Disclosed are tissue culture assays that can be used to study the pathway and downstream effects of metabolite detection gene or peptide agonists and/or antagonists.

Disclosed herein are methods for ameliorating muscle pain and fatigue in disorders or diseases that are characterized by muscle fatigue, pain and weakness such as muscular dystrophy by administering and effective amount of one or more metabolite detecting gene or peptide antagonists, in an amount sufficient to ameliorate pain and/or fatigue.

The invention will be further described with reference to the following examples; however, it is to be understood that the invention is not limited to such examples. Rather, in view of the present disclosure that describes the current best mode for practicing the invention, many modifications and variations would present themselves to those of skill in the art without departing from the scope and spirit of this invention. All changes, modifications, and variations coming within the meaning and range of equivalency of the claims are to be considered within their scope.

### EXAMPLES

### Example 1

### Methods

*Harvesting* /*culturing cells*/*loading cells:* Thirty-nine three week old (4 wells of DRG neurons plated from each mouse), C57B1/6 mice were anesthetized with isoflurane, and euthanized by cervical dislocation. Dorsal root ganglia (DRG) from lumbar regions were removed into HBSS, treated with trypsin, washed with MEM (Invitrogen 51200-038) containing 10% FBS (Hyclone), 2.4% glucose, 1% glutamax (Invitrogen 35050-061) and 1% penicillin/streptomycin (Atlanta Biological B2110). DRG were triturated, pre-plated and incubated at 37°C for one hour to reduce non-neuronal cells. Neurons were gently swirled and centrifuged (110 x g, 5 min). Cells were removed, re-suspended and 30µL plated onto the 4 center wells of 24-well plates coated with poly-L-lysine and laminin. A 0.5mm thick silicone ring with a 4 mm diameter opening sealed to the surface of each well reduced the plating area in each well. Following 1hour, wells were filled with 37°C culture medium with 10 ng/mL GDNF (Glial Derived Neurotrophic Factor) (Preprotech 450-10). Sixteen to 23 hours after plating, cultures were loaded with Fura-2 AM (Molecular Probes F-1221) fluorescent dye for 40-60 minutes, then washed with pH 7.4 oxygenated observation medium containing no additional ATP (145 mM NaCl, 5 mM KCl, 2 mM CaCl₂, 1mM MgCl₂, 1mM Citrate, 10 mM glucose, 10 mM MES, 10 mM HEPES). The final well volume was 500 µL.

*Choice and concentrations of metabolites:* Because the afferent receptors in muscle have been reported to be on the outside of blood vessels supplying muscle, values obtained for muscle interstitium from experiments using cat, rat, and human for muscle contraction evoked by mild to moderate to extreme exercise were used.

*Photons (pH):* Values between 7.6 and 6.2 were used spanning the range found in skeletal and cardiac muscle in mammals from above resting values to those found with injury and disease.

*Lactate:* 1-50 mM lactate was used, a range of values that spans from rest to ischemia in muscles of humans and rats and mice.

*ATP:* ATP concentrations in muscle interstitium increase from 300 nM at rest to 5 µM with ischemic muscle contraction (Hellsten et al., 1998; Li et al., 2003; Li et al., 2005). These levels were used in the experiments described below. ATP receptors, particularly many P2X receptor subtypes, demonstrate considerable if not complete desensitization within a minute of agonist binding. To allow for physiological levels of desensitization of possible P2X receptors, freshly prepared ATP at 300nM was used for at least 30 seconds before any further increases in ATP combined with other metabolites, to establish resting conditions in DRG neurons in these experiments. Freshly prepared ATP was necessary, as it was determined that ADP was not capable of enhancing metabolite responses. Thus, while 1µM concentrations of ATP were capable of enhancing metabolite responses when freshly prepared, solutions kept at room temperature for more than 20 minutes were ineffective, presumably because some of the ATP had hydrolyzed to ADP. Taking all of this information together, protons, lactate, and ATP in combinations as shown in Table 1 were used. Also used were pH 6.0 and 4.0 without additional increases in metabolites to confirm that the imaging experiments could replicate recent reports using patch-clamp techniques (Leffler et al., 2006).

*Combinations of metabolites applied to mimic naturally occurring conditions:* When applied in an escalating series to mimic increases in metabolites that occur with muscle exercise and ischemic muscle exercise, the metabolite values shown in Table 1 were used. The values associated with pH 7.6 are at or below those normally found at rest in the mouse. The values at 7.4 approximate those found at rest. Generally, the low metabolite values associated with pH higher than 7.0 are likely to be non-noxious, while higher metabolite values at pH 7.0 and lower may be associated with pain. These values should bracket the physiological range of metabolites found in skeletal muscle with the last two values (those associated with 6.4 and 6.2) being higher than the physiological range (Bangsbo et al., 1993; Bangsbo et al., 2001; Li et al., 2003; Li et al., 2005; Reeh and Kress, 2001; Steinhagen et al., 1976).

In initial experiments the same concentrations of metabolites as in the table below were used, but returned to a "resting" value of pH 7.4, 1mM lactate, and 300 nM ATP after each application of increasing metabolites to determine the amount of adaptation that occurred due to increasing escalation of metabolites values. In still other experiments, the indicated metabolites were applied in reverse order.

**Table 1**

| **pH** | 7.6 | 7.4 | 7.3 | 7.2 | 7.0 | 6.8 | 6.6 | 6.4 | 6.2 |
|---|---|---|---|---|---|---|---|---|---|
| **Lactate** | 1mM | 1mM | 5mM | 10mM | 15mM | 20mM | 50mM | 50mM | 50mM |
| **ATP** | 300nM | 300nM | 400nM | 500nM | 1µM | 2µM | 5µM | 5µM | 5µM |

*ASIC antagonists:* Amiloride antagonizes all ASIC receptor responses to low pH. However, (Yagi et al., 2006) recently demonstrated that ASIC3 responses to moderate pH levels (pH 7.0) are actually enhanced by amiloride. APETx2, a peptide extracted from sea anemone can selectively inhibit ASIC3 and ASIC3 heteromers (Chagot et al., 2005; Diochot et al., 2004). However, A-317567 was used as an effective antagonist in the experiments described herein. A-317567 is an antagonist of all ASICs with IC 50s of 2.0 µM; 9.1 µM; and 9.5 µM for ASIC1, ASIC2 and ASIC3 respectively (Dube et al., 2005). A-317567 caused no decrements in calcium responses evoked by capsaicin, ATP or KCl. If applied for more than 20 minutes A-317567 caused some decrements in these responses.

P2X antagonists: PPADS has the same IC₅₀ at P2X3 and P2X5 receptors (0.2 µM) (Gever et al., 2006), and has an IC₅₀ of 25 µM at mouse P2X4 receptors (Jones et al., 2000; Khakh et al., 2001). TNP-ATP has an IC₅₀ of 0.31 nM at P2X3 receptors; 0.5 µM at P2X5 receptors (Wildman et al., 2002) and 16 µM at P2X4 receptors (Khakh et al., 2001).

TRPV1 antagonists: Capsazepine, the most used TRPV1 antagonist, has little inhibitory action on pH responses of TRPV1 receptors in mouse (Correll et al., 2004). In these investigations, JYL-1433 and LJO-328 were used which are potent full antagonists of TRPV1 (Lee et al., 2003; Reilly et al., 2005; Wang et al., 2002).

*Preparation of Antagonists:* TNP-ATP and PPADS were dissolved in observation media to a concentration of 5mM, quick-frozen and stored at -20° C. JYL-1422, LJO-328, A-317567, and a selected stereo isomer of A-317567 were each dissolved in 5% DMSO/95% water and a few µL of 1M HCl to a concentration of 10mM, then quick frozen and stored at -20° C. Aliquots of antagonists were thawed and diluted to the appropriate concentrations just prior to use in the experiments. The strong buffers maintained the pH of these antagonists when mixed with metabolites.

*Di-I labeling:* In some experiments, neurons were identified as innervating muscle by prior muscle labeling with Di-I. Twelve 7-9 day old mice were anesthetized by cooling. Using a dissecting microscope, hindlimb muscles (both heads of the gastrocnemius, plantaris, tibialis anterior, biceps femoris, and in some mice, semitendinosus) were exposed through a small skin incision, and each muscle was injected with 0.05 µL of Di-I (Molecular Probes) diluted in dimethyl formamide, using glass micropipettes with tips broken to 10-25 µm diameter attached to Hamilton syringes. Any DiI leaking to the muscle surface was immediately absorbed with cotton-tip applicators. The incision was closed with cyanoacrylate. The DRG from the labeled mice were harvested at 2-3 weeks of age (1-2 weeks after Di-I labeling). DiI labeled DRG neurons were imaged and outlined immediately before each experiment. Similar strategies have been used for patch-clamp investigations of the properties of retrogradely labeled neurons (Connor et al., 2005; Jiang et al., 2006; Molliver et al., 2005; Rau et al., 2007; Yagi et al., 2006).

*Imaging procedure:* All experiments were performed at room temperature (20-25°C) to allow correspondence with most previous experiments using calcium imaging and patch clamp of DRG neurons. Cells were imaged using the Meta Imaging Series Metafluor program (Universal Imaging Corp). All cells identifiable as neurons based on their round shape, clear, single nucleus, were selected from a phase image of the field. At the end of each experiment, 200 nM capsaicin was applied, followed by a wash, and then applied 50 mM KCl. Capsaicin activation indicated the presence of TRPV1 receptors on neurons, and KCl ensured that recorded objects were active neurons, and established the maximal calcium response in each neuron. For some neurons the administration of capsaicin abolished the potential for responding to KCl, thus either a response to capsaicin or to KCl indicated viability of neurons, and only viable neurons were included in analyses.

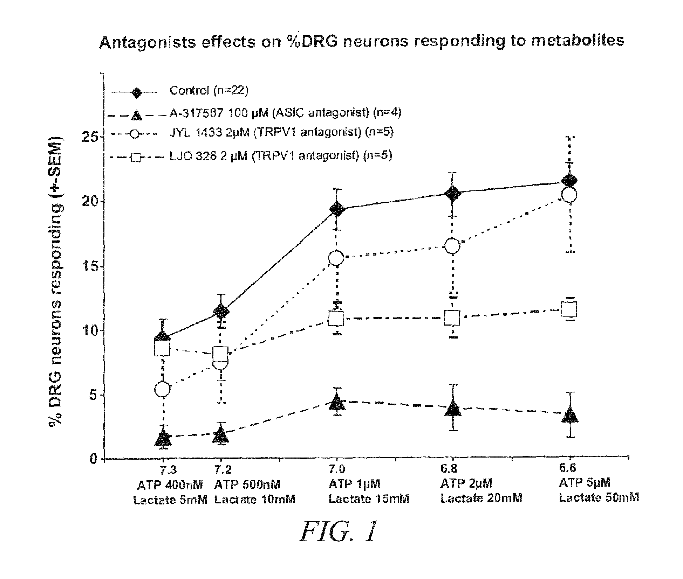

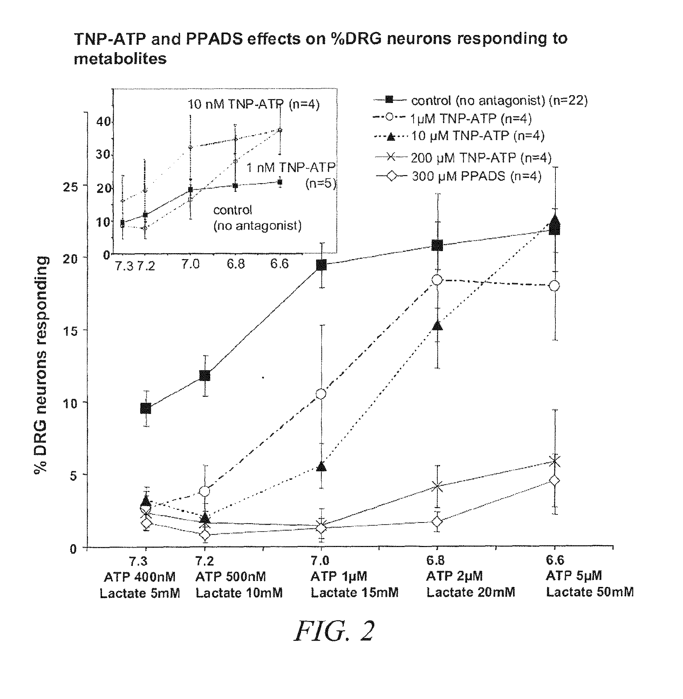

For each field an average of 151 + 10 cells were tracked real-time. For each cell the ratio of fluorescent intensities were computed (340 nm/380 nm normalized to a maximum of 1.0, minimum of 0.0), and recorded in a data table as well as displayed in a graph. Figures 1 and 2 show traces from these graphs. For each experiment approximately 30 baseline images were acquired before the addition of drug or metabolite changes, then 600 - 1000 fluorescent images were acquired (2 seconds between each image) to record the effect of metabolite and drug applications.

*Drug*/*metabolite application:* The metabolite or metabolite/drug solutions were applied via a custom-designed peristaltic pump apparatus. In other experiments, metabolites and drugs were delivered using two pipettes: one pulling solution out of the well immediately followed by the other pumping solution back into the well. In using the two-pipette method, clear artifacts were visible on the real-time graphs that correlated directly to the changing volumes in the well during the pipetting process, which also served as an indicator of the exact time the metabolite levels changed.

The two-pipette method also served as a control for the pump data omitting the effects of the flow of solutions across the well, for the potential for release of absorbed substances from the walls of the tubing and for the possible pressure changes that may have occurred while actually switching from one solution to the next. Both techniques produced similar results, so data was combined except where noted. For all of the data described herein, each well was used for only one metabolite series.

*Analysis:* Offline data from each run were loaded into an Excel spreadsheet for further analysis. This analysis subtracted baseline variations, and computed baseline means and standard deviations and compared them with means and standard deviations during each metabolite addition. Responses were defined as increases of Fura ratios more than 2 standard deviations above the baseline. The baseline was 30 - 60 seconds of imaging prior to the addition of the metabolites. Figures 1 and 2 show typical responses with both pump and pipette application of metabolites.

For statistical analyses, standard, one-way analysis of variance was used to compare the responses to the doses, with α< 0.01. Means were compared only if the ANOVA was significant. For multiple means compared to controls, Dunnett's test was used with α< 0.05. To compare means to each other, Tukey-Kramer Honestly Significant Difference analysis was used with α< 0.05.

*Calcium imaging of Cultured Dorsal root Ganglion neurons:* In general, molecular receptor clusters determined from cultured DRG neurons have proven to be similar to those found in specific types of sensory receptor endings (Lawson, 1996; Leffler et al., 2006; Petruska et al., 2000b; Petruska and Mendell, 2004; Price et al., 2001; Rau et al., 2005; Rau et al., 2007; Sluka et al., 2003). In spite of the advantages of sample size and access to the membrane surface, for a variety of reasons the responses of dorsal root ganglion neurons may differ from the responses of receptor endings in the tissues they normally innervate.

Imaging of increased intracellular calcium was used as an indicator that the DRG neurons were more likely to activate action potentials, not as an indicator of other calcium mediated cellular processes. Rapid increases in calcium may not reflect an increased likelihood of action potential generation in some circumstances. However, it has been shown that data obtained with calcium imaging relates well to data derived using patch-clamp [e.g.,(Hjerling-Leffler et al., 2007; Jiang et al., 2006; Leffler et al., 2006; Lu et al., 2006; Munns et al., 2007; Smith et al., 2004)]. However, for these and other reasons, it will be important to verify the findings reported here using in vitro and in vivo recordings from intact sensory fibers innervating muscle to determine the responses of their receptor endings in situ.

### Results

*Responses of DRG neurons to protons:* In initial experiments, metabolites were applied individually and returned to control levels after each application of increased metabolites. Experiments were performed to show responses of individual DRG neurons to increases in pump-applied metabolites, with responses to 200 nM capsaicin and 50 mM KCl added at the end of each trace. A portion of the trace was stopped, then started in order to allow the capsaicin and KCl responses to be included. 17 DRG neurons were retrogradely labeled from skeletal muscle with, and 7 of these that responded to metabolite increases.In most of the experiments reported here, increasing metabolites were applied in a more physiological manner. Experiments were performed to show responses of individual DRG neurons to changes in metabolites, with their responses to 200 nM capsaicin and 50 mM KCl included at the end of each trace. Pipetting artifacts were reduced or omitted. Note apparent metabolite responses at pH 7.4 caused by application 300 nM ATP, as no other metabolites were increased during this application. These ATP induced artifacts were not seen initially because ATP responses had adapted before the application of pH 7.4. All 10 of the metabolite responsive DRG neurons labeled with DiI from the hindlimb muscles. Twenty-two DRG neurons were labeled with DiI in this well but 12 of these did not respond to metabolite increases. All others were labeled as previously described. Thus, the DRG neurons were initially in a medium that contained the approximate levels of three metabolites (ATP, lactate, and protons) found under resting conditions in vivo. The medium in the well was then changed to the next lower pH level and corresponding increased lactate and ATP level. Thus, metabolites increased much the way they do with increasing muscle work. In the following experiments, a decrease in pH is indicated as "increasing protons", and "increasing protons" are included as one of the metabolites. The other two are lactate and ATP.

Experiments were performed to show the percent of DRG neurons with increases in intracellular calcium evoked by the metabolite levels. The metabolites were administered in the increasing order. A total of 5,307 DRG active neurons were analyzed. Very few DRG neurons responded to pH values above 6.0 without lactate and ATP present. Less than 4% of DRG neurons responded with increases in intracellular free calcium when protons alone were increased to physiologically relevant values in a series of steps (pH 7.4, 7.3, 7.2, 7.0, 6.8, 6.6). Previous reports using whole-cell patch clamp had similar findings (Jiang et al., 2006; Yagi et al., 2006). However, when the pH was reduced directly from 7.4 to 6.0 with no lactate, or ATP present, 9% of the DRG neurons responded (compare with 18% reported by (Leffler et al., 2006) using patch clamp). When lowered directly from pH 7.4 to pH 4.0, 27% of the DRG neurons responded. If only neurons under 25 µM in diameter were included in this analysis, 35% of the active neurons responded at pH 4. This compares with 41% of DRG neurons in the mouse that responded to pH 4 with current increases when whole-cell patch clamp was used and small and medium cells (comparable to neurons under 25 µM) were selected as reported by (Leffler et al., 2006). Thus, altering protons alone produced results similar to previous experiments using patch-clamp of mouse DRG neurons.

*Responses to Lactate:* Lactate alone (1-50mM) activated few DRG neurons, but lactate enhanced proton induced responses. Lactate alone (1-50 mM) caused no increases in DRG neuron responses at pH 7.4. Similarly, protons at pH 7.3-6.8 combined with lactate at 5-15 mM activated less than 4% of DRG neurons. The total percent of DRG neurons responding with a rapid calcium increase to the application of increasing metabolite combinations as applied to wells was calculated. To calculate the percentages, all of the DRG neurons responding at any of the 5 concentrations of metabolites presented in increasing amounts were counted. Because many neurons responded at more than one, but not all doses of metabolites, the total percentage of responding neurons was higher than any single percentage shown previously. These results indicate that pH 6.6, and lactate at 50 mM, activated 9.5% of DRG neurons, with a total of more than 10% of DRG neurons activated if all levels of metabolites were included. This moderate enhancement of pH responsiveness by lactate was consistent with patch-clamp experiments reported previously (Yagi et al., 2006).

*Responses to ATP:* High levels of ATP alone or with lactate present activated DRG neurons independently of pH. However, physiological levels of ATP presented alone activated few DRG neurons. ATP at 5µM, with lactate present and pH held constant at 7.4, activated up to 30% of DRG neurons if applied to cells naive to ATP. As has been indicated by other investigators (Gao et al., 2007), lower pH (6.8) slightly reduced responses of DRG neurons to ATP. However, ATP applied at lower doses (300 nM) and maintained for more than a few seconds prevented most of the calcium increases to ATP applied at higher doses, presumably through desensitization. Thus, an increasing, graded series of ATP applications (300nM, 400nM, 500nM, 1µM, 2 µM, 5µM), activated less than 10% of DRG neurons when 2 µM was reached, and approximately 16% of DRG neurons when going from 2 µM to 5 µM.

*Responses to combinations of metabolites:* DRG responses to ATP combined with "increasing protons" was more than additive. As noted previously (Naves and McCleskey, 2005; Spelta et al., 2004), ATP appeared to act synergistically with protons to enhance responses of DRG neurons. This "more than additive" effect was most apparent at moderate metabolite concentrations. Thus, at pH 7.0-ATP 1µM the percent of DRG neurons responding to increasing protons with the addition of ATP was 11.2. This percentage was more than that of DRG neurons responding to pH 7.0 protons alone (2.7) plus the percent of DRG neurons responding to ATP with lactate (4.7) = 7.4.

Combinations of physiological levels of ATP, lactate and protons produced by working muscles significantly increased intracellular free calcium in one third of mouse DRG neurons in culture. While individual or combinations of two of the metabolites activated relatively few DRG neurons, increasing the combination of all three metabolites within the physiological range of working skeletal muscle activated 32.4 + 1.6%. The combination of all three metabolites activated more DRG neurons than any single metabolite activated, and more than combinations of any two metabolites activated (ANOVA F=11.96, DF=5:31, p<.0001-Dunnett's test for DRG with all three metabolites vs. all other conditions p<.05).

The total number of DRG neurons that responded to any of the metabolite combinations cannot be determined from previous results, because many DRG neurons responded to several of the doses of metabolite combinations, but most did not respond to all doses of metabolites.

A higher percentage of DRG neurons innervating skeletal muscle responded to increasing metabolites than in the overall population of DRG neurons. Forty four percent of DRG neurons retrogradely labeled from skeletal muscle responded to the combination of all 3 metabolites. This percentage (44.2+- 6.3%, range 32-89%) was higher than the percentage of all DRG neurons responding to the combination of all 3 metabolites (32.4+-1.6%, range 26-49%). In addition, a larger range of metabolites indicated that DRG neurons responded at resting metabolite values, and that DRG neurons showed nearly flat metabolite dose-response relationships above pH 7.0, ATP 1 µM, lactate 15 mM.

Note that the percent of neurons responding increased until pH 7.0-lactate 15 mM-ATP 1µM. More extreme metabolite increases to pH 6.4-50 mM lactate-5 µM ATP, and further to pH 6.2-50 mM lactate-5 µM ATP caused only small (statistically non-significant) additional increases in the percent of responsive neurons. Overall, these data indicated that the responses to increasing metabolites were flat above pH 7.0. Thus if the entire population of DRG neurons were lumped together, they unambiguously encoded increases in metabolites only up to pH 7.0-15mM lactate-1µM ATP.

When collecting these data, it was noted that responses of DRG neurons were heterogeneous. Some neurons began responding near the resting values of metabolites: pH 7.4-1 mM lactate-300 nM ATP. "Low metabolite responders" increased their responses only up to pH 7.0-15 mM lactate-1µM ATP, then showed a decrease in response as metabolite values increased further. Other neurons only began to respond with metabolites near pH 7 and increased their responses to metabolites at pH 6.8, and 6.6, but sometimes decreased responses at higher metabolite levels.

These observations led to dividing the DRGs into two groups. One group, "High metabolite responders" included those that discriminated metabolite values higher than pH 7.0, ATP 1µM, Lactate 15 mM. The other group, "Low metabolite responders", included those capable of detecting metabolite values pH 7.0, ATP 1µM, Lactate 15 mM or lower.

The percentage of DRG neurons responding to progressive increases of metabolites (lactate, ATP, and protons) where divided into low metabolite and high metabolite responding types for all DRG neurons and those retrogradely labeled from skeletal muscle. These two populations of DRG neurons have different peak percentages of responding neurons. The low metabolite responding population had a peak at pH 7.0, and the high metabolite responding population had a peak at pH 6.4. For DRG that were retrogradely labeled from hindlimb skeletal muscle the high metabolite peak was at pH 6.6, but this was not statistically different than the value at 6.4. The characterization as low or high metabolite responsive was relatively stable. Returning a well to resting values for 5 minutes after a complete series of metabolites, then applying another series of metabolites did not shift neurons from one classification to another. In other words, "low metabolite responders" did not become "high metabolites responders" or vice versa. However, some desensitization was evident, as 10-15% of the DRG neurons that responded during the first series failed to respond to the second series of metabolites.

This data indicated approximately equal numbers of low metabolite vs. high metabolite responding DRG neurons. For the 44% of DRG neurons retrogradely labeled from skeletal muscle and activated by metabolites, 46.5% responded best to low metabolites levels while 53.5% responded best to high metabolite levels. For all DRG neurons responding to metabolites, 54.9% responded best to low metabolite levels while 45.1 % responded best to high metabolite levels.

Calcium responses of DRG neurons were retrogradely labeled from skeletal muscle to the application of the indicated metabolites. DRG neurons were divided into those that increased responses up to metabolites associated with pH 7.0, then decreased calcium responses to higher levels of metabolites (Low metabolite responders) and those that increased calcium responses to metabolites associated with pH levels lower than 7.0 (High metabolite responders). Only neurons that showed a significant calcium increase above baseline were used in this analysis. Only neurons that showed a significant calcium increase above baseline were used in this analysis. The magnitude of the calcium responses of muscle innervating DRG neurons are very similar to percent responding DRG neurons for the low metabolite vs. high metabolite groups. While the drop off in responses (past pH 7.0 for low metabolite responders, and pH 6.6 for high metabolite responders) could have been interpreted as "ceiling" effects, the much higher calcium responses evoked by capsaicin, and/or KCl indicated otherwise.

*Receptor antagonist effects on responses to increasing metabolites:* A selective ASIC antagonist blocked all responses of DRG neurons to increased metabolites. To determine the likely molecular receptors mediating the responses to increased metabolites, antagonists selective for the receptors previously hypothesized by other investigators as mediators of metabolite responses in sensory neurons innervating muscle were used. It was determined that 100 µM A-317567 [a selective antagonist for ASIC1, 2 and 3 (Dube et al., 2005)] reduced the responses to metabolites to 23% of the control level (p<.0001) as shown in Figure 1 (filled triangles). A-317567 at 200 µM blocked all responses to metabolites. As confirmation of selectivity, A-317567 (applied for 10 minutes or less) at any dose did not decrease responses to capsaicin, ATP, or KCl.

The TRPV1 antagonist LJO-328 at 2µM blocked some of the DRG neuron responses as shown in Figure 1 (squares) (p<.01, Dunnett's test vs. controls). DRG labeled from muscle were similarly affected. TRPV1 antagonist inhibition of responses to metabolites appeared to affect both low and high metabolite responding neurons. As evidence for the effectiveness and selectivity of these antagonists, the 2 µM doses of LJO-328 and JYL-1433 abolished nearly all responses to capsaicin (less than 2% responded), but responses to KCl application remained normal.

P2X antagonists show involvement of more than one P2X subtype on the responses of DRG neurons to increases in metabolites. At 10 µM, TNP-ATP, a potent P2X1 and P2X3 antagonist increased responses to metabolites (see insert in Figure 2) (p<.05 Dunnett's test). The 10 µM dose was well above the IC 50 of P2X3 (0.3 nM), but below the IC 50 for both P2X4 and P2X5 (16 µM and 0.5 µM respectively). At 1 and 10 µM, however, TNP-ATP reduced responses of DRG neurons evoked by increasing metabolites between pH 7.3-7.0 (see Figure 2 triangles) (p<.05 Dunnetts test). As shown in Figure 2, TNP-ATP applied at 200 µM abolished most responses to increasing metabolites. However, capsaicin and KCl responses were unaffected by any dose of TNP-ATP, indicating specificity of TNP-ATP. PPADS at 300 µM (capable of blocking P2X3, 4 and 5) eliminated most responses to increasing metabolites.

*Capsaicin responses of DRG neurons responding to metabolites-pH:* The percentage of DRG neurons responding with rapid calcium increase to application of 200 nM capsaicin was calculated. When all DRG neurons were included, 50.9 ± 4.2% responded to 200 nM capsaicin. This is higher than previous reports of capsaicin responses in DRG neurons in the mouse using patch-clamp techniques [36% reported by (Leffler et al., 2006) and 20% reported by (Lin et al., 2008)]. Significantly more metabolite-responding DRG neurons also responded to capsaicin (71.9 + 4.2% - p<.01, overall ANOVA-p<0.0005). For DRG neurons retrogradely labeled from muscle, as with all DRG neurons, more metabolite responsive neurons also responded to capsaicin (all muscle labeled neurons 48.8 + 5.8% vs. muscle labeled metabolite responders 63.4 + 5.8%, p<0.05).

*Size of responsive DRG neurons:*Other groups using whole-cell recordings indicated that all sizes of DRG neurons respond to pH decreases, but especially intermediate size neurons (Lin et al., 2008; Molliver et al., 2005). The data is consistent with these observations. Table 2 contains the mean diameters, standard errors, ranges and comparisons for a sample of 2838 active, cultured mouse DRG neurons. Table 2 shows that, while metabolite responsive neurons were of all sizes, they were most commonly smaller neurons with an average diameter of 20.8 µm compared to 22.2 µm for all active DRG neurons. Metabolite responsive neurons were about the same size as capsaicin responsive neurons. Low metabolite responsive neurons were not different in size from high metabolite responsive neurons (p>0.64).

Muscle-innervating DRG neurons, however, were larger on average than all DRG neurons (average diameter 23.7 µm vs. 22.2 µm). More surprising, but consistent with findings in rats, the diameters of metabolite-responsive, muscle-labeled DRG neurons were larger on average than the diameters of metabolite responsive neurons from the overall DRG neuron populations. In addition, the diameters of capsaicin-responsive, muscle-innervating DRG neurons were also larger than the diameters of capsaicin responsive neurons from the overall DRG population. Histograms of diameters of muscle labeled DRG neurons responsive to increasing metabolites were performed. Diameter histograms were generated from 184 active muscle labeled DRG neurons, 82 metabolite responsive muscle labeled DRG neurons, 90 capsaicin responsive, muscle labeled DRG neurons which were scaled to the number of metabolite responsive neurons. There was an overabundance of small diameters and decreased abundance of large diameters in the metabolite responsive DRG neurons compared with all active muscle labeled DRG neurons. Histograms of muscle labeled DRG neurons demonstrated the smaller number of very large DRG neurons responding to metabolites, compared with all muscle-innervating DRG neurons. Muscle labeled metabolite responsive neurons also included many sizes, and capsaicin responding neurons had a similar size distribution to muscle labeled, metabolite responding neurons.

Overall, these data show that most metabolite responding DRG neurons innervating muscle were among the smallest neurons innervating muscle, and therefore, most of these DRG neurons likely had unmyelinated or thinly myelinated axons characteristic of Group III and IV neurons. However, the muscle-innervating, metabolite responding DRG neurons were larger on average than metabolite responding DRG neurons that did not innervate muscle.

Experiments showed that combinations of increasing protons, lactate, and ATP activated more DRG neurons than individual or pairs of metabolites. Increasing protons alone (pH from 7.4 to 6.6) was ineffective in activating DRG neurons as determined by calcium imaging. Increasing lactate alone from 1-50 mM activated very few mouse DRG neurons if the pH was constant at 7.4. (Rotto and Kaufman, 1988) previously showed that buffered sodium lactate alone had little or no effect on Group III and IV muscle afferents in the cat. ATP alone at 5 µM induced calcium responses in nearly 30% of DRG neurons (independent of pH between 7.4 and 6.6) when applied to naive cultures. Others have reported excitatory effects of ATP on DRG neurons (Burnstock and Wood, 1996; Chen et al., 1995; Cook et al., 1997; Hu and Li, 1996; Jahr and Jessell, 1983; Kindig et al., 2007; Rang et al., 1991; Zimmermann, 1994). However, if neurons were exposed to the amounts of ATP found in resting muscle interstitium, then ATP was gradually increased in amounts similar to those found during muscle contractions in vivo (Li et al., 2003; Li et al., 2005), fewer than 11% of DRG neurons were activated, and then only at concentrations higher than 2 µM.

Previous investigations thought that individually applied ATP, lactate, or protons can activate muscle afferents at high concentrations, but are relatively ineffective at lower concentrations (Graham et al., 1986; Hanna and Kaufman, 2004; Hoheisel et al., 2004; Kaufman and Rybicki, 1987; Kindig et al., 2006; Li and Sinoway, 2002; Reinohl et al., 2003; Rotto and Kaufman, 1988; Rybicki et al., 1985; Sinoway et al., 1993; Thimm and Baum, 1987). Thus, the metabolites lactate, protons and ATP, evoked few responses in DRG neurons when applied individually in the normal, physiological ranges. However, protons or ATP, individually applied, can evoke responses at the high concentrations caused by vascular and muscle injury, and can play an active role in detecting traumatic muscle damage.

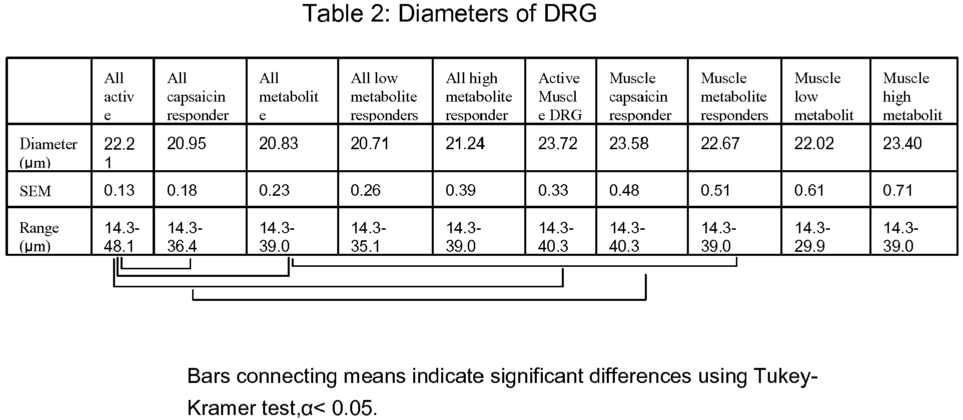

The effects of individually applied metabolites contrasted sharply with the excitatory effects of the combination of all three metabolites (lactate, ATP and protons). The combination of all three metabolites, even at modest levels (pH 7.0, ATP 1 µM, lactate 15 mM) activated more than 20% of all DRG neurons and more than 30% of DRG neurons innervating skeletal muscle. Higher amounts of all three metabolites activated 30% of all DRG neurons and nearly 50% of those innervating muscle. Previous investigations demonstrated that lactate, presumably by chelating calcium, enhanced the responses of DRG neurons to protons in the physiological range (Immke and McCleskey, 2001; Immke and McCleskey, 2003; Naves and McCleskey, 2005; Yagi et al., 2006). It has also been demonstrated that ATP acting via a P2X receptor enhanced ASIC3 responses to protons. Thus, it appears that all three of the metabolites, protons, lactate, and ATP, play a role in the response of DRG neurons to the concentrations of metabolites that are normally produced by contraction of skeletal muscle.

These experiments show that active muscle contraction rather uniquely produces increases in this combination of metabolites. The metaboreceptor requirement for this specific combination of three metabolites reduced the chances of muscle afferent responses to proton changes not caused by muscle contraction, such as metabolic or respiratory acidosis and alkalosis. In addition to the metabolites described herein, several other metabolites closely associated with muscle injury, but also shown to increase with muscle contraction, may enhance the activation of small-diameter-fiber afferents from muscle. These include bradykinin, potassium, and arachidonic acid metabolites such as prostaglandin E₂, and various cytokines (Cui et al., 2007; Hoheisel et al., 2005; Kaufman et al., 1983; Kaufman and Rybicki, 1987; Mense, 1977; Mense and Schmidt, 1974; Rotto and Kaufman, 1988).

Most of these substances activate muscle afferent fibers at relatively high concentrations, although the actual effective dose at the nerve endings may be in the physiological range. It is interesting that non-steroidal anti-inflammatory compounds that have been used to indicate prostaglandin involvement can directly block ASIC3 receptors (Voilley et al., 2001). Potassium has an effect on muscle afferents at relatively low concentrations. However, (Kaufman and Rybicki, 1987) indicated that K⁺ application produced rapidly adapting responses that would not be capable of encoding the sustained discharge observed with the increases in metabolites caused by muscle contraction. While it is unknown whether these metabolites enhance responses of muscle afferents under normal conditions, it is likely that some or all of these agents may play a role in the enhancement of sensitivity of Group III and IV afferents under a variety of conditions such as muscle ischemia, muscle injury, and metabolic insults caused by toxins, infections, and disease. The direct effects of increased oxidative metabolism (low oxygen and/or high carbon dioxide levels) have not been shown to increase the responses of Group III and IV muscle afferents (Wenk and McCleskey, 2006).

The results herein also support the existence of at least two populations of DRG neurons: one responding to higher metabolites, the other to lower metabolites. One population should detect innocuous levels of metabolites and contribute to the sympathetic pressor response evoked by active muscle contraction, but should not contribute to muscle pain. The other population should detect noxious levels of metabolites and contribute to muscle pain (Kniffki et al., 1978; Kniffki et al., 1981; Mense, 1993; Mense, 1996; Mense and Stahnke, 1983).

Both populations could contribute to sympathetic reflexes and to increasing perceptions of fatigue. Even one of the earliest descriptions of the exercise pressor reflex in human subjects indicated that increasing levels of metabolites evoked innocuous perceptions associated with the exercise pressor response preceding the perception of pain (Alam and Smirk, 1937). These results herein show that a population of innocuous receptors distinct from nociceptors can exist that can detect increases in muscle-metabolite levels before painful levels are reached.

The three most likely metabolites in combinations produced by contracting muscles that span the range from resting values to the values observed with maximal muscle pain. Using this combination of three metabolites, it is demonstrated herein that at least two populations of DRG neurons innervating skeletal muscle in the mouse. First, there are low metabolite responsive DRG neurons (46.5% of metabolite responsive neurons). One population of DRG neurons labeled from skeletal muscle was maximally activated at metabolite values of pH 7.0, lactate 15 mM, ATP 1 µM. These metabolite values have been reported with heavy, but not necessarily painful muscle exercise (Gibala et al., 2002). In this population of DRG neurons, when higher levels of metabolites were applied, both the percent of responding DRG neurons decreased, and the magnitude of calcium responses decreased. With still higher levels of metabolites, the responses in this population plateaued or increased slightly. However, this increase never reached the maximum response observed at pH 7.0, lactate 15 mM and ATP 1 µM.

Low metabolite responsive DRG neurons were sensitive to resting, or lower than resting, values of metabolites (pH 7.4, lactate 1 mM, ATP 300nM). The sensitivity to increased metabolites and the observed response profile seem to mirror the pressor response evoked by sustained muscle contraction. This sensitivity also would be adequate to mediate the sense of "tiredness" and/or "pressure" described by many subjects with the initiation and maintenance of sustained muscle contraction (Alam and Smirk, 1937). (Adreani et al., 1997; Adreani and Kaufman, 1998) observed that 16 of 19 group IV muscle afferents (in cats) responded to levels of exercise that were consistent with non-painful conditions in human subjects. Interestingly, 9 of these 19 increased their response when the muscle was made ischemic, raising the metabolite levels to potentially painful levels. This could indicate that about half of the group IV neurons they recorded were low metabolite responsive, while the other half were high metabolite responsive, proportions consistent with our findings in mice (see also reviews by (Kaufman et al., 2002; Kaufman and Hayes, 2002).

High metabolite responsive DRG neurons (53.5% of metabolite responsive neurons represent a second population of DRG neurons labeled from skeletal muscle was maximally activated at metabolite values of pH 6.6, lactate 50 mM, ATP 5 µM. The proportion of responding neurons increased steadily to the maximum value noted above. This population would seem well suited to detect large increases in metabolites between pH 7.0 and pH 6.6 values. These values have been associated with ischemic, contracting muscle that is often painful (Alam and Smirk, 1937; Bangsbo et al., 1993; Bangsbo et al., 2001; Li et al., 2003; Li et al., 2005; Reeh and Kress, 2001; Steinhagen et al., 1976). The separation of these two populations of DRG neurons was empirical, based on the initial observations of responses of cultured neurons to increases in the three metabolites used in these experiments.

Both of the populations of DRG neurons described here ("low and high metabolite responders"), by virtue of their responsiveness to capsaicin and protons, could belong to the "class 8a" defined by (Jiang et al., 2006; Petruska et al., 2000b). These reports indicated that class 8a afferents innervate specific types of tissues, such as blood vessels, that are distributed throughout various organs and tissues of the body. Muscle innervating afferents may be one type of class 8a afferents, since Group III and IV muscle afferents are most often associated with neurovascular bundles (Molliver et al., 2005).

Selective antagonists indicate the synergy of 3 molecular receptor types in mediating DRG responses to increasing metabolites (protons, lactic acid, and ATP). A-317567 at 100 µM, a potentially selective ASIC antagonist (Dube et al., 2005), blocked ∼80% of DRG neuron responses to metabolites increased as high as pH 6.6, lactate 50 mM, ATP 5 µM. Two hundred µM A-317567 blocked all responses to metabolites. These same concentrations of A-317567 did not block DRG neuron capsaicin responses, KCl responses, or responses to 5 µM ATP applied to naïve DRG neurons. The blockade by A-317567 shows that at least one of the subtypes of ASIC 1-3 is necessary for all responses of DRG neurons to metabolites less than or equal to pH 6.6, lactate 50 mM, ATP 5 µM. When combined with lactate and ATP, these receptors in heterologous expression systems have a sustained response to lowering pH that mimics the sustained response of DRG neurons. However, other investigators (Benson et al., 2002); (Hesselager et al., 2004) have demonstrated that splice variant heteromers of ASIC1 and 2 can also respond to pH in a manner that is consistent with responses of DRG neurons. Benson et al. (2002) also showed that mice lacking any one of the ASIC receptor subtypes had DRG neurons that still responded to pH, although in a somewhat different fashion. Interestingly, using rats, (Molliver et al., 2005) demonstrated that 51% of small DRG neurons (<25 µM), retrogradely labeled from muscle, expressed ASIC3 as determined by immunohistochemistry. This is consistent with the 44% of muscle labeled neurons responsive to metabolites in mouse described herein. Thus, the data described herein shows that ASIC receptors are likely to be major mediators of responses to increased metabolites produced by contracting muscles.

*P2X antagonists: TNP-ATP and PPADS:* The experiments described herein showed that doses of TNP-ATP that were effective at the P2X3 receptor (10 nM) can increase responses of DRG neurons to increasing metabolites. Doses of TNP-ATP effective at the P2X5 receptor (1 and 10 µM) eliminated most responses to low metabolites but had little effect on metabolite responses associated with pH 6.8 and above. Doses of TNP-ATP and PPADS effective at all P2X receptors, including P2X4 (200-300 µM) eliminated nearly all responses to increasing metabolites (see Figure 2). These data show that P2X3 receptors do not enhance the response of DRG neurons to increasing metabolites. However, P2X5 receptors can be necessary for responses to low levels of metabolites, and both P2X5 and P2X4 receptors can be necessary for responses to high levels of metabolites. Many other groups have demonstrated that ATP can directly activate sensory neurons. Much of this activation has been attributed to P2X3 and P2X2 receptors and their heteromers (see reviews by (Khakh and North, 2006; North, 2004; Petruska et al., 2000a). These mechanisms may explain direct effects by sudden surges of ATP similar to the levels that tissue injury can cause.

Contrary to what might be expected, ATP concentrations in muscle interstitium are increased during muscle contractions (Li et al., 2003; Li et al., 2005). The concentrations of ATP present at sensory receptor endings in muscle fascia, even at rest, should be sufficient to activate, and then desensitize most P2X receptors on nerve endings within skeletal and cardiac muscle. In addition, P2X receptors have been reported to become less sensitive with the increasing proton concentration that occurs with muscle contraction (Gao et al., 2007). From this information, it would seem that most P2X receptors would not add to the generator potential of sensory neurons as metabolites increase with muscle exercise. Thus, the direct detection of muscle metabolites by P2X receptors is problematic. In addition, it is problematic that in our experiments, both P2X antagonists and ASIC antagonists applied individually can block virtually all responses to physiological levels of metabolites.

These problems can be resolved by recent experiments demonstrating that ATP is a potent modulator of ASIC3, dramatically enhancing responsiveness to pH in the normal physiological range (Naves and McCleskey, 2005; Spelta et al., 2004). This group has also reported co-transfection experiments that demonstrated that only P2X4 and P2X5 (Spelta et al., 2004) are effective in causing this enhancement of ASIC3 responsiveness. This modulation requires ligand binding, but does not require ionic current flow. The modulation is synergistic, in that the currents activated by ASICs and P2X channels individually are far less than the currents activated by ligand binding to both types of channels when they are co-expressed. Precisely how this interaction occurs is unknown. Thus, the data described herein shows that both ASIC and P2X receptors are necessary to detect normally occurring muscle metabolites in the physiological range.

*TRPV1 antagonists:* The TRPV1 antagonist LJO-328 significantly reduced the response of DRG neurons to increasing metabolites. Even though experiments reported here showed that metabolite responding neurons were more likely to respond to capsaicin (indicating that they very likely had TRPV1 receptors) than non-metabolite responding neurons. Previous investigations in mouse have shown that pH values lower than 6.0 are necessary to evoke substantial TRPV1 mediated current from mouse DRG neurons (Leffler et al., 2006) and from heterologous expression systems (Caterina et al., 1997; Jordt et al., 2000; Tominaga et al., 1998). In addition, the findings herein show that the selective ASIC antagonist, A-317567 blocked all metabolite responsiveness also showed that ASICs, not TRPV1 contributed to the response to all metabolite levels used in the experiments reported here.

The data herein shows that antagonizing TRPV1 can reduce the responsiveness of DRG neurons to metabolites. Thus, the TRPV1 activation can sum with activation of ASICs, and/or TRPV1 activation can enhance the responsiveness of ASICs similar to the way that P2X receptors enhance responses. Thus, TRPV1 receptors, in addition to ASIC and P2X receptors contribute some of the responsiveness of DRG neurons to physiological levels of metabolites.

While the combination of these three molecular receptor types (ASICs, P2Xs and TRPs) can respond in a fashion that is adequate to explain the normal response of muscle sensory afferents to the metabolites produced by muscle contraction, it is possible that other molecular receptors on the primary afferent endings can participate in modulating these responses. Thus, receptors for various cytokines and arachiodonic acid metabolites might sensitize muscle afferent endings under a variety of conditions, and may be responsible for enhanced perceptions of fatigue and pain (Hayes et al., 2006; Rotto et al., 1990a; Rotto et al., 1990b). In addition, recently discovered G-protein coupled receptors such as OGR1 (Huang et al., 2007), and proton inhibiting potassium channels such as TASks (Cooper et al., 2004; Rau et al., 2006) may play a role in detecting and signaling metabolite increases in sensory neuronal endings.

The population of afferents responsive to high metabolite levels described here could mediate the sensory experience of muscle pain caused by sustained and/or ischemic muscle contraction (Arendt-Nielsen et al., 2003; Graven-Nielsen et al., 2004). However, the sensory experience mediated by DRG neurons responding to low metabolite levels may be different. (Alam and Smirk, 1937) indicated that muscle contractions with the circulation occluded induced a pre-pain sensory experience of "tiredness or heaviness not causing appreciable discomfort" that coincided with an increase in the mean arterial pressure. This pre-pain perception occurred well before the onset of muscle ache and pain. Recent reports indicate that this pre-pain "pressure" sensation is not mediated by cutaneous afferents, but by small diameter muscle afferents (Graven-Nielsen et al., 2004). Very little is known about the non-painful sensation that can emanate from muscle, and may be associated with, if not mediated by the same afferent neurons that mediate the exercise pressor reflex.

The sensation evoked by non-painful metabolite accumulation in muscle can be that of muscle "fatigue". This would be only loosely related to actual "muscle fatigue" which is the failure of muscle contraction. Several investigators have indicated that small diameter afferent fibers innervating muscle are at least partially responsible for the inhibition of motor command that causes true muscle fatigue under many conditions (see review (Garland and Kaufman, 1995). These same afferent fibers can engage sensory systems in the brain analogous to pain pathways and can be responsible for the perceptual experience of muscle "pressure and tiredness" (Alam and Smirk, 1937) that accompanies the accumulation of metabolites in muscle. This sensory experience could be the familiar perception of "muscle fatigue".

### Conclusion

The data presented here demonstrate that physiological levels of three metabolites produced by contracting muscles- protons, ATP, and lactate- act together to activate a significant population of DRG neurons that innervate skeletal muscle. No individual or pair of these metabolites has the effectiveness of the proper combination of all three metabolites. The DRG neurons responding to metabolites are heterogeneous, consisting of at least two populations. One population responds to the levels of metabolites produced by non-painful muscle contractions and may contribute significantly to the exercise pressor reflex, and perhaps to the sensory experience of "fatigue". The other population responds to levels of metabolites that are likely to be associated with stronger, possibly painful muscle contractions, and could contribute to acute muscle pain produced by ischemic muscle contraction, or by muscle inflammation and injury.

At least three molecular receptors appear to work in synergy to detect the full range of metabolites produced by muscle contraction and muscle injury. These receptors may be an ASIC receptor (possibly ASIC3) a P2X receptor (possibly P2X5 for moderate metabolite levels and P2X4 for high levels of metabolites) and TRPV1. Other metabolites and receptors may also play a role in detection of metabolites in normal muscle contractions, and still others may play a role in the enhancement of responses that can occur following muscle injury, inflammation, and disease.

### Example 2

To determine how sensory neurons respond to increases in lactate and ATP normally produced by contracting muscles, further experiments were conducted. Calcium imaging of Cultured Dorsal root Ganglion neurons were carried out as described above. The percent of neurons responding increased until pH 7.0-lactate 15 mM-ATP 1µM, gently falling off at lower pHs and higher ATP and lactate concentrations. When pH reduction was accomplished by the increase in lactate and ATP normally produced by contracting muscles, sensory neurons responded much like people's reports of pain.

This data in combination with the experiments described above established that combinations of metabolites produced by muscle contraction activate sensory neurons innervating muscle. The metabolites include, but are not limited to, lactate, ATP and protons (lowered pH). This data also established that there are two populations of muscle innervating DRGs, one population that responds to metabolites at painful levels and one that responds to metabolites at non-painful levels.

### Example 3

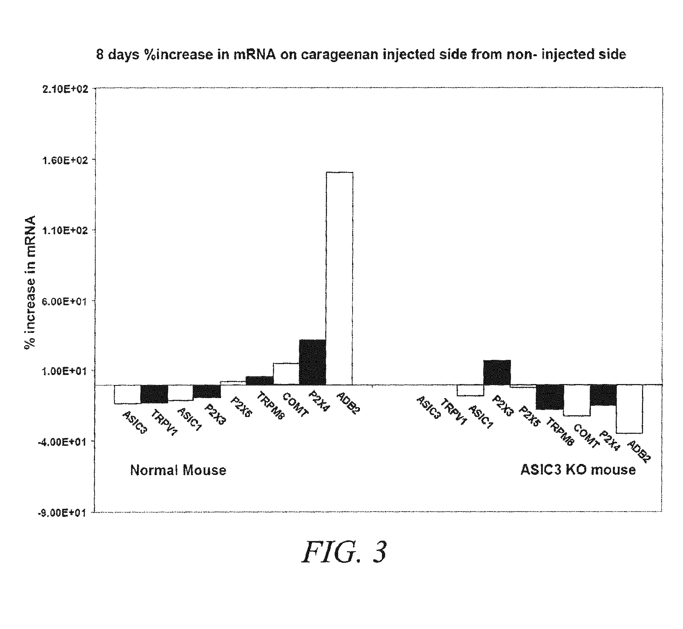

The role of ASIC3 in muscle inflammation was also assessed. The percent mRNA increase on carageenan injected side vs non-injected side after 24 hours was calculated and indicated a % increase in mRNA levels of the ASIC1, ASIC3, ADB2, COMT, P2X3, P2X4, P2X5, and TRPM8 receptors. The carageenan injection causes myaligia for at least 3 weeks. Specifically, mRNA levels of various receptors were assessed after carageenan injection into wither normal or ASIC3 KN mice. The results showed that ASIC3 is necessary for the increase of many receptors by muscle inflammation. The %mRNA increase on carageenan injected side vs non-injected side was also calculated after after eight days is shown in Figure 3.

### Example 4

Patients with Chronic fatigue syndrome (CFS) with and without fibromyalgia syndrome (FMS) were subjected to exercise. P2X4 receptor expression was determined before exercise and 0.5, 8, 24, and 48 hours after exercise. It was shown that CFS patients with and without FMS had greater increases in P2X4 receptors beginning at 8 hours after exercise.

Female patients with CFS were also examined in a similar fashion for expression of ASIC3 receptor expression. The results showed that ASIC3 receptors had greater increases in ASIC3 beginning at 8 hours after exercise.

Expression of β-2 adrenergic receptors in CFS patients with and without FMS was also determined. CFS patients with and without FMS were subjected to exercise. β-2 adrenergic receptors expression was determined before exercise and 0.5, 8, 24, and 48 hours after exercise. It was shown that CFS patients with and without FMS had greater increases in β-2 adrenergic receptors beginning at 30 minutes after exercise and persisting through 48 hours.

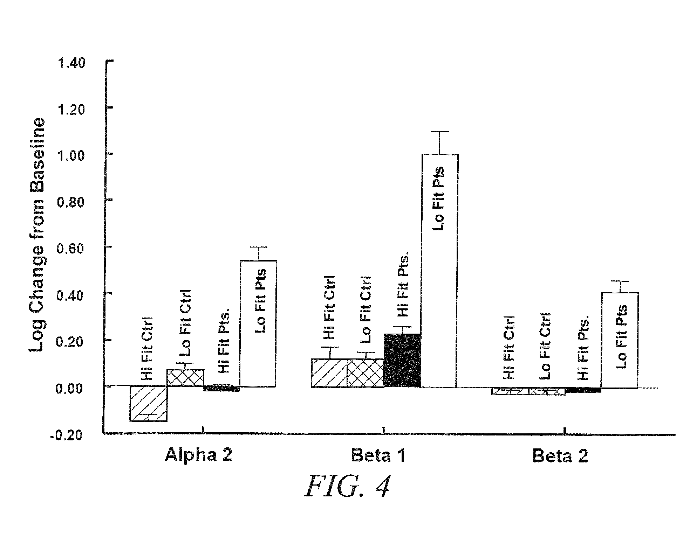

Expression of α-2, α-1 and β-2 adrenergic receptors in unfit CFS patients with and without FMS was also determined. Unfit CFS patients with and without FMS were subjected to exercise. α-2, β-1 and β-2 adrenergic receptor expression was determined before exercise and 0.5, 8, 24, and 48 hours after exercise. It was shown that CFS patients with and without FMS had greater increases in α-2, β-1 and β-2 adrenergic receptors beginning at 30 minutes after exercise and persisting through 48 hours (See Figure 4).

### Example 5

Data were obtained during the same experiments as reported in White et al. (White et al., 2009). Due to some data loss during mRNA assays, the sample for the present report included 19 CFS patients (15 females) and 16 Control subjects (11 females). One Control subject was excluded from these analyses as this subject developed Fibromyalgia within a year after being tested. All CFS patients met the CDC criteria for CFS (Fukuda et al., 1994). Previous evaluations had excluded all other known causes for persistent or relapsing fatigue in these CFS patients. Exclusion criteria included use of corticosteroids, sympathetic agonists, or prescription analgesics known to affect sympathetic, HPA or cytokine activity, uncontrolled cardiovascular or pulmonary disease. All patients were asked to discontinue any such drugs for 3 days before and 3 days during the exercise protocol (verified at the time of testing). Due to the 4-6 week downward-titration required, use of antidepressants was not discontinued and 8 of the 19 CFS patients (42%) and 5 of the 15 Controls (33%) were tested on antidepressants. Preliminary analyses indicated no differences in any gene expression measures between Controls tested with vs. without antidepressants and in CFS patients only one measure, baseline TLR4, showed a significant difference reflecting lower levels in those on antidepressants (P < .05) . All patients were also screened for fibromyalgia using the American College of Rheumatology (ACR) criteria, which includes presence of widespread pain for at least 6 months, and pain reported at 11 or more of 18 sites during Tender Point examination. Thirteen of the 19 CFS patients (68%) also met ACR criteria for FMS. For primary analyses, all 19 CFS Patients were compared to the 15 Controls. In separate secondary analyses, the 13 Patients meeting criteria for both CFS and FMS were compared to Controls.

All participants refrained from formal exercise other than the required exercise test for a period of 4 days beginning 48 hours preceding the exercise test until after the final (48 hr) blood sample had been drawn. Venous blood samples were obtained at baseline and at 0.5, 8, 24, and 48 hours post-exercise. To assess severity of pre-existing and exercise-related fatigue and myalgia symptoms, at the same time as each blood draw and also at the midpoint and immediately after completing the exercise task, the subject also provided numerical ratings of mental fatigue, physical fatigue and overall body pain using a 0-100 scale where 100 was defined as the greatest level of fatigue or pain the subject ever imagine experiencing. Immediately following the baseline blood draw, participants began the exercise session, as described below.

*Exercise Protocol:* A combined arm-leg cycle ergometer (Schwinn Air-Dyne) was used for the 25-min exercise test. In the first five minutes of exercise, subjects were asked to increase pedaling rate until 70% age-predicted maximal heart rate was achieved. Thereafter, workrate was adjusted in order to maintain this target heart rate throughout the protocol. Rating of Perceived Exertion (RPE) was obtained on a scale of 1-10 every 5 minutes, heart rate was recorded each minute, and blood pressure was measured at baseline, every 10 minutes during exercise, and upon completion of the exercise.

*mRNA extraction and analysis:* All blood processing and analyses were performed by personnel blinded as to the subject's group. At each of the 5 blood sampling times, blood was collected in EDTA solution. Seven minutes after blood collection, the blood was centrifuged at 3200 rpm (1315 x g- Clay Adams Compact II Centrifuge) for 12 minutes, plasma removed, and the white layer carefully collected in RNase-Out and RNA-later, then quickly frozen using a methanol-dry ice slurry, and stored at -80°. RNA was extracted using RNeasy kits (Qiagen 74104), according to manufacture's directions, and treated with RNase-free DNase-I (Qiagen 79254). Immediately following extraction, RNA was converted to a cDNA library using the ABI High Capacity cDNA Archive Kit.

The cDNA samples were stored at -80°C until analysis by quantitative real-time PCR. The cDNA libraries were analyzed using the ABI quantitative, real-time PCR system on the ABI Prism 7900 Sequence Detection System. ABI Taq Master Mix was used. Samples were loaded onto preplates with final robot loading of 385 well plates. Plates were spun to remove any air bubbles in the wells. Each sample was run in triplicate, with one "no template" control. Each 385 well plate contained samples from two subjects/patients, and all genes were analyzed on this same plate. Primer probes were: ASIC3 - Hs00245097_m1; P2X4 - Hs00175706_m1; P2X5 - Hs00175712_m1; TRPV1 - Hs00218912_m1, Adrenergic A2A - Hs00265081_s1; Adrenergic B1 - Hs02330048_s1; Adrenergic B2 - Hs00240532_s1; COMT - Hs00241349_m1; IL6-Hs00174131_m1; IL10 - Hs00174086_m1; TNFα - Hs00236874_m1; TLR4 - Hs00152937_m1; CD14 - Hs00169122_g1. Control primer probes included TF2B - Hs00155321_m1; β-Actin-Hs99999903_m1; PSMB6 - Hs00382586_m1; 18S - 4333760T; and GAPDH - Hs99999905_m1 (all from TaqMan Gene Expression Assays, ABI and commercially available from Life Technologies Corporation, Foster City, CA.).

All primer probes except for the adrenergic receptors and CD14 recognize sequences that cross splice sites, and therefore, make detection of genomic DNA unlikely. In all cases the genomic DNA is quenched and controls ran to ensure that genomic DNA does not contaminate the final results. All of these primer probes are designed and tested to be used together, and have similar efficiencies to help eliminated inaccuracy. Evaluation of controls indicated that TF2B varied the least, had count range that was similar to the genes of interest, and did not increase or decrease due to the exercise protocol. Real-time results were analyzed with SDS 2.1 (ABI), real-time inspected to determine artifacts, (loading errors, robot errors, thresholding errors, etc.). Count numbers were exported to an Excel spreadsheet, and analyzed according to the ddCT method described in ABI User Bulletin #2. Baseline data was collected relative to TF2B, and this baseline was used as the comparator for all measures taken after the exercise period (see statistical methods below for further analysis methods).

*Complete Blood Cell Counts (CBC):* Complete blood cell counts with differential subtyping were obtained by standard laboratory assays from separate blood samples collected in EDTA tubes. Measures obtained included total red blood cells, hematocrit and hemoglobin, platelets, and total white blood cells plus subtypes including monocytes, eosinophils, lymphocytes and granulocytes.

*Statistical Analysis:* Raw gene expression (mRNA) data were non-normally distributed; therefore, all such data were log transformed to yield distributions that were appropriately analyzed with parametric statistics. Baseline differences between Controls and Patients (and secondarily between CFS-FMS Patients and Controls) were determined with one-way ANOVA. Similar approaches were used to compare cardiovascular, work rate and RPE measures obtained during the exercise task.

Post-exercise values for each gene expression measure were first normalized relative to the same subject's baseline levels (1.00 = baseline). To minimize the increase in the likelihood of obtaining false positive results when examining multiple outcomes, two conservative steps were taken. First, instead of examining group differences at each of the 4 post-exercise time points, they were combined into a single measure labeled Area Under the Curve (AUC) from 0.5 through 48 hours after exercise. The post-exercise AUC for each gene expression measure was computed by summing the 0.5, 8, 24, and 48 hour values, after which these AUC measures were log transformed. Second, before examining univariate ANOVA results, the AUC measures were first grouped into 3 categories (metabolite detecting markers, adrenergic markers and immune markers), and then employed multivariate analyses of variance (MANOVA) to examine whether there were reliable group differences in each of these 3 outcome categories. Only when each of these three MANOVAs yielded significant overall effects of Group (F 4,23 = 2.21, *P* < .05, F 4,23 = 3.29, *P* < .015 and F5,23 = 3.29, *P* < .005 respectively one-tailed) did the examination of univariate effects of Group determined with one-way ANOVA proceed.

To examine whether Group differences were related to differences in age, gender, or BMI, the MANOVAs and univariate ANOVAs were repeated with each of these included in the model as covariates. In no instance was age or gender a significant covariate, and BMI was significant only for two measures: β-1 and β-2 AUC; thus, results for these measures are reported both before and after adjusting for BMI. In addition to group comparisons, Pearson r correlations were used to examine relationships between exercise variables, pain and fatigue ratings, and receptor expression. Unless otherwise indicated, significance was set at *P* <.05, for two-tailed tests. All data are presented as means and standard errors.

### Results

*Primary Analyses:* Table 3 gives age and BMI as well as the cardiovascular, work performance and subjective exertion data for all CFS Patients vs. Controls. Groups did not differ in age or in blood pressure or heart rate levels either at baseline or during the exercise task (all *P* > .05). The CFS Patients did have significantly higher BMI than Controls (*P* < .009), and they reported significantly higher mean RPE levels (*P* < .0001) even though they exercised at significantly lower work rates than Controls (*P* < .0001).

A study of the mental and physical fatigue and pain ratings before, during and after 48 hours of the exercise task indicated that CFS Patients had significantly higher ratings than Controls for all three symptoms even at baseline, and these differences continued at all other times of measurement (Group Effect F 1,32 = 30.88, 52.33, and 25.60, *P* < .0001 for mental and physical fatigue and pain, respectively.) There were also differences from baseline levels in these symptoms during and after exercise (Time Effects F 4,128 = 2.62, 2.66 and 3.07, *P* < .04 respectively). During on-going exercise, both Controls and CFS Patients showed increases over baseline levels in physical fatigue (*P* < .05), but only CFS Patients continued to show increased physical fatigue at 0.5, 8, 24 and 48 hours post-exercise (*P* < .05). Also, only CFS Patients showed significantly increased levels of pain at 8, 24 and 48 hours and increased mental fatigue at 8 and 48 hours after exercise (*P* < .05).

**Table 3. Subject Characteristics for Controls and Patients**

| | *Controls (n=15; 11F)* | *All Patients (n=19; 15F)* | *df* | *F* | *P* |
|---|---|---|---|---|---|
| Age (years) | 35.6 ± 3.0 | 42.2 ± 2.7 | 1,32 | 2.69 | *NS* |
| *Body Mass Index (kg*/*m2)* | 22.9 ± 0.73 | ***27.0* ±*1.18**** | ***1,32*** | ***7.69*** | ***.003*** |
| Resting SBP (mmHg) | 126 ± 3.1 | 122 ± 3.4 | 1,32 | 0.59 | *NS* |
| Resting DBP (mmHg) | 77 ± 2.5 | 79 ± 2.2 | 1,32 | 0.13 | *NS* |
| Exercise SBP (mmHg) | 159 ± 8.9 | 146 ± 5.5 | 1,32 | 0.60 | *NS* |
| Exercise DBP (mmHg) | 89 ± 3.4 | 88 ± 2.1 | 1,32 | 0.19 | *NS* |
| Exercise HR (BPM) | 130 ±1.6 | 123 ± 3.0 | | | |
| Exercise HR (%PMHR) | 70.6 ± 0.78 | 69.3 ± 1.18 | 1,32 | 0.82 | *NS* |
| Exercise WR (Kcal/kg/min) | 7.5 ± 0.42 | **4.3 ± 0**.**36*** | **1,32** | **34.85** | **.0001** |
| Exercise RPE | 3.0 ± 0.21 | **4.8 ± 0.34*** | **1,32** | **17.60** | **.0001** |

| | | | | | |
|---|---|---|---|---|---|
| BMI = body mass index; SBP = systolic blood pressure; DBP = diastolic blood pressure; %PMHR = percent of age-predicted maximal heart rate; WR = workrate; RPE = rating of perceived exertion; *NS* = non-significant; * and **bold** indicates significant difference from Controls, *P*<.05. | | | | | |

Baseline levels of gene expression markers are provided in Table 4. β-2 adrenergic receptor expression was significantly lower in CFS Patients vs. Controls, while α-2a adrenergic receptor expression was marginally higher (*P* < .09). None of the other gene expression measures differed at baseline between Groups.

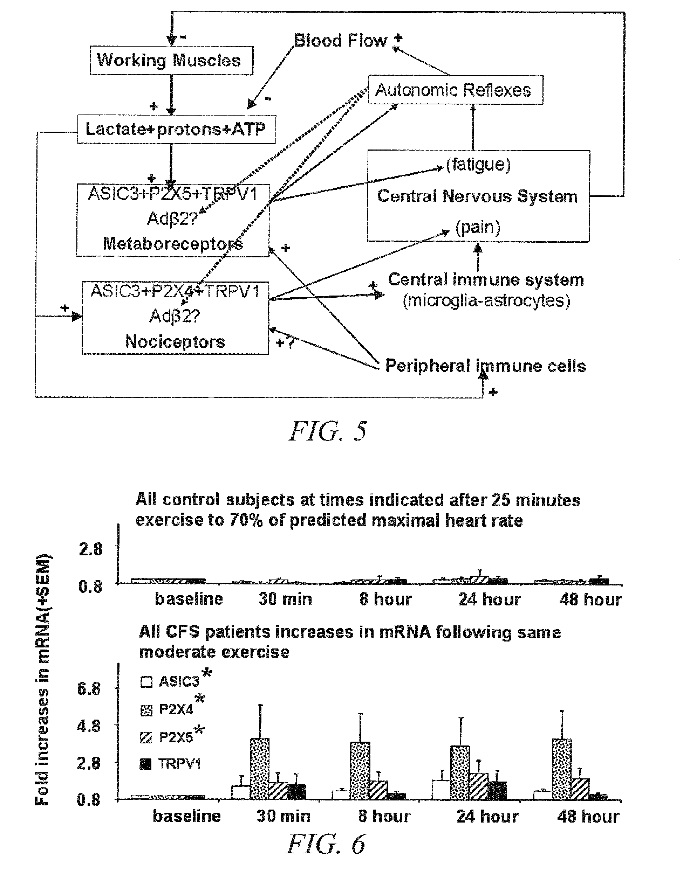

For post-exercise AUC measures, initial MANOVAs yielded significant effects of Group for metabolite detecting markers, adrenergic markers and immune markers. Subsequent univariate ANOVAs for metabolite sensing markers yielded significant Group differences in post-exercise AUC between CFS Patients and Controls for ASIC3 (*P* < .012); P2X4 (*P* < .002), and P2X5 (*P* =.028), but only a non-significant trend for TRPV 1 (*P* = .11). Depicted in Figure 6 as fold increases relative to baseline levels (where 1.0 fold = baseline), CFS Patients showed consistently greater mean increases in ASIC3, P2X4 and P2X5 gene expression than Controls across all post-exercise time points.

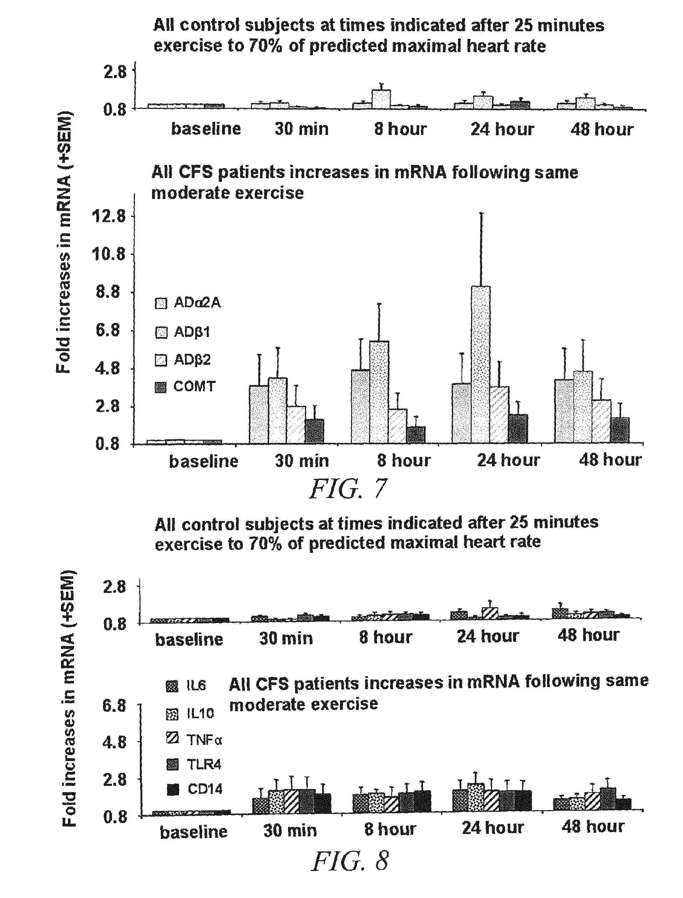

Significant effects of Group were also obtained in the univariate ANOVAs for all of the post-exercise adrenergic AUC markers, due to consistently greater mean increases in α2a (*P <* .003), β-1 *(P* < .001) and β-2 *(P* < .001) adrenergic receptor expression, and greater COMT (*P* < .009) gene expression in the CFS Patients vs. Controls, as shown in Figure 7. For β-1 and β-2 where BMI was observed to be a significant covariate, ANOVAs were repeated including BMI in the model and Group differences in post-exercise AUC β-2 receptor expression were still significant. Surprisingly, for post-exercise AUC increase in β-1 receptor expression, where mean group differences had been greater than any other gene expression marker, inclusion of BMI in the model caused the effect of Group to become non-significant (*P* = .19).

For post-exercise immune markers, univariate ANOVAs showed significant group differences in TLR4 (*P* < .016), CD14 (P=.05), and IL-10 *(P* < .0001) AUC expression, but not in IL-6, or TNF-α AUC expression. For both immune measures, CFS Patients showed greater post-exercise increases than Controls, as depicted in Figure 8.

Correlations between post-exercise fatigue and pain and the post-exercise AUC gene expression measures are depicted in Table 5 along with the intercorrelations among the various gene expression measures.

**Table 4. Baseline values for all mRNAs relative to β-Actin in Controls and Patients.**

| ***Metabolite Detecting*** | | | | | | | | |
|---|---|---|---|---|---|---|---|---|
| | Controls Baseline | Patients Baseline | df Baseline | F Baseline | P (1-tailed) Baseline | AUC df | AUC F | AUC P (1-tail) |
| **ASIC3** | 8.72E-03 1.05E-03 | 1.01E-02 ± 1.19E-03 | 1,26 | 0.73 | 0.20 | 1,29 | 5.55 | **0.012** |
| **P2X4** | 1.29E-01 ± 1.86E-02 | 1.45E-01 ± 2.64E-02 | 1,27 | 0.21 | 0.33 | 1,32 | 8.37 | **0.002** |
| **P2X5** | 2.37E-01 ± 3.68E-02 | 2.47E-01 ± 4.69E-02 | 1,26 | 0.03 | 0.43 | 1,30 | 3.78 | **0.028** |
| **TRPV1** | 9.82E-03 1.33E-03 | 1.13E-02 ± 1.37E-03 | 1,23 | 0.57 | 0.23 | 1,26 | 1.50 | 0.117 |

| | **Adrenergic** | | | | | | | |
|---|---|---|---|---|---|---|---|---|
| | Controls | Patients | | | | | | |
| **α2a** | 7.37E-03 ± 1.59E-03 | **1.53E-02^{τ}** ± 3.56E-03 | 1,25 | 3.11 | **.045** | 1,28 | 6.68 | **0.003** |
| **β-1** | 4.73E-03 ± 4.73E-03 | 1.61E-01 ± 1.49E-01 | 1,27 | 0.89 | 0.18 | 1,30 | 10.06 | **0.001** |
| **β-2** | 1.47E+00 ± 2.75E-01 | **9.06E-01*** ± 1.09-01 | 1,27 | 4.26 | **0.03** | 1,30 | 12.80 | **0.0001** |
| **COMT** | 2.07E-01 ± 2.80E-02 | 2.28E-01 ± 3.96E-02 | 1,25 | 0.17 | 0.34 | 1,29 | 5.61 | **0.009** |

| | **Immune** | | | | | | | |
|---|---|---|---|---|---|---|---|---|
| | Controls | Patients | | | | | | |
| **IL-6** | 6.83E-03 ± 4.33E-03 | 3.71E-03 ± 8.77E-04 | 1,27 | 0.60 | 0.22 | 1,28 | 1.37 | 0.119 |
| **IL-10** | 3.93E-03 ± 6.60E-04 | 4.81E-02 ± 4.47E-02 | 1,25 | 0.90 | 0.18 | 1,28 | 13.30 | **0.0001** |
| **TNFα** | 4.70E-02 ± 7.84E-03 | 7.35E-02 ± 2.24E-02 | 1,27 | 1.05 | 0.15 | 1,30 | 1.21 | 0.124 |
| **TLR4** | 7.38E-01 ± 1.93E-01 | 5.82E-01 ± 8.35E-02 | 1,27 | 0.63 | 0.22 | 1,30 | 4.33 | **0.016** |
| **CD14** | 1.81E+00 ± 2.32E-01 | 2.00E+00 ± 3.89E-01 | 1,25 | 0.17 | 0.34 | 1,28 | 2.95 | **0.044** |

**Table 5. Correlations of Post-exercise AUC Gene Expression Measures with Post-exercise AUC Fatigue and Pain and with Each Other**

| | ASIC3 | P2X4 | P2X5 | TRPV1 | Alpha-2a | Beta-1 | Beta-2 | COMT | IL-10 | TLR4 |
|---|---|---|---|---|---|---|---|---|---|---|
| Mental fatigue | +.53 | +.62 | +.38 | +.43 | +.63 | +.41 | +.58 | +.51 | +.64 | +.48 |
| | | | | | | | | | | |
| Physical fatigue | +.53 | +.58 | +.37 | +.41 | +.60 | +.43 | +.58 | +.48 | +.63 | +.45 |
| Pain | +.54 | NS | NS | +.38 | +.43 | NS | +.50 | NS | +.47 | NS |
| ASIC3 | --- | +.60 | +.76 | +.83 | +.51 | NS | +.62 | +.77 | +.53 | +.55 |
| P2X4 | | --- | +.58 | +.63 | +.70 | +.51 | +.65 | +.72 | +.80 | +.64 |
| P2X5 | | | --- | +.72 | +.38 | NS | +.51 | +.78 | NS | +.39 |
| TRPV1 | | | | --- | +.39 | NS | +.49 | +.79 | +.59 | +.61 |
| Alpha-2a | | | | | --- | +.77 | +.86 | +.35 | +.63 | +.48 |
| Beta-1 | | | | | | --- | +.89 | NS | +.63 | +.50 |
| Beta-2 | | | | | | | --- | +.42 | +.70 | +.59 |
| COMT | | | | | | | | --- | +.43 | +.61 |
| IL-10 | | | | | | | | | --- | +.67 |

| | | | | | | | | | | |
|---|---|---|---|---|---|---|---|---|---|---|
| All listed correlations P<.05. For those correlations > +.44, P<.01. | | | | | | | | | | |

As indicated by these relationships, post-exercise increases in metabolite detecting receptor, adrenergic receptors and certain immune markers occurred in parallel and greater increases in all of them were all associated with greater fatigue. Post-exercise pain symptoms were not associated with P2X4, P2X5 or β-1 receptor expression increases, but were linked to ASIC3, TRPV1, α-2a, β-2 and IL-10 increases. When examined among the CFS Patients by themselves, exercise work rate was not correlated with post-exercise fatigue or pain or with increase any metabolite detecting or immune marker, and it was inversely related to increases in β-1 and β-2 AUC receptor expression (r = -.71 and -.60, *P* < .05).

*Secondary Analyses:* Although heart rate level and percent of age of predicted maximum heart rate were quite closely matched between Groups during exercise, Controls did exercise at a higher work rate than CFS Patients. However, when repeated, the univariate ANOVAs for post-exercise AUC gene expression comparing Groups including exercise work rate as a covariate in the model were found not to be a significant covariate for any measure except Beta-1 receptor expression (*P* < .02). As with inclusion of BMI as a covariate, covarying for work rate did cause the Group effect for post-exercise β-1 receptor expression to become non-significant. This pattern indicates that high BMI, low exercise work and greater post-exercise increase in β-1 receptor expression are all interlinked in CFS.

Univariate ANOVAs were also repeated after dropping the 6 CFS Patients who did not also meet ACR criteria for FMS. The findings were essentially unchanged from those reported above except that for two immune markers, TLR4 and CD14, differences between CFS-FMS Patients and Controls were then only marginally significant (*P* =.07). For all other measures where significant Group differences were obtained in the primary analyses, the same measures showed significant differences between the CFS-FMS Patients and Controls in the secondary analyses. This is indicative of the great overlap between the two Patients groups, and indicates that the observed patterns of results apply to patients with FMS as well as to those with CFS.

*CBC counts:* CBC counts were taken from all patients and controls. As detailed in White et al, at pre-exercise baseline, total white blood cell counts from CFS patients were slightly higher than controls, and specifically, monocyte, eosinophil, and granulocyte counts were significantly higher in patients than in controls (P<.05). Counts in both patients and controls were significantly increased from baseline at 8 hours but returned to baseline levels at 24 and 48 hours. However, hemoglobin levels were lower rather than higher in patients, and these directionally opposite effects in white vs. red cell counts indicate that hydration differences were not responsible. These CBC differences were not clinically significant, and similar differences have been reported by others. An initial analysis indicated that most of the mRNA for the genes described below was from the monocyte-lymphocyte fraction, with the exception of COMT, ADB2 and TLR4 mRNA, which had equal quantities in the monocyte-lymphocyte and granulocyte fractions.

*mRNA Levels:* Baseline mRNA levels for all of the genes examined were not different between controls and patients except for adrenergic β-2 which was slightly but significantly less and α-2A which weakly tended to be higher in the CFS patients. Decreased β-2 and increased α-2A receptors in the vasculature lead to enhanced total vascular resistance. These findings are also consistent with previous gene expression studies of CFS patients studied only at rest which have not observed consistent increases in any of these mRNAs with the exception of TNFα. (Powell et al., 2003). However, following 25 minutes of moderate, whole-body exercise (when summed at times including 30 minutes, 8 hours, 24 hours and 48 hours following exercise), mRNA levels for nearly all of the genes examined here were greatly increased in the CFS patients relative to controls. Controls exhibited no significant increased in expression of any of the genes. The increases in mRNA included those for ASIC3, P2X4, P2X5, α2A, β1, β2, COMT, TLR4 and CD14. mRNAs not increased by exercise in CFS patients were IL6 and TNFα. These cytokines had previously been shown to increase following heavy exercise in normal subjects (Nieman et al., 2005). However, investigators have also found that while cytokine levels of IL6 and TNFα in blood were increased, gene expression as determined with quantitative PCR methods was not increased for IL6 and TNFα (Moldoveanu et al., 2000; Natelson et al., 1996; Nieman et al., 2006). However, mRNA for these cytokines taken from exercised muscle did show increases in other experiments (Nieman et al., 2003)

Inaddition, mRNA increases were observed in patients as early as 30 minutes following exercise, a time period previously observed to show no increases in control subjects, even with high intensity exercise (Light et al., 2007). In the present study with moderate exercise, control subjects also had no increases in mRNA levels at 30 minutes following exercise. Increases in mRNA, with the exception of β1, were not influenced by BMI, or work rate, and were present in both CFS patients with, and without co-morbid CFS. Underscoring the relationship between the primary symptoms defining CFS, strong correlations were found between both summed post exercise physical and mental fatigue and the summed increases in mRNA of the genes listed above (see Table 5). The marked alterations caused by exercise in gene expression from circulating leukocytes of CFS patients observed in the present experiments indicate that they can be used as an objective marker for CFS.

In the present sample, ∼90% of the CFS patients were distinguished from controls using 4 of the genes measured (P2X4, β1, β2, IL10). If all of the 9 genes that were increased in CFS-FMS patients relative to controls were used, a similar result was found, However, the condition that is defined by expression of these genes is exaggerated exercise induced fatigue, and not necessarily CFS. The genes used here can discriminate other causes of fatigue, and/or other genes can be used to discriminate known causes of fatigue, such as viral infections, abnormalities in metabolism, etc. A test based on the present findings using a commonly assayed medium (blood) can provide an objective measure for fatigue that is greatly outside the norm. This information can be helpful to CFS patients (or patients with fatigue from other causes) that often have difficulty convincing physicians that the condition they have is very much outside the norm. In addition, tests of this type help distinguish subtypes of CFS, as well as possible causes of these subtypes. Finally, these tests can be used to decide on appropriate treatments for individual patients, and to monitor effectiveness of treatments for CFS.

The present example found substantial levels of adrenergic α2A, β1, and β2 receptor mRNAs, as well as high levels of COMT, a major enzyme involved in inactivating epinephrine and norepinephrine. Expression of all of these mRNAs in leukocytes was increased by exercise in CFS patients much more than in controls. This greatly enhanced upregulation indicates powerful upstream signaling on the immune system. IL10 mRNA was upregulated by exercise in CFS patients compared with controls. IL10 is an anti-inflammatory cytokine, inhibiting the production of pro-inflammatory cytokines such as TNFα. The moderate exercise utilized here had almost no effect on IL10 mRNA in controls. Intense exercise has been shown to increase IL10 mRNA in athletes (and IL10 cytokine levels) (Nieman et al., 2005; Nieman et al., 2006). However, the IL10 cytokine levels in CFS patients were lower than controls at all time points than controls in the present study. The mRNAs for TLR4 and CD 14 were also increased by exercise in CFS patients and not in controls. TLR4 is an immune function receptor that transduces bacterial invasion by detecting the lipopolysaccaride coat on bacteria whereas CD 14 is a co-receptor that combines with TLR4 to detect bacterial invasion.

The examples reported here indicate large and rapid increases in gene expression of leukocytes following 25 minutes of moderate exercise in CFS patients that are not seen in control subjects. Genes that increased included those that can detect increases in muscle produce metabolites (ASIC3, P2X4, P2X5) genes that are essential for sympathetic processes (adrenergic α2A, β1, and β2, as well as COMT) and immune function genes (IL10, TLR4, and CD 14). These gene alterations indicate a potential role for sensory signaling alterations in the symptoms of CFS. They also indicate that a blood test can be devised as an objective biomarker for fatigue and muscle pain in CFS.

### Example 6

*Participants*: Nineteen CFS patients and 17 controls participated in this study. All patients met the CDC 1994 diagnostic criteria for CFS (Fukuda et al., 1994) and had been evaluated previously by a practitioner who excluded all other causes for their severe persistent or relapsing fatigue and related symptoms before assigning a diagnosis of CFS. Subjects taking corticosteroids, sympathetic agonists, or prescription analgesics known to affect sympathetic, HPA, or cytokine activity were excluded, as were subjects with uncontrolled cardiovascular or pulmonary disease. In addition, all patients underwent screening for fibromyalgia (FMS) using the American College of Rheumatology (ACR) criteria, including presence of widespread pain symptoms for at least 6 months and pain reported at 11 or more of 18 sites during Tender Point examination. Fourteen of the CFS patients also met ACR diagnostic criteria for FMS. Subject characteristics are presented in Table 6.

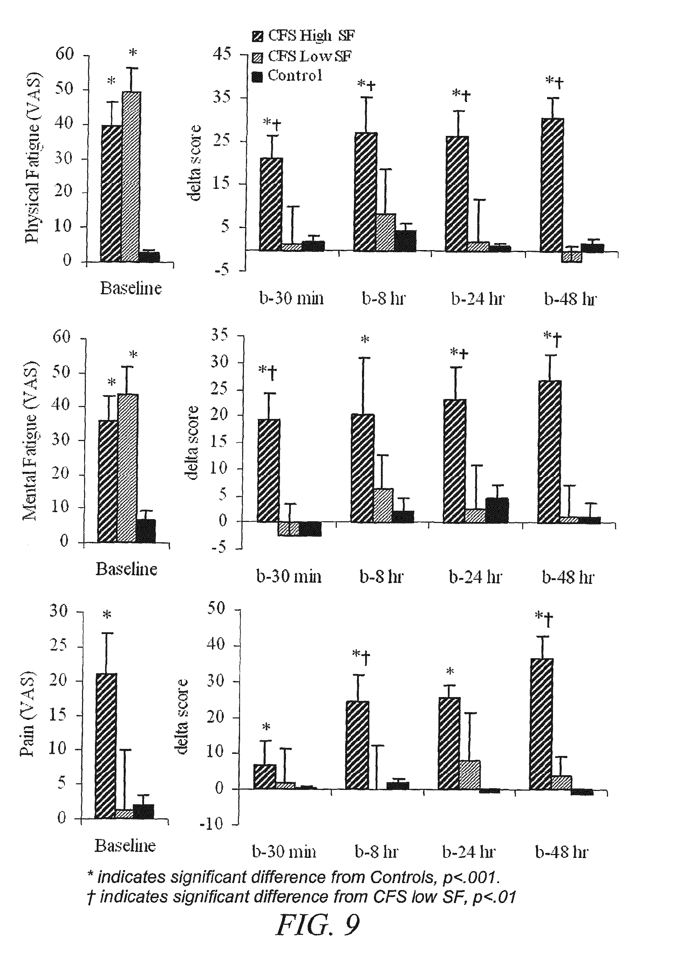

Results are presented for all CFS Patients vs. Controls as well as for patients subgrouped into those showing High vs. Low Symptom Flare (SF). Subgrouping was accomplished by examining baseline to 48-hr post-exercise changes in physical fatigue ratings. Those CFS patients demonstrating increases in physical fatigue at or above the group median were classified as High SF while those patients below the median were classified as Low SF. Figure 9 illustrates the results of this subgrouping, and also shows that relative to the low SF Patients as well as to Controls, the High SF Patients had greater increases in mental fatigue and pain ratings as well as physical fatigue 8-48 hours post-exercise, which supports this definition of symptom flare.

*Protocol:* All participants refrained from formal exercise other than the required exercise test for a period of 4 days beginning 48 hours preceding the exercise test until after the final 48 hr post-exercise blood sample had been drawn. Venous blood samples were obtained at baseline and at 0.5, 8, 24, and 48 hours post-exercise. To minimize degradation of sample, they were centrifuged immediately and serum was flash frozen within 18 minutes, and then kept at -80°C until assay. Leukocytes were also obtained from most of these same subjects (and from some additional subjects) which were used to determine gene expression of metabolite detecting and adrenergic receptors and a small subset of immune markers; these gene expression results are reported in a separate paper (Light, Hughen, White & Light, 2009). To assess severity of pre-existing and exercise-related fatigue and myalgia symptoms, at each of these blood sampling times, the subject also provided numerical ratings of mental fatigue, physical fatigue and overall body pain using a 0-100 scale where 100 was defined as the greatest level of fatigue or pain the subject ever imagined experiencing. Immediately following the baseline blood draw, participants began the exercise session, as described below.

*Exercise Protocol:* A combined arm-leg cycle ergometer (Schwinn^{®} Airdyne^{®}) was used for the 25-min exercise test. In the first five minutes of exercise, subjects were asked to increase pedaling rate until 70% age-predicted maximal heart rate was achieved. Thereafter, workrate was adjusted in order to maintain this target heart rate throughout the protocol. Rating of Perceived Exertion (RPE) was obtained on a scale of 1-10 (Borg, 1982) every 5 minutes, and blood pressure was measured at baseline, every 10 minutes during exercise, and upon completion of the exercise.

*Cytokine Assays:* A multiplexed fluorescent microsphere immunoassay developed at the ARUP Institute for Clinical and Experimental Research (Salt Lake City, UT) was used to simultaneously assess the concentration of 12 cytokines/ligands, including 6 pro-inflammatory (IL-1β, IL-2, IL-12, TNFα, soluble CD40 Ligand [CD40L] and IFNγ), 3 anti-inflammatory/ regulatory (IL-4, IL-10, IL-13), and two categorized as exercise-responsive with mixed pro-and anti-inflammatory effects (IL-6 and IL-8), all from only 75 uL of serum (Martins, Pasi, Litwin, & Hill, 2004). The Luminex Multi-Analyte Profiling system (Luminex 100, Luminex Corp, Austin, TX) is a flow cytometry based instrument that allows multiple analytes to be assayed simultaneously in a single sample (Fulton, McDade, Smith, Kienker, & Kettman, 1997).

*Complete Blood Cell Counts (CBC):* Complete blood cell counts with differential subtyping were obtained by standard laboratory assays from separate blood samples collected in EDTA tubes. Measures obtained included total red blood cells, hematocrit and hemoglobin, platelets, and total white blood cells plus subtypes including monocytes, eosinophils, lymphocytes and granulocytes.

*Statistical Analysis:* Raw cytokine data were non-normally distributed; therefore, a constant was added to the actual value at each time point for each cytokine, and then all cytokine data were log transformed to yield distributions to be appropriately analyzed with parametric statistics. Baseline differences between Controls and Patients and patient subgroups were determined with one-way ANOVA (SPSS v. 16). When group effects were significant, *post hoc* Games-Howell analyses were performed to identify which groups differed.

Two (CFS Patients vs. Controls) by four (baseline to 0.5, 8, 24, and 48 hr post-exercise) repeated measures ANOVAs were used to analyze changes in cell counts and cytokines. In the second set of analyses, High and Low SF Patients were compared to each other and to Controls using 3 x 4 repeated measures ANOVAs. Simple contrasts comparing each post-exercise time point to baseline were built into the model to determine which time points were significantly different from baseline, and Games-Howell *post hoc* analyses were used to determine group differences. Relationships between baseline and post-exercise cytokines vs. pain and fatigue symptoms were examined using Pearson correlations. Significance level was set at .05. Data are presented as means and standard errors.

### Results

All subjects, including the most deconditioned patients, completed the 25 min exercise protocol. Exercise heart rate, expressed as percent of age-predicted maximal heart rate (PMHR), shows that the relative exercise intensity was not different between Controls and CFS Patients (see Table 6). Although groups exercised at the same relative intensity, average work rate for All Patients and both patient subgroups was significantly lower (F[2,35] = 15.79, *p*<.001) and their RPE was significantly higher compared to Controls (F[2,35] = 13.28, *p*<.001). There were no group differences for age or resting or exercise blood pressure, but All Patients and specifically the high SF subgroup had higher body mass index (BMI) than Controls (F[2,35] = 4.62, *p*<.05).

**Table 6. Subject characteristics for controls, all patients, and patient subgroups**

| | Controls (n=17; 11F) | All Patients (n=19; 15F) | CFS High SF (n=11;8F) | CFS Low SF (n=8; 7F) |
|---|---|---|---|---|
| Age (years) | 33.6 ± 2.7 | 40.1 ± 2.8 | **42.9 ± 3.5*** | 38.2 ± 2.8 |
| BMI (kg·m²) | 22.9 ± 0.65 | **26.9 ± 1.36*** | **28.4 ± 1.77*** | 25.0 ± 2.07 |
| Resting SBP (mm Hg) | 123 ± 3.8 | 122 ± 3.5 | 130 ± 4.1 | **112 ± 3.9^{*†}** |
| Resting DBP (mm Hg) | 77 ± 2.1 | 80 ± 2.4 | 85 ± 2.7 | **72 ± 2.8^{†}** |
| Exercise HR (% PMHR) | 71.4 ± 0.76 | 69.9 ± 1.13 | 71.6 ± 1.40 | 67.6 ± 1.71 |
| Exercise WR (Kcal·kg⁻¹·min⁻¹) | 6.9 ± 0.33 | **4.2 ± 0.35^{*}** | **4.4 ± 0.49^{*}** | **4.0 ± 0.49^{*}** |
| Exercise RPE (0-10) | 2.8 ± 0.15 | **4.6 ± 0.35^{*}** | **4.1 ± 0.36^{*}** | **5.2 ± 0.63^{*}** |
| Exercise SBP (mm Hg) | 146 ± 5.8 | 147 ± 6.1 | 157 ± 10.2 | 136 ± 5.8 |
| Exercise DBP (mm Hg) | 86 ± 1.7 | 89 ± 2.4 | 92 ± 3.8 | 85 ± 3.0 |

| | | | | |
|---|---|---|---|---|
| Values are means ± SE. CFS High SF = CFS patients with physical fatigue scores at or above patient median at 48 hrs post-exercise (high symptom flare); CFS Low SF = CFS patients with physical fatigue scores below the median at 48 hrs post-exercise (low symptom flare); BMI = body mass index; SBP = systolic blood pressure; DBP = diastolic blood pressure; %PMHR = percent of age-predicted maximal heart rate; WR = work rate; RPE = rating of perceived exertion * indicates significant difference from Controls, p<.05 † indicates significant difference between CFS High SF and CFS Low SF, p<.05 | | | | |

*Baseline Blood Cell Counts and Cytokines:* At baseline, the high SF patients showed significantly higher total white cells than Controls, indicative of pre-existing immune activation, while the low SF Patients had normal white cells (see Table 7). Subtypes that were elevated in the SF Patients included eosinophils, granulocytes and basophils while monocytes and lymphocytes were not reliably increased. In the low SF but not the high SF Patients, baseline hemoglobin levels were significantly lower than Controls, and platelet counts did not differ by group. For baseline cytokines, group differences were seen for only one pro-inflammatory marker, CD40L, which was lower in All Patients than in Controls (F[2,35] = 3.66, *p*<.05), an effect principally due to the high SF subgroup which had the lowest CD40L (see Table 8).

**Table 7. Baseline cell counts for controls, all patients, and patient subgroups**

| | Controls (n=17; 11F) | All Patients (n=15; 12F) | CFS High SF (n=7; 5F) | CFS Low SF (n=8; 7F) |
|---|---|---|---|---|
| White cells (K·µL⁻¹) | 5.99 ± 0.35 | 6.89 ± 0.48 | **8.08 ± 0.59^{*‡}** | 6.59 ± 0.46 |
| Platelets (K·µL⁻¹) | 271 ± 13 | 283 ± 17 | 293 ± 24 | 277 ± 24 |
| Monocytes (K·µL⁻¹) | 0.31 ± 0.02 | 0.37 ± 0.03 | 0.41 ± 0.07 | 0.35 ± 0.04 |
| Eosinophils (K·µL⁻¹) | 0.16 ± 0.04 | 0.27 ± 0.04 | **0.36 ± 0.08^{†}** | 0.21 ± 0.04 |
| Lymphocytes (K·µL⁻¹) | 1.88 ± 0.12 | 2.24 ± 0.18 | 2.37 ± 0.33 | 2.00 ± 0.19 |
| Granulocytes (K·µL⁻¹) | 3.45 ± 0.25 | **4.27 ± 0.30^{*}** | **4.92 ± 0.51^{*}** | 3.84 ± 0.32 |
| Basophils (K·µL⁻¹) | 0.04 ± 0.01 | 0.06 ± 0.01 | **0.10 ± 0.00*^{§}** | 0.03 ± 0.02 |
| Red cells (M·µL⁻¹) | 4.72 ± 0.12 | 4.66 ± 0.11 | 4.78 ± 0.19 | 4.58 ± 0.12 |
| Hemoglobin (g·dL⁻¹) | 14.8 ± 0.31 | 14.1 ± 0.32 | 14.3 ± 0.59 | **13.9 ± 0.35^{†}** |

| | | | | |
|---|---|---|---|---|
| Values are means ± SE. CFS High SF = CFS patients with physical fatigue scores at or above patient median at 48 hrs post-exercise (high symptom flare); CFS Low SF = CFS patients with physical fatigue scores below the median at 48 hrs post-exercise (low symptom flare). * indicates significant difference from Controls, p<.05 § indicates significant difference from CFS Low SF, p<.05 † indicates borderline difference from Controls, .05<p<.10 ‡ indicates borderline difference from CFS Low SF, .05<p<.10 | | | | |

*Effect of Moderate Exercise on Perceptions of Pain and Fatigue:* The subjects groups were scored for physical fatigue, mental fatigue, and pain from baseline to 30 min and 8, 24, and 48 hrs post-exercise. At baseline, both the High SF and the Low SF Patients with CFS demonstrated significantly higher scores for mental fatigue and physical fatigue, and the high SF subgroup also showed higher overall body pain compared to Controls (F[2,34] > 26.24, *p*<.001; see Figure 9). After exercise, only the high SF Patients showed significantly worsening of their fatigue and pain symptoms, differing significantly from Controls at all post-exercise times for all 3 symptoms (F[2,33] > 7.04, *p*<.003). High SF Patients also showed greater increases in physical fatigue than low SF Patients at all times, greater increases in mental fatigue at 30 min, 24 and 48 hours, and greater increases in pain at 8 and 48 hours (*p*<.01). In the high SF Patients, mean pain ratings increased more and more over the 48 hours after moderate exercise, and fatigue ratings also showed no evidence of decreasing over time.

**Table 8. Baseline cytokines for controls, all patients, and patient subgroups**

| | Controls (n=17; 11F) | All Patients (n=19; 15F) | CFS High SF (n=11;8F) | CFS Low SF (n=8; 7F) |
|---|---|---|---|---|
| Pro-Inflammatory | | | | |
| IL1-β | 2.04 ± 0.02 | 2.02 ± 0.01 | **2.00 ± 0.00^{†}** | 2.04 ± 0.02 |
| IL-2 | 1.89 ± 0.02 | 1.88 ± 0.00 | 1.87 ± 0.00 | 1.88 ± 0.01 |
| IL-12 | 1.50 ± 0.02 | 1.48 ± 0.01 | 1.46 ± 0.00 | 1.49 ± 0.02 |
| IFNγ | 1.05 ± 0.02 | 1.02 ± 0.00 | 1.01 ± 0.00 | 1.02 ± 0.01 |
| CD40L | 2.29 ± 0.05 | **1.94 ± 0.12^{*}** | **1.89 ± 0.16^{*}** | 2.02 ± 0.17 |
| TNFα | 1.68 ± 0.02 | 1.64 ± 0.01 | **1.63 ± 0.00^{†}** | 1.66 ± 0.01 |

| Anti-Inflammatory | | | | |
|---|---|---|---|---|
| IL-4 | 0.61 ± 0.02 | 0.61 ± 0.01 | **0.59 ± 0.00^{§}** | 0.64 ± 0.02 |
| IL-10 | 1.36 ± 0.05 | 1.18 ± 0.11 | **1.03 ± 0.15^{*}** | 1.35 ± 0.16 |
| IL-13 | 2.60 ± 0.02 | 2.63 ± 0.03 | 2.59 ± 0.02 | 2.67 ± 0.05 |

| Mixed Response | | | | |
|---|---|---|---|---|
| IL-6 | 1.34 ± 0.02 | 1.34 ± 0.01 | 1.33 ± 0.01 | 1.36 ± 0.03 |
| IL-8 | 1.03 ± 0.02 | 1.04 ± 0.03 | 1.00 ± 0.00 | 1.05 ± 0.06 |

| | | | | |
|---|---|---|---|---|
| Values are log transformed means ± SE * indicates significant difference from Controls, p<.05 § indicates significant difference from CFS Low SF, p<.05 † indicates borderline difference from Controls, .05<p<.10 | | | | |

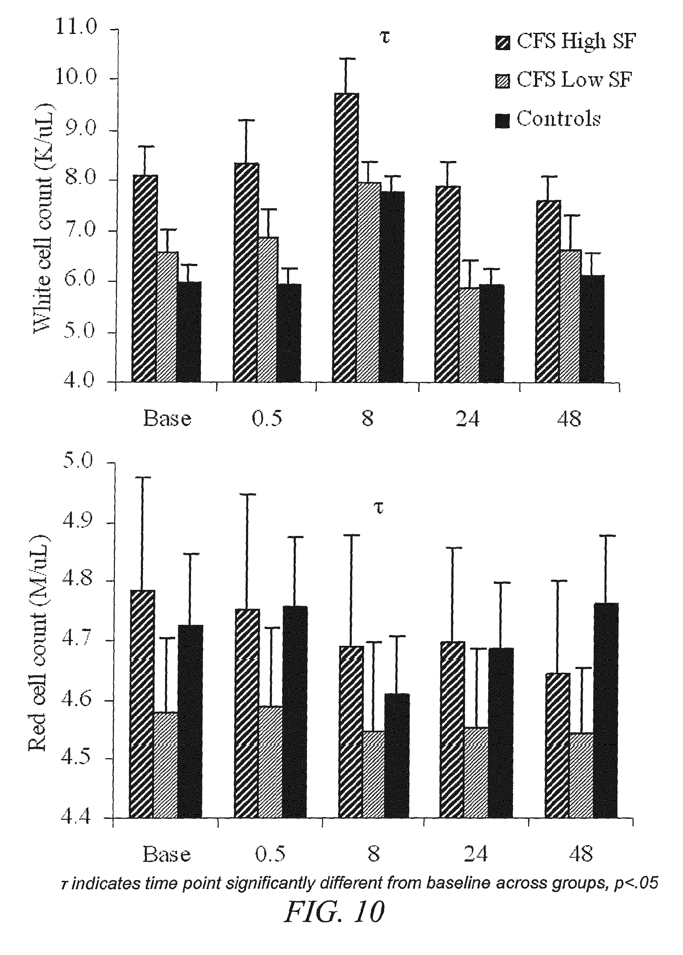

*Effect of Moderate Exercise on Blood Cell Counts:* Post-exercise changes in blood cell count measures did not differ between High and Low SF Patients, although the High SF subgroup maintained their higher white cell counts relative to Controls. Thus, analyses involving post-exercise changes compared Controls vs. all CFS Patients combined. For white cell counts, a significant Time effect was observed, in which Patients and Controls demonstrated increases at 8 hours post-exercise (F[3.4,92.7] = 16.38, *p*<.001, see Figure 10), marking this point as the likeliest time for enhanced immune activation. A significant Group by Time interaction and Time effect were observed for post-exercise changes in red cell counts (F[4,108] = 3.14, *p*<.02 and F[4,108] = 4.55, *p*<.002, respectively), showing a reduction for Controls and Patients at 8 hours post-exercise, with Controls returning to baseline by 48 hours post-exercise while Patients' red cell levels remained low during the same time period (Figure 10). The fact that white and red cell counts changed in opposite directions confirms that these effects were not due to dehydration or rehydration. Because females typically have lower red cell counts, these analyses were repeated with sex as a covariate and found the results were not changed.

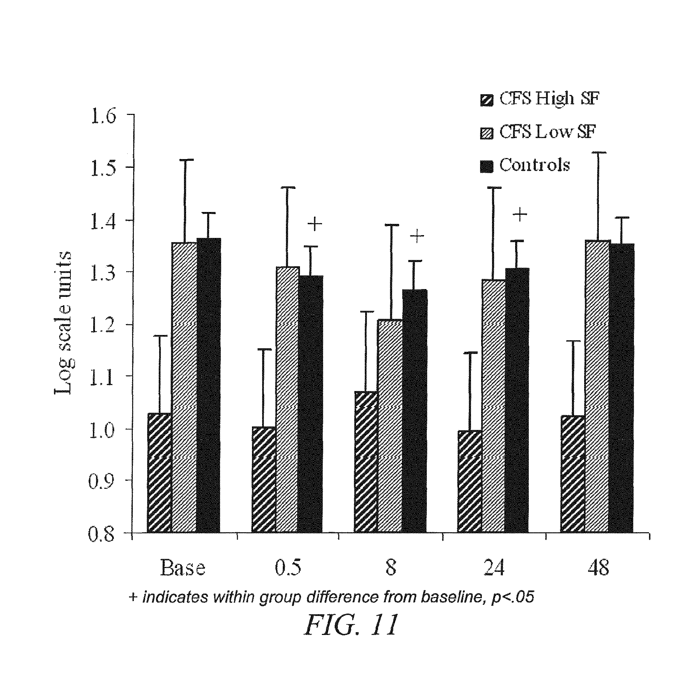

*Effect of moderate exercise on post-exercise changes in cytokines:* IL-10 levels were determined at baseline and at 0.5, 8, 24, and 48 hours post-exercise by group. A significant main effect of Time was seen for post-exercise changes from baseline in IL-10 (F[2, 33] = 2.87, *p*<.05), which decreased from baseline in Controls at 0.5, 8, and 24 hours (*p*<.05; see Figure 11). The low SF Patients showed levels and post-exercise decreases that were similar to the Controls, while high SF Patients showed a trend (p < .07) to have lower overall IL-10 levels than Controls.

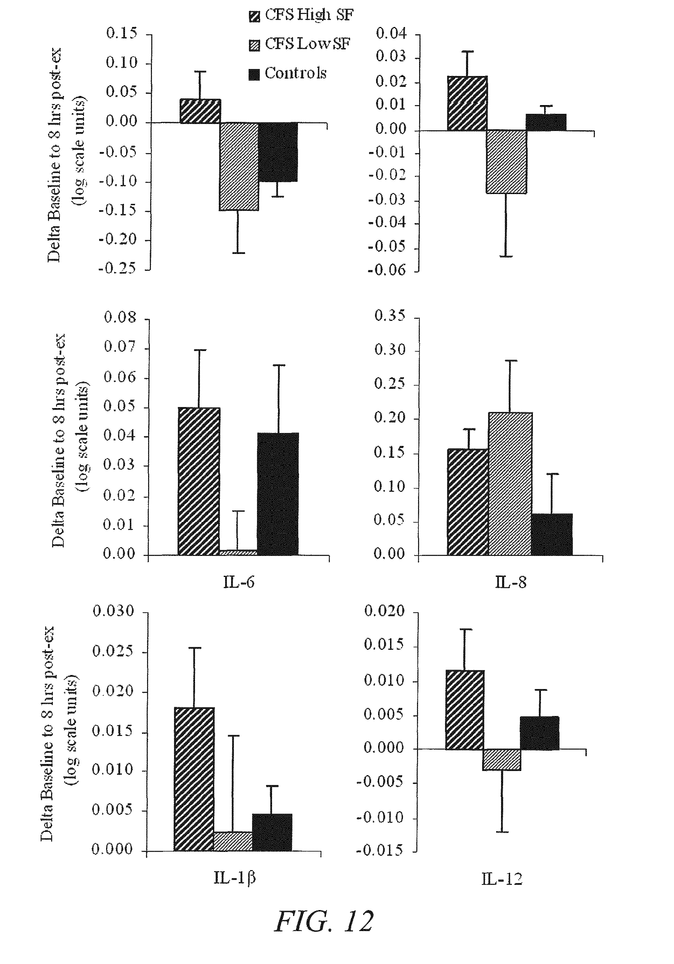

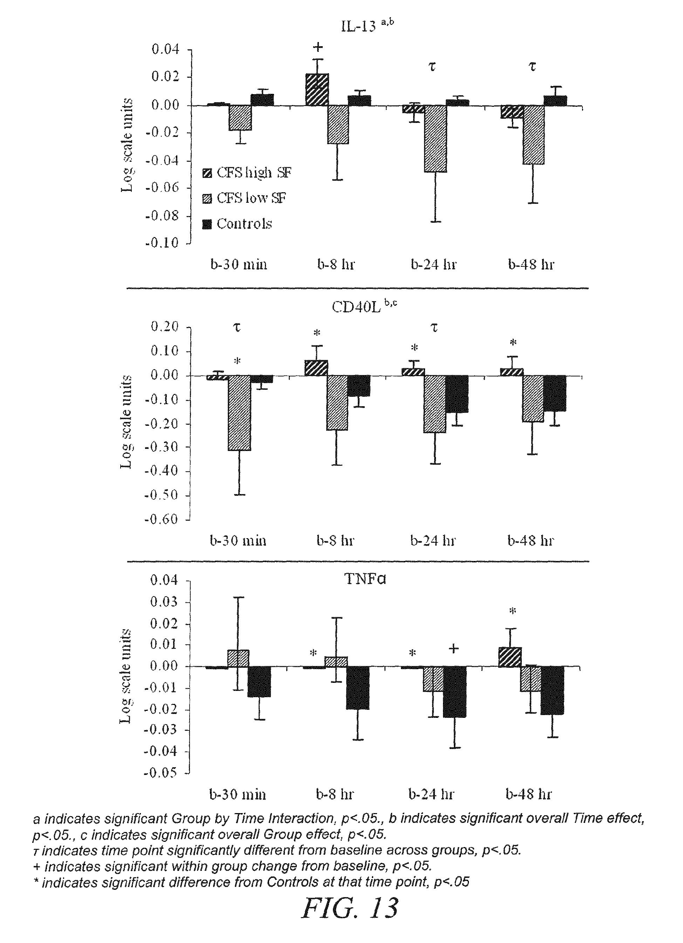

Changes from baseline to 8 hrs post-exercise were determined by group for IL-10, IL-13, IL-6, IL-8, IL-1β and IL-12. As indicated by the increase in white cells at 8 hours post-exercise, the largest increases in cytokines were observed at this time point (see Figure 12). A significant main effect of Time was obtained for IL-8 (F[2.4, 79.7] = 11.34, *p*<.01) which increased significantly only at 8 hours in both CFS subgroups (*p*<.01) while showing a non-significant increase in Controls. In the high SF Patients only, significant increases in IL-6, IL1β, IL12, IL-10 and IL-13 were seen at 8 hours (but no other times), while the low SF Patients and Controls showed decreases in IL-10 and no reliable change in any of the other cytokines at 8 hours except for IL-8 which increased significantly in the low SF as well as high SF Patients. and in IL-13 (F[2,33] = 3.92, *p*<.05) and CD40L (F[2,33] = 3.32, *p*<.05), where low SF Patients showed consistent overall decreases from 0.5 through 48 hours that were not seen in high SF Patients or Controls (see Figure 13). Controls showed gradually decreasing levels of both CD40L and TNFα after exercise, becoming significantly lower than baseline by 24 hours and showing no recovery even at 48 hours. In sum, the low SF patients and Controls showed a pattern of post-exercise decreases in both pro- and anti-inflammatory cytokines (with the exception of increases in IL-8), while the high SF Patients showed a pattern of increases in both cytokine types at 8 hours and no decreases at any time.

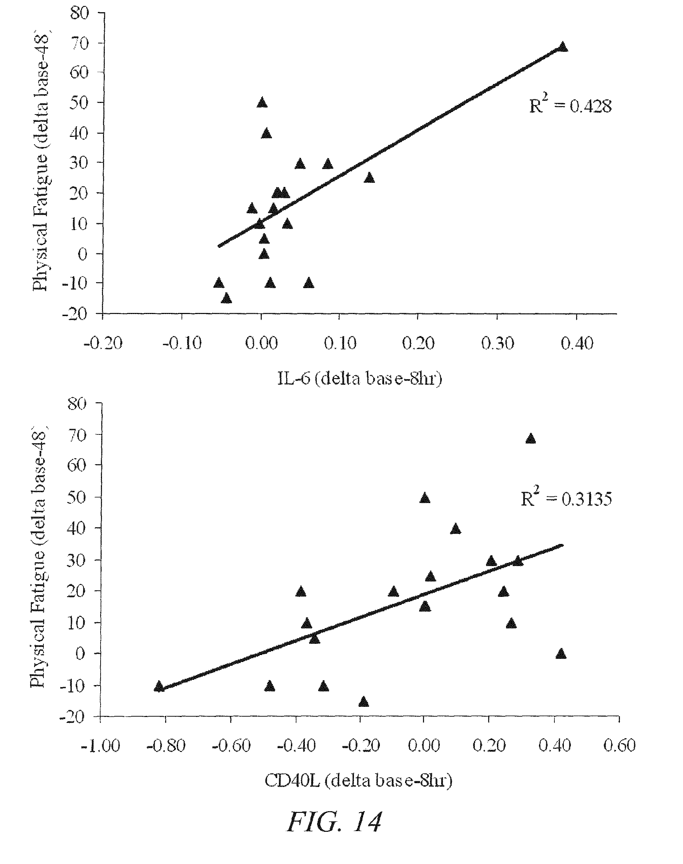

At baseline, the cytokine that had the strongest association with baseline fatigue was IL-6 (r = +.43 and +.57 with physical fatigue and mental fatigue ratings, *p*<.02). The predictive power of the relationships between changes cytokines/ligands at 8 hours post-exercise (when greatest cytokine changes usually occurred) and severity of symptom flare at 48 hours were also examined in all CFS Patients using linear regression. Increases in IL-6 and in CD40L showed the strongest predictive relationships to sustained increases in physical fatigue, accounting for 43% and 31% of the variance in fatigue increases respectively (*p* < .01; see Figure 14). Increase in IL-6 at 8 hours also accounted for 29% of the variance in increased pain at 48 hours (*p* < .01; not depicted).

In summary, CFS patients as a total group demonstrated one only immune difference from healthy controls: lower levels of pro-inflammatory CD40 ligand before and after exercise, although CFS patients with higher baseline levels of physical and mental fatigue did have higher pre-exercise IL-6 levels. After 25 minutes of moderate arm and leg exercise, healthy controls and the CFS patients who experienced milder symptom flare showed increases in only one cytokine, IL-8 at 8 hours post-exercise, while showing decreases in several pro- and anti-inflammatory cytokines (CD40L, TNFα, IL-10 and IL-13,). In contrast, those CFS patients experiencing a greater symptom flare lasting through 48 hours post-exercise showed increases in 6 cytokines at 8 hours post-exercise (pro-inflammatory IL1β, IL-8 and IL12, anti-inflammatory IL-10 and IL-13, and in IL-6). The high SF group also failed to show post-exercise decreases in CD40L, TNFα or IL-13 as shown by controls or by the less symptomatic CFS patients. IL-6 and CD40L responses at 8 hours post-exercise (when white cell counts increased significantly) accounted for 43% and 31% of the variance in long-lasting fatigue flare.

The following items are furthermore part of the disclosure:
1. A method of detecting a pathological muscle/fatigue condition in a sample comprising determining the expression level of one or more metabolite detecting genes in a sample and comparing those expression levels to the expression levels of a normal sample.
2. The method of item 1, wherein an increase in the expression level of the metabolite detecting genes above the expression levels in the normal sample indicates the presence of a pathological muscle/fatigue condition.
3. The method of item 1, further comprising determining the expression level of one or more adrenergic genes in a sample and comparing those levels to a normal sample.
4. The method of item 3, wherein an increase in the expression level of one or more adrenergic genes above the levels in the normal sample indicates the presence of a pathological muscle/fatigue condition.
5. The method of item 1, further comprising determining the expression level of one or more immune mediated genes in a sample and comparing those levels to a normal sample.
6. The method of item 5, wherein an increase in the expression level of one or more immune mediated genes above the levels in the normal sample indicates the presence of a pathological muscle/fatigue condition.
7. A method of determining responsiveness of a pathological muscle/fatigue condition in a subject to treatment with a pharmaceutical agent, said method comprising, administering the pharmaceutical agent to the subject, determining the expression level of adrenergic and immune function genes in a sample and comparing those expression levels to the expression levels of the subject prior to administering the pharmaceutical agent, wherein a decrease in the expression level of one or more adrenergic and immune function genes in the subject after administering the metabolite detecting receptor inhibitor to the subject indicates responsiveness to the pharmaceutical agent.
8. A method for treating a subject with a pathological muscle/fatigue condition, the method comprising administering to the subject in need thereof an effective amount of one or more metabolite detecting peptide antagonists as described herein in an amount sufficient to ameliorate one or more symptoms of said condition.
9. A method of ameliorating one or more symptoms associated with a pathological muscle/fatigue condition comprising administering to a subject in need thereof an effective amount of one or more metabolite detecting peptide antagonists as described herein.
10. A method of treating pain comprising administering to a subject in need thereof an effective amount of one or more metabolite detecting peptide antagonists as described herein in an amount sufficient to ameliorate pain.
11. A method of treating fatigue comprising administering to a subject in need thereof an effective amount of one or more metabolite detecting peptide antagonists as described herein in an amount sufficient to ameliorate fatigue.
12. A method of screening the efficacy of a pharmaceutical agent for the ability to treat a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent.
13. The method of item 12, further comprising determining the expression level of one or more adrenergic genes wherein a decrease in the expression level of the one or more adrenergic genes in the subject after treatment indicates efficacy of the pharmaceutical agent.
14. The method of item 12, further comprising determining the expression level of one or more immune mediated genes wherein a decrease in the expression level of the one or more immune mediated genes in the subject after treatment indicates efficacy of the pharmaceutical agent.
15. A method of screening the efficacy of a pharmaceutical agent for the ability to ameliorate one or more symptoms associated with a pathological muscle/fatigue condition comprising determining the expression level of one or more metabolite detecting genes in a subject, wherein the pharmaceutical agent was administered to the subject, wherein a decrease in the expression level of one or more metabolite detecting genes in the subject after treatment indicates efficacy of the pharmaceutical agent.
16. The method of item 15, further comprising determining the expression level of one or more adrenergic genes wherein a decrease in the expression level of the one or more adrenergic genes in the subject after treatment indicates efficacy of the pharmaceutical agent.
17. The method of item 15, further comprising determining the expression level of one or more immune mediated genes wherein a decrease in the expression level of the one or more immune mediated genes in the subject after treatment indicates efficacy of the pharmaceutical agent.

### EQUIVALENTS

Those skilled in the art will recognize, or be able to ascertain, using no more than routine experimentation, numerous equivalents to the specific embodiments described specifically herein. Such equivalents are intended to be encompassed in the scope of the following claims.

## Claims

1. A method of detecting a pathological muscle/fatigue condition in a sample, comprising determining the expression level of one or more metabolite detecting genes in a sample, wherein at least one of the one or more metabolite detecting genes is a TRPV1 gene, and comparing those expression levels to the expression levels of a normal sample, wherein a decrease in the expression level of the one or more metabolite detecting genes below the expression levels in the normal sample indicates the presence of a pathological muscle/fatigue condition.

2. The method of claim 1, further comprising determining the expression level of one or more adrenergic genes in a sample and comparing those levels to a normal sample.

3. The method of claim 2, wherein a decrease in the expression level of one or more adrenergic genes above the levels in the normal sample indicates the presence of a pathological muscle/fatigue condition.

4. The method of any of the preceding claims, further comprising determining the expression level of one or more immune mediated genes in a sample and comparing those levels to a normal sample, wherein a decrease in the expression level of one or more immune mediated genes below the levels in the normal sample indicates the presence of a pathological muscle/fatigue condition.

5. A method of determining responsiveness of a pathological muscle/fatigue condition in a subject to treatment with a pharmaceutical agent, said method comprising, administering the pharmaceutical agent to the subject, determining the expression level of one or more metabolite detecting genes including at least TRPV1 and one or more adrenergic and immune function genes in a sample and comparing those expression levels to the expression levels of the subject prior to administering the pharmaceutical agent, wherein an increase in the expression level of one or more adrenergic and metabolite detecting genes including at least TRPV1 and one or more immune function genes in the subject after administering the metabolite detecting receptor inhibitor to the subject indicates responsiveness to the pharmaceutical agent.

6. A method of screening the efficacy of a pharmaceutical agent for the ability to treat a pathological muscle/fatigue condition, comprising determining the expression level of one or more TRPV genes including at least TRPV1 in a subject, wherein the pharmaceutical agent was administered to the subject, wherein an increase in the expression level of one or more TRPV genes including at least in the subject after treatment indicates efficacy of the pharmaceutical agent.

7. The method of claim 6, further comprising determining the expression level of one or more adrenergic genes wherein a decrease in the expression level of the one or more adrenergic genes in the subject after treatment indicates efficacy of the pharmaceutical agent.

8. The method of claims 6 or 7, further comprising determining the expression level of one or more immune mediated genes wherein an increase in the expression level of the one or more immune mediated genes in the subject after treatment indicates efficacy of the pharmaceutical agent.

9. A method of screening the efficacy of a pharmaceutical agent for the ability to ameliorate one or more symptoms associated with a pathological muscle/fatigue condition comprising determining the expression level of one or more TRPV genes including at least TRPV1 in a subject, wherein the pharmaceutical agent was administered to the subject, wherein an increase in the expression level of one or more TRPV genes including at least TRPV1 in the subject after treatment indicates efficacy of the pharmaceutical agent.

10. The method of claim 9, further comprising determining the expression level of one or more immune mediated genes wherein an increase in the expression level of the one or more immune mediated genes in the subject after treatment indicates efficacy of the pharmaceutical agent.
